(19) Europäisches Patentamt
European Patent Office
Office européen des brevets

(11) EP 3 055 332 B1

(12) # EUROPEAN PATENT SPECIFICATION

(45) Date of publication and mention of the grant of the patent:
**25.09.2019 Bulletin 2019/39**

(21) Application number: **14852834.2**

(22) Date of filing: **08.10.2014**

(51) Int Cl.:
***A61K 47/68** (2017.01)* ***C07K 16/28** (2006.01)*
***A61P 35/00** (2006.01)*

(86) International application number:
**PCT/US2014/059716**

(87) International publication number:
**WO 2015/054400 (16.04.2015 Gazette 2015/15)**

(54) **ANTI-FOLR1 IMMUNOCONJUGATE DOSING REGIMENS**

DOSIERPLAN FÜR ANTI-FOLR1-IMMUNOKONJUGATE

SCHÉMAS POSOLOGIQUES D'IMMUNOCONJUGUÉS ANTI-FOLR1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.10.2013 US 201361888337 P**
**08.10.2013 US 201361888365 P**
**05.03.2014 US 201461948363 P**
**29.05.2014 US 201462004815 P**

(43) Date of publication of application:
**17.08.2016 Bulletin 2016/33**

(73) Proprietor: **ImmunoGen, Inc.**
**Waltham, MA 02451-1477 (US)**

(72) Inventors:
- **LUTZ, Robert J.**
  **Wayland, Massachusetts 01778 (US)**
- **PONTE, Jose**
  **Weymouth, Massachusetts 02188 (US)**

(74) Representative: **Lahrtz, Fritz**
**Patentanwälte**
**Isenbruck Bösl Hörschler PartG mbB**
**Prinzregentenstraße 68**
**81675 München (DE)**

(56) References cited:
**WO-A1-2011/106528 WO-A1-2012/138749**
**WO-A2-2012/135675 WO-A2-2014/036495**
**US-A1- 2011 021 555 US-A1- 2012 009 181**
**US-A1- 2012 009 181 US-A1- 2012 207 771**

- **JOSE F. PONTE ET AL: "Abstract 4641: Development of modified dosing approaches to achieve specific pharmacokinetic (PK) objectives in the first-in-human phase I clinical trial of IMGN853, a folate receptor [alpha]-targeting antibody drug conjugate", CANCER RESEARCH, vol. 74, no. 19 Supplement, 1 October 2014 (2014-10-01), pages 4641-4641, XP055361390, US ISSN: 0008-5472, DOI: 10.1158/1538-7445.AM2014-4641**
- **JASON A KONNER ET AL: "Farletuzumab, a humanized monoclonal antibody against folate receptor alpha, in epithelial ovarian cancer: a phase I study", CLINICAL CANCER RESEARCH, THE AMERICAN ASSOCIATION FOR CANCER RESEARCH, US, vol. 16, no. 21, 1 November 2010 (2010-11-01), pages 5288-5295, XP002675051, ISSN: 1078-0432, DOI: 10.1158/1078-0432.CCR-10-0700 [retrieved on 2010-09-20]**
- **KONNER ET AL.: 'Farletuzumab, a humanized monoclonal antibody against folate receptor alpha, in epithelial ovarian cancer: a phase I study' CLIN. CANCER RES. vol. 16, no. 21, 20 September 2010, pages 5288 - 5295, XP002675051**

EP 3 055 332 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The field of the invention generally relates to anti-FOLR1 immunoconjugates for use in the treatment of a FOLR1-expressing cancer. The treatment provides dosing regimens that minimize unwanted side-effects.

### Background of the Invention

Cancer is one of the leading causes of death in the developed world, with over one million people diagnosed with cancer and 500,000 deaths per year in the United States alone. Overall it is estimated that more than 1 in 3 people will develop some form of cancer during their lifetime. There are more than 200 different types of cancer, four of which-breast, lung, colorectal, and prostate-account for over half of all new cases (Jemal et al., 2003, Cancer J. Clin. 53:5-26).

Folate Receptor 1 (FOLR1), also known as Folate Receptor-alpha, or Folate Binding Protein, is an N-glycosylated protein expressed on plasma membrane of cells. FOLR1 has a high affinity for folic acid and for several reduced folic acid derivatives. FOLR1 mediates delivery of the physiological folate, 5-methyltetrahydrofolate, to the interior of cells.

FOLR1 is overexpressed in vast majority of ovarian cancers, as well as in many uterine, endometrial, pancreatic, renal, lung, and breast cancers, while the expression of FOLR1 on normal tissues is restricted to the apical membrane of epithelial cells in the kidney proximal tubules, alveolar pneumocytes of the lung, bladder, testes, choroid plexus, and thyroid (Weitman SD, et al., Cancer Res 52: 3396-3401 (1992); Antony AC, Annu Rev Nutr 16: 501-521 (1996); Kalli KR, et al. Gynecol Oncol 108: 619-626 (2008)). This expression pattern of FOLR1 makes it a desirable target for FOLR1-directed cancer therapy.

Because ovarian cancer is typically asymptomatic until advanced stage, it is often diagnosed at a late stage and has poor prognosis when treated with currently available procedures, typically chemotherapeutic drugs after surgical de-bulking (von Gruenigen V et al., Cancer 112: 2221-2227 (2008); Ayhan A et al., Am J Obstet Gynecol 196: 81 e81-86 (2007); Harry VN et al., Obstet Gynecol Surv 64: 548-560 (2009)). Thus there is a clear unmet medical need for more effective therapeutics for ovarian cancers.

Antibodies are emerging as a promising method to treat such cancers. In addition, immunoconjugates, which comprise an antibody conjugated to another compound, for example, a cytotoxin, are also being investigated as potential therapeutics. In particular, immunoconjugates comprising maytansinoids, which are plant derived anti-fungal and anti-tumor agents, have been shown to have some beneficial activities. The isolation of three ansa macrolides from ethanolic extracts of *Maytenus ovatus* and *Maytenus buchananii* was first reported by S. M. Kupchan *et al*. and is the subject of U.S. Pat. No. 3,896,111 along with demonstration of their anti-leukemic effects in murine models at the microgram/kg dose range. Maytansinoids, however, have unacceptable toxicity, causing both central and peripheral neuropathies, and side effects: particularly nausea, vomiting, diarrhea, elevations of hepatic function tests and, less commonly, weakness and lethargy. This overall toxicity is reduced to some extent by the conjugation of maytansinoids to antibodies because an antibody conjugate has a toxicity which is several orders of magnitude lower on antigen-negative cells compared to antigen-positive cells. US 2012/009181 A1 describes agents including antibodies that bind to human FOLR1 and immunoconjugates comprising these agents and a cytotoxin for the treatment of cancer. WO 2012/135675 A2 discloses antibodies that bind to human FOLR1 and immunoconjugates comprising these antibodies and a cytotoxin for increasing the efficacy of cancer therapy, wherein an increased expression of FOLR1 gene or protein has been detected. However, there is still a need to identify particular dosage regimens of anti-FOLR1 immunoconjugates that are therapeutically effective in humans but avoid adverse effects.

### BRIEF SUMMARY OF THE INVENTION

In a first aspect, the present invention relates to an immunoconjugate which binds to FOLR1 polypeptide for use in the treatment of a FOLR1-expressing cancer, wherein the immunoconjugate comprises a maytansinoid and an antibody or antigen-binding fragment thereof that comprises the variable light chain (VL) complementarity determining region (CDR)-1 of SEQ ID NO: 6, the VL CDR-2 of SEQ ID NO: 7, the VL CDR-3 of SEQ ID NO: 8, the variable heavy chain (VH) CDR-1 of SEQ ID NO: 9, the VH CDR-2 of SEQ ID NO: 11, and the VH CDR-3 of SEQ ID NO: 12, wherein the cancer is ovarian cancer, cancer of the peritoneum, endometrial cancer, or uterine cancer, preferably, wherein the cancer is a FOLR1-expressing ovarian cancer, and wherein the immunoconjugate is administered at a dose of 6 milligrams (mg) per kilogram (kg) of adjusted ideal body weight (AIBW).

In a further aspect, the present invention relates to an immunoconjugate which binds to FOLR1 polypeptide for use in the treatment of a FOLR1-expressing ovarian cancer, wherein the immunoconjugate comprises DM4 and an antibody comprising (i) a heavy chain comprising the same amino acid sequence as the amino acid sequence of the heavy chain encoded by the plasmid deposited with the American Type Culture Collection (ATCC) as PTA-10772 and

(ii) a light chain comprising the same amino acid sequence as the amino acid sequence of the light chain encoded by the plasmid deposited with the ATCC as PTA-10774, wherein the DM4 is linked to the antibody by sulfo-SPDB, wherein the immunoconjugate is formulated for IV administration, and wherein the immunoconjugate is administered at a dose of 6 milligrams (mg) per kilogram (kg) of adjusted ideal body weight (AIBW).

In yet another aspect, the present invention relates to an immunoconjugate which binds to FOLR1 polypeptide for use in the treatment of a FOLR1-expressing cancer of the peritoneum, wherein the immunoconjugate comprises DM4 and an antibody comprising (i) a heavy chain comprising the same amino acid sequence as the amino acid sequence of the heavy chain encoded by the plasmid deposited with the American Type Culture Collection (ATCC) as PTA-10772 and (ii) a light chain comprising the same amino acid sequence as the amino acid sequence of the light chain encoded by the plasmid deposited with the ATCC as PTA-10774, wherein the DM4 is linked to the antibody by sulfo-SPDB, wherein the immunoconjugate is formulated for IV administration, and wherein the immunoconjugate is administered at a dose of 6 milligrams (mg) per kilogram (kg) of adjusted ideal body weight (AIBW).

Treatment by administering an anti-FOLR1 immunoconjugate at a therapeutically effective dosing regimen that minimizes unwanted side-effects is provided herein. As described in more detail below, administration of the same dose of an anti-FOLR1 immunoconjugate to different patients results in substantial variations in the pharmacokinetics (e.g., Cmax and AUC) of the immunoconjugate. The present inventors have discovered, and the experiments provided herein demonstrate, that ocular toxicity correlates with a high Cmax and high initial AUC value. However, the high Cmax and initial AUC values are not required for efficacy. Accordingly, provided herein is the treatment of a patient having cancer comprising administering to the patient an effective dose of an immunoconjugate which binds to FOLR1 of the present invention, wherein the immunoconjugate is administered at a dose of 6 mg/kg of adjusted ideal body weight (ADJ or AIBW). The abbreviations "ADJ" and "AIBW" can be used interchangeably to refer to adjusted ideal body weight. Also, provided is the treatment of a patient having cancer comprising administering to the patient an effective dose of an immunoconjugate which binds to FOLR1 of the present invention, wherein the immunoconjugate is administered once a week for three weeks on a four-week schedule (e.g., on days 1, 8, and 15 of a four-week schedule). Described herein are methods for treating a patient having cancer comprising administering to the patient an effective dose of an immunoconjugate which binds to FOLR1, wherein the immunoconjugate is administered at a dose about 1 to about 7 mg/kg, wherein the kilograms are adjusted to IBW, LBW, or AIBW (ADJ), and wherein the immunoconjugate is administered once a week for three weeks on a four-week schedule (e.g., on days 1, 8, and 15 of a four-week schedule). The treatment and methods described herein can result in a decrease in toxicity, e.g., ocular toxicity.

In the treatment of a patient having cancer comprising administering to the patient an effective dose of an immunoconjugate which binds to FOLR1, the Cmax and initial AUC values that result in toxicity are not exceeded. For example, in some embodiments, the administration produces a Cmax of about 90-160 μg/mL, and in some embodiments, the administration produces a Cmax of about 110-160 μg/mL. In some embodiments, the administration produces an $AUC_{0-24}$ of no more than 2785 hr•μg/mL. In some embodiments, the administration produces an $AUC_{0-24}$ of no more than 2741 hr•μg/mL. In some embodiments, the administration produces an $AUC_{0-24}$ of no more than 2700 hr•μg/mL. In some embodiments, the administration produces a Cmax of no more than 160 μg/mL. In some embodiments, the administration produces a Cmax of no more than 150 μg/mL.

In some embodiments, the administration produces an $AUC_{0-24}$ of about 1000-3500 hr•μg/mL. In some embodiments, the administration produces an $AUC_{0-24}$ of about 1000-3000 hr•μg/mL. In some embodiments, the administration produces an $AUC_{0-24}$ of about 1000-2785 hr•μg/mL. In some embodiments, the administration produces an $AUC_{0-24}$ of about 1000-2741 hr•μg/mL. In some embodiments, the administration produces an $AUC_{0-24}$ of about 1000-2700 hr•μg/mL. In some embodiments, the administration produces an $AUC_{0-24}$ of about 1000-2500 hr•μg/mL. In some embodiments, the administration produces an $AUC_{0-24}$ of no more than 1500-3500 hr•μg/mL. In some embodiments, the administration produces an $AUC_{0-24}$ of no more than 1500-3000 hr•μg/mL. In some embodiments, the administration produces an $AUC_{0-24}$ of no more than 1500-2785 hr•μg/mL. In some embodiments, the administration produces an $AUC_{0-24}$ of no more than 1500-2741 hr•μg/mL. In some embodiments, the administration produces an $AUC_{0-24}$ of no more than 1500-2700 hr•μg/mL. In some embodiments, the administration produces an $AUC_{0-24}$ of about 1500-2500 hr•μg/mL.

In some embodiments, the administration produces a Cmax of 110-160 μg/mL. In some embodiments, the administration produces a Cmax of 110-150 μg/mL. In some embodiments, the administration produces a Cmax of 110-140 μg/mL. In some embodiments, the administration produces a Cmax of 120-160 μg/mL. In some embodiments, the administration produces a Cmax of about 120-150 μg/mL. In some embodiments, the administration produces a Cmax of about 120-140 μg/mL. In some embodiments, the administration produces a Cmax of 90-160 μg/mL. In some embodiments, the administration produces a Cmax of about 90-150 μg/mL. In some embodiments, the administration produces a Cmax of about 90-140 μg/mL. In some embodiments, the administration produces a Cmax of 100-160 μg/mL. In some embodiments, the administration produces a Cmax of about 100-150 μg/mL. In some embodiments, the administration produces a Cmax of about 100-140 μg/mL.

The anti-FOLR1 immunoconjugate can comprise a charged linker. In some embodiments, the anti-FOLR1

immunoconjugate comprises the antibody huMovl9, the linker sulfo-SPDB, and the maytansinoid DM4.

The antibody or fragment thereof of the immunoconjugate of the present invention comprises the CDRs of huMOV19 (i.e., SEQ ID NOs: 6-9, 11, and 12). In some embodiments, the antibody or antigen-binding fragment thereof comprises the variable region sequences of huMOV19 (i.e., SEQ ID NOs: 3 and 4 or 5). The antibodies or fragments do not comprise the six CDRs of murine Mov19 (i.e., SEQ ID NOs:6-9, 16, and 12). In some embodiments, the antibody is huMov19. According to the invention, the immunoconjugate comprises a maytansinoid. In some embodiments, the maytansinoid is DM4. In some embodiments, the immunoconjugate comprises a linker that is sulfo-SPDB. In some embodiments, the immunoconjugate is IMGN853 (huMov19-sulfo-SPDB-DM4).

Described is that the anti-FOLR1 binding agent (e.g., huMov19-sulfo-SPDB-DM4) may be administered at a dose of about 1 to 7 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 1.0 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 1.1 mg/kg, wherein the kilograms of body weight are adjusted to IBW, LBW, BSA, or AIBW (ADJ). The anti-FOLR1 binding agent (e.g., huMovl9-sulfo-SPDB-DM4) may be administered at a dose of about 1.5 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 1.8 mg/kg, wherein the kilograms of body weight are adjusted to IBW, LBW, BSA, or AIBW (ADJ). The anti-FOLR1 binding agent (e.g., huMov19-sulfo-SPDB-DM4) is administered at a dose of about 2.0 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 2.5 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 2.8 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 3.0 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 3.3 mg/kg, wherein the kilograms of body weight are adjusted to IBW, LBW, BSA, or AIBW (ADJ). may be he anti-FOLR1 binding agent (e.g., huMov19-sulfo-SPDB-DM4) may be administered at a dose of about 3.75 mg/kg, wherein the kilograms of body weight are adjusted to IBW, LBW, BSA, or AIBW (ADJ). The anti-FOLR1 binding agent (e.g., huMovl9-sulfo-SPDB-DM4) may be administered at a dose of about 4.0 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 4.1 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 4.2 mg/kg, wherein the kilograms of body weight are adjusted to IBW, LBW, BSA, or AIBW (ADJ). The anti-FOLR1 binding agent (e.g., huMovl9-sulfo-SPDB-DM4) may be administered at a dose of about 5 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 5.5 mg/kg, wherein the kilograms of body weight are adjusted to IBW, LBW, BSA, or AIBW (ADJ). The anti-FOLR1 binding agent (e.g., huMovl9-sulfo-SPDB-DM4) may be administered at a dose of about 5.6 mg/kg, wherein the kilograms of body weight are adjusted to IBW, LBW, BSA, or AIBW (ADJ). The anti-FOLR1 binding agent (e.g., huMovl9-sulfo-SPDB-DM4) may be administered at a dose of about 6 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 6.5 mg/kg, wherein the kilograms of body weight are adjusted to IBW, LBW, BSA, or AIBW (ADJ). The anti-FOLR1 binding agent (e.g., huMov19-sulfo-SPDB-DM4) may be administered at a dose of about 7 mg/kg, wherein the kilograms of body weight are adjusted to IBW, LBW, BSA, or AIBW (ADJ). The kilograms of body weight may be adjusted to AIBW (ADJ). According to the invention, the immunoconjugate of the present invention is administered at a dose of 6 mg/kg of adjusted ideal body weight (AIBW).

In some embodiments, the anti-FOLR1 binding agent (e.g., huMov19-sulfo-SPDB-DM4) is administered about once every 4 weeks. In some embodiments, the anti-FOLR1 binding agent (e.g., huMov19-sulfo-SPDB-DM4) is administered about once every 3 weeks. In some embodiments, the anti-FOLR1 binding agent (e.g., huMov19-sulfo-SPDB-DM4) is administered about once every 2 weeks. In some embodiments, the anti-FOLR1 binding agent (e.g., huMov19-sulfo-SPDB-DM4) is administered about once every 1 week.

Described is that the immunoconjugate may be administered at a dose of about 1 to 7 mg/kg once a week for three weeks on a four-week schedule. The immunoconjugate may be administered at a dose of about 1.5 mg/kg to about 6 mg/kg once a week for three weeks on a four-week schedule. The immunoconjugate may be administered at a dose of about 1.5, 2.0, 2.5, 3, 3.75, 4.0, 4.1, 4.2, 5.0, 5.5, or 6.0 mg/kg once a week for three weeks on a four-week schedule. The immunoconjugate may be administered at a dose of about 1.1, 1.8, 2.5, 3.3, or 4.2 mg/kg once a week for three weeks on a four-week schedule. The anti-FOLR1 immunoconjugate may be administered at a dose of about 1.0 mg/kg once a week for three weeks on a four-week schedule. The anti-FOLR1 immunoconjugate may be administered at a dose of about 1.1 mg/kg once a week for three weeks on a four-week schedule. The anti-FOLRl immunoconjugate may

be administered at a dose of about 1.5 mg/kg once a week for three weeks on a four-week schedule. The anti-FOLR1 immunoconjugate may be administered at a dose of about 1.8 mg/kg once a week for three weeks on a four-week schedule. The anti-FOLR1 immunoconjugate may be administered at a dose of about 2 mg/kg once a week for three weeks on a four-week schedule. The anti-FOLR1 immunoconjugate may be administered at a dose of about 2.5 mg/kg once a week for three weeks on a four-week schedule. The anti-FOLRl immunoconjugate may be administered at a dose of about 2.8 mg/kg once a week for three weeks on a four-week schedule. The anti-FOLR1 binding agent may be administered at a dose of about 3.0 mg/kg once a week for three weeks on a four-week schedule. The anti-FOLR1 immunoconjugate may be administered at a dose of about 3.3 mg/kg once a week for three weeks on a four-week schedule. The anti-FOLR1 immunoconjugate may be administered at a dose of about 3.75 mg/kg once a week for three weeks on a four-week schedule. The anti-FOLR1 binding agent may be administered at a dose of about 4.0 mg/kg once a week for three weeks on a four-week schedule. The anti-FOLR1 immunoconjugate may be administered at a dose of about 4.1 mg/kg once a week for three weeks on a four-week schedule. The anti-FOLR1 immunoconjugate may be administered at a dose of about 4.2 mg/kg once a week for three weeks on a four-week schedule. The anti-FOLR1 immunoconjugate may be administered at a dose of about 5 mg/kg once a week for three weeks on a four-week schedule. The anti-FOLR1 binding agent may be administered at a dose of about 5.5 mg/kg once a week for three weeks on a four-week schedule. The anti-FOLR1 immunoconjugate may is administered at a dose of about 5.6 mg/kg once a week for three weeks on a four-week schedule. Te anti-FOLR1 binding agent may be administered at a dose of about 6 mg/kg once a week for three weeks on a four-week schedule. The anti-FOLR1 immunoconjugate may be administered at a dose of about 6.5 mg/kg once a week for three weeks on a four-week schedule. The anti-FOLR1 immunoconjugate may be administered at a dose of about 7 mg/kg once a week for three weeks on a four-week schedule.

Described is that the immunoconjugate (e.g., huMov19-sulfo-SPDB-DM4) may be administered at a dose of about 1 to 7 mg/kg, wherein the kilograms are adjusted to IBW, LBW, BSA, or AIBW (ADJ), and wherein the immunoconjugate is administered once a week for three weeks on a four-week schedule. The immunoconjugate (e.g., huMov19-sulfo-SPDB-DM4) may be administered at a dose of about 1.5 mg/kg to about 6 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 1 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 1.1 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 1.5 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 1.8 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 2 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 2.5 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 2.8 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 3 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 3.3 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 3.75 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 4 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMovl9-sulfo-SPDB-DM4) may be administered at a dose of about 4.1 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 4.2 mg/kg, wherein the kilograms are adjusted to IBW, LBW, BSA, or AIBW (ADJ), and wherein the immunoconjugate is administered once a week for three weeks on a four-week

schedule. The anti-FOLR1 immunoconjugate (e.g., huMov19-sulfo-SPDB-DM4) may be administered at a dose of about 5 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 5.5 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 5.6 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 6 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 6.5 mg/kg, wherein the kilograms are adjusted to IBW, LBW, BSA, or AIBW (ADJ), and wherein the immunoconjugate is administered once a week for three weeks on a four-week schedule. The anti-FOLR1 immunoconjugate (e.g., huMov19-sulfo-SPDB-DM4) may be administered at a dose of about 7 mg/kg, wherein the kilograms are adjusted to IBW, LBW, BSA, or AIBW (ADJ), and wherein the immunoconjugate is administered once a week for three weeks on a four-week schedule. The kilograms may be adjusted to AIBW (ADJ).

In some embodiments, the FOLR1-binding agents are administered to obtain the AUC obtained in Examples 1-6 and shown in Figures 1-2 and 7-12.

In some embodiments, the FOLR1-binding agents are administered to obtain the Cmax obtained in Examples 1-6 and shown in Figures 1-6 and 9-12.

In some embodiments, the anti-FOLR1 binding agent (e.g., huMov19-sulfo-SPDB-DM4) is administered intravenously.

According to the invention, the immunoconjugate of the present invention is used in the treatment of cancer, whereby the cancer is selected from the group consisting of ovarian cancer, cancer of the peritoneum, uterine cancer or endometrial cancer,. In some embodiments, the cancer is ovarian cancer. In some embodiments, the cancer is epithelial ovarian cancer.

The cancer expresses FOLR1 polypeptide or nucleic acid. In some embodiments, the cancer has an increased expression level of FOLR1 polypeptide as measured by immunohistochemistry (IHC). For example, in some embodiments, the cancer is a cancer that expresses FOLR1 polypeptide at a level of 1 hetero or higher by IHC. In some embodiments, the cancer is a cancer that expresses FOLR1 polypeptide at a level of 1 homo or higher by IHC. In some embodiments, the cancer is a cancer that expresses FOLR1 polypeptide at a level of 2 hetero or higher by IHC. In some embodiments, the cancer is a cancer that expresses FOLR1 polypeptide at a level of 2 homo or higher by IHC. In some embodiments, the cancer is a cancer that expresses FOLR1 polypeptide at a level of 3 hetero or higher by IHC. In some embodiments, the cancer is a cancer that expresses FOLR1 polypeptide at a level of 3 homo or higher by IHC. Described is that the cancer may be a lung cancer that expresses FOLR1 polypeptide at a level of 2 hetero or higher by IHC. Described is that the cancer may be a lung cancer that expresses FOLR1 polypeptide at a level of 3 hetero or higher by IHC. In some embodiments, the cancer is an epithelial ovarian cancer (e.g., platinum resistant or relapsed or refractory) that expresses FOLR1 polypeptide at a level of 2 hetero or higher. In some embodiments, the cancer is an epithelial ovarian cancer (e.g., platinum resistant or relapsed or refractory) that expresses FOLR1 polypeptide at a level of 3 hetero or higher. In some embodiments, the cancer is an endometrioid cancer that expresses FOLR1 polypeptide at a level of 1 hetero or higher. In some embodiments, the cancer is an endometrioid cancer that expresses FOLR1 polypeptide at a level of 2 hetero or higher.

In some embodiments, the treatment further comprises administering a steroid to the patient. The steroid can be administered as a pre-treatment, i.e., prior to the administration of the anti-FOLRl binding agent. The steroid can be dexamethasone.

The treatment can result in a decrease in tumor size. The treatment can result in a decrease in CA125 levels in ovarian cancer patients. In one example, CA125 levels are measured in a sample from an ovarian cancer patient prior to treatment and then one or more times after treatment, and a decrease in the CA125 level over time is indicative of therapeutic efficacy. The treatment can result in an increased time between cancer treatments. The treatment can result in increased progression free survival (PFS). The treatment can result in increased disease-free survival (DFS). The treatment can result in increased overall survival (OS). The treatment can result in increased complete response (CR). The treatment can result in increased partial response (PR). The treatment can result in increased stable disease (SD). The treatment can result in increased decrease in progressive disease (PD). The treatment can result in a reduced time to progression (TTP).

In particular, the dosing regiments provided herein achieve an optimal balance between efficacy (e.g., PR) and reduced toxicity as demonstrated, for instance, in Examples 1-4 and Figures 1-7.

BRIEF DESCRIPTION OF THE DRAWINGS/FIGURES

Figures 1A and B provide pharmacokinetic data resulting from the administration of IMGN853 (0.15 mg/kg to 7.0 mg/kg) as described in Example 1. Figure 1B provides a later summary of the pharmacokinetic data that includes the data from Figure 1A and additional data obtained from additional patients.

Figures 2A-C show the responses and the occurrence of ocular toxicity in patients with a range of Cmax and $AUC_{0-24}$ and $AUC_{0-168}$ values.

Figure 3 shows the range of Cmax values measured at various doses.

Figure 4 shows the dependence of Cmax on patient body weight.

Figure 5 shows the variance in Cmax and $AUC_{0-24}$ associated with alternate dosing approaches.

Figure 6 shows the projected dependence of Cmax on body weight using alternate dosing approaches.

Figure 7 shows a plot of the $AUC_{0-24}$ values observed in 24 patients receiving 3.3, 5, or 7 mg/kg IMGN853 based on total body weight (actual). These values are compared to projected values if all the patients had been treated with 5 mg/kg based on total body weight, (TBW 5 mg/kg) and the projected values if all of the patients were dosed at 5, 5.4, or 6 mg/kg based on adjusted ideal body weight (ADJ 5, 5.4 or 6). The actual data of 7 patients treated at 5 mg/kg by adjusted ideal body weight (5 ADJ Actual) is also shown. The percentage of patients that have or are projected to have AUC values above the ocular toxicity threshold is shown in the table below the plot.

Figure 8 shows $AUC_{0-24}$ values for all patients in the 3.3 - 7.0 mg/kg. TBW cohorts were used to calculate predicted $AUC_{0-24}$ values at the indicated dose levels. Actual patient $AUC_{0-24}$ data for the indicated doses are also plotted, including patients dosed in the 5.0 and 6.0 adjusted idea body weight (AIBW) cohorts to date.

Figure 9 shows the anti-tumor activity, predicted plasma concentration, and other pharmacokinetic parameters of IMGN853 in mice treated with single doses of 2.8 mg/kg, 5.6 mg/kg, or 8.5 mg/kg of the immunoconjugate.

Figure 10 shows the anti-tumor activity, predicted plasma concentration, and other pharmacokinetic parameters of IMGN853 in mice treated with a single dose of 8.5 mg/kg, three daily doses of 2.8 mg/kg, or three doses of 2.8 mg/kg every three days.

Figure 11 shows the anti-tumor activity, predicted plasma concentration, and other pharmacokinetic parameters of IMGN853 in mice treated with a single dose of 5.6 mg/kg or 1.4 mg/kg daily for three days.

Figure 12 shows the anti-tumor activity, predicted plasma concentration, and other pharmacokinetic parameters of IMGN853 in mice treated with a single dose of 8.5 mg/kg or 2.8 mg/kg weekly for three weeks.

DETAILED DESCRIPTION OF THE INVENTION

The present invention provides new dosing regimens for FOLR1 binding immunoconjugates.

**I. Definitions**

To facilitate an understanding of the present invention, a number of terms and phrases are defined below.

The terms "human folate receptor 1," "FOLR1," or "folate receptor alpha (FR-α)", as used herein, refers to any native human FOLR1, unless otherwise indicated. Thus, all of these terms can refer to either a protein or nucleic acid sequence as indicated herein. The term "FOLR1" encompasses "full-length," unprocessed FOLR1 as well as any form of FOLR1 that results from processing within the cell. The term also encompasses naturally occurring variants of FOLR1, e.g., splice variants, allelic variants and isoforms. The FOLR1 polypeptides described herein can be isolated from a variety of sources, such as from human tissue types or from another source, or prepared by recombinant or synthetic methods. Examples of FOLR1 sequences include, but are not limited to NCBI reference numbers P15328, NP_001092242.1, AAX29268.1, AAX37119.1, NP_057937.1, and NP_057936.1.

The term "antibody" means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. As used herein, the term "antibody" encompasses intact polyclonal antibodies, intact monoclonal antibodies, antibody fragments (such as Fab, Fab', F(ab')2, and Fv fragments), single chain Fv (scFv) mutants, multispecific antibodies such as bispecific antibodies generated from at least two intact antibodies, chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. An antibody can be of any the five major classes of immunoglobulins: IgA, IgD, IgE, IgG, and IgM, or subclasses (isotypes) thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well known subunit structures and

three-dimensional configurations. Antibodies can be naked or conjugated to other molecules such as toxins, radioisotopes, etc.

A "blocking" antibody or an "antagonist" antibody is one which inhibits or reduces biological activity of the antigen it binds, such as FOLR1. In some embodiments, blocking antibodies or antagonist antibodies substantially or completely inhibit the biological activity of the antigen. The biological activity can be reduced by 10%, 20%, 30%, 50%, 70%, 80%, 90%, 95%, or even 100%.

The term "anti-FOLR1 antibody" or "an antibody that binds to FOLR1" refers to an antibody that is capable of binding FOLR1 with sufficient affinity such that the antibody is useful as a diagnostic and/or therapeutic agent in targeting FOLR1. The extent of binding of an anti-FOLR1 antibody to an unrelated, non-FOLR1 protein can be less than about 10% of the binding of the antibody to FOLR1 as measured, e.g., by a radioimmunoassay (RIA). In certain embodiments, an antibody that binds to FOLR1 has a dissociation constant (Kd) of $\leq 1\ \mu M$, $\leq 100$ nM, $\leq 10$ nM, $\leq 1$ nM, or $\leq 0.1$ nM.

The term "antibody fragment" refers to a portion of an intact antibody and refers to the antigenic determining variable regions of an intact antibody. Examples of antibody fragments include, but are not limited to Fab, Fab', F(ab')2, and Fv fragments, linear antibodies, single chain antibodies, and multispecific antibodies formed from antibody fragments.

A "monoclonal antibody" refers to a homogeneous antibody population involved in the highly specific recognition and binding of a single antigenic determinant, or epitope. This is in contrast to polyclonal antibodies that typically include different antibodies directed against different antigenic determinants. The term "monoclonal antibody" encompasses both intact and full-length monoclonal antibodies as well as antibody fragments (such as Fab, Fab', F(ab')2, Fv), single chain (scFv) mutants, fusion proteins comprising an antibody portion, and any other modified immunoglobulin molecule comprising an antigen recognition site. Furthermore, "monoclonal antibody" refers to such antibodies made in any number of manners including but not limited to by hybridoma, phage selection, recombinant expression, and transgenic animals.

The term "humanized antibody" refers to forms of non-human (e.g. murine) antibodies that are specific immunoglobulin chains, chimeric immunoglobulins, or fragments thereof that contain minimal non-human (e.g., murine) sequences. Typically, humanized antibodies are human immunoglobulins in which residues from the complementary determining region (CDR) are replaced by residues from the CDR of a non-human species (e.g. mouse, rat, rabbit, hamster) that have the desired specificity, affinity, and capability (Jones et al., 1986, Nature, 321:522-525; Riechmann et al., 1988, Nature, 332:323-327; Verhoeyen et al., 1988, Science, 239:1534-1536). In some instances, the Fv framework region (FR) residues of a human immunoglobulin are replaced with the corresponding residues in an antibody from a non-human species that has the desired specificity, affinity, and capability. The humanized antibody can be further modified by the substitution of additional residues either in the Fv framework region and/or within the replaced non-human residues to refine and optimize antibody specificity, affinity, and/or capability. In general, the humanized antibody will comprise substantially all of at least one, and typically two or three, variable domains containing all or substantially all of the CDR regions that correspond to the non-human immunoglobulin whereas all or substantially all of the FR regions are those of a human immunoglobulin consensus sequence. The humanized antibody can also comprise at least a portion of an immunoglobulin constant region or domain (Fc), typically that of a human immunoglobulin. Examples of methods used to generate humanized antibodies are described in U.S. Pat. 5,225,539. In some embodiments, a "humanized antibody" is a resurfaced antibody.

A "variable region" of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies. There are at least two techniques for determining CDRs: (1) an approach based on cross-species sequence variability (i.e., Kabat et al. Sequences of Proteins of Immunological Interest, (5th ed., 1991, National Institutes of Health, Bethesda Md.)); and (2) an approach based on crystallographic studies of antigen-antibody complexes (Al-lazikani et al (1997) J. Molec. Biol. 273:927-948)). In addition, combinations of these two approaches are sometimes used in the art to determine CDRs.

The Kabat numbering system is generally used when referring to a residue in the variable domain (approximately residues 1-107 of the light chain and residues 1-113 of the heavy chain) (e.g., Kabat et al., Sequences of Immunological Interest. 5th Ed. Public Health Service, National Institutes of Health, Bethesda, Md. (1991)).

The amino acid position numbering as in Kabat, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence can contain fewer or additional amino acids corresponding to a shortening of, or insertion into, a FR or CDR of the variable domain. For example, a heavy chain variable domain can include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues can be determined for a given antibody by alignment at regions of homology of the sequence of the antibody with a "standard" Kabat numbered sequence. Chothia refers instead to the location of the structural loops (Chothia and

Lesk J. Mol. Biol. 196:901-917 (1987)). The end of the Chothia CDR-H1 loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). The AbM hypervariable regions represent a compromise between the Kabat CDRs and Chothia structural loops, and are used by Oxford Molecular's AbM antibody modeling software.

| Loop | Kabat | AbM | Chothia |
|---|---|---|---|
| L1 | L24-L34 | L24-L34 | L24-L34 |
| L2 | L50-L56 | L50-L56 | L50-L56 |
| L3 | L89-L97 | L89-L97 | L89-L97 |
| H1 | H31-H35B | H26-H35B | H26-H32..34 |
| | | (Kabat Numbering) | |
| H1 | H31-H35 | H26-H35 | H26-H32 |
| | | (Chothia Numbering) | |
| H2 | H50-H65 | H50-H58 | H52-H56 |
| H3 | H95-H102 | H95-H102 | H95-H102 |

The term "human antibody" means an antibody produced by a human or an antibody having an amino acid sequence corresponding to an antibody produced by a human made using any technique known in the art. This definition of a human antibody includes intact or full-length antibodies, fragments thereof, and/or antibodies comprising at least one human heavy and/or light chain polypeptide such as, for example, an antibody comprising murine light chain and human heavy chain polypeptides.

The term "chimeric antibodies" refers to antibodies wherein the amino acid sequence of the immunoglobulin molecule is derived from two or more species. Typically, the variable region of both light and heavy chains corresponds to the variable region of antibodies derived from one species of mammals (e.g. mouse, rat, rabbit, etc.) with the desired specificity, affinity, and capability while the constant regions are homologous to the sequences in antibodies derived from another (usually human) to avoid eliciting an immune response in that species.

The term "epitope" or "antigenic determinant" are used interchangeably herein and refer to that portion of an antigen capable of being recognized and specifically bound by a particular antibody. When the antigen is a polypeptide, epitopes can be formed both from contiguous amino acids and noncontiguous amino acids juxtaposed by tertiary folding of a protein. Epitopes formed from contiguous amino acids are typically retained upon protein denaturing, whereas epitopes formed by tertiary folding are typically lost upon protein denaturing. An epitope typically includes at least 3, and more usually, at least 5 or 8-10 amino acids in a unique spatial conformation.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative embodiments are described in the following.

"Or better" when used herein to refer to binding affinity refers to a stronger binding between a molecule and its binding partner. "Or better" when used herein refers to a stronger binding, represented by a smaller numerical Kd value. For example, an antibody which has an affinity for an antigen of "0.6 nM or better", the antibody's affinity for the antigen is <0.6 nM, i.e. 0.59 nM, 0.58 nM, 0.57 nM etc. or any value less than 0.6 nM.

By "specifically binds," it is generally meant that an antibody binds to an epitope via its antigen binding domain, and that the binding entails some complementarity between the antigen binding domain and the epitope. According to this definition, an antibody is said to "specifically bind" to an epitope when it binds to that epitope, via its antigen binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D."

By "preferentially binds," it is meant that the antibody specifically binds to an epitope more readily than it would bind to a related, similar, homologous, or analogous epitope. Thus, an antibody which "preferentially binds" to a given epitope would more likely bind to that epitope than to a related epitope, even though such an antibody may cross-react with the related epitope.

An antibody is said to "competitively inhibit" binding of a reference antibody to a given epitope if it preferentially binds to that epitope to the extent that it blocks, to some degree, binding of the reference antibody to the epitope. Competitive inhibition may be determined by any method known in the art, for example, competition ELISA assays. An antibody may be said to competitively inhibit binding of the reference antibody to a given epitope by at least 90%, at least 80%, at least 70%, at least 60%, or at least 50%.

The phrase "substantially similar," or "substantially the same", as used herein, denotes a sufficiently high degree of similarity between two numeric values (generally one associated with an antibody of the invention and the other associated with a reference/comparator antibody) such that one of skill in the art would consider the difference between the two values to be of little or no biological and/or statistical significance within the context of the biological characteristic measured by said values (e.g., Kd values). The difference between said two values can be less than about 50%, less than about 40%, less than about 30%, less than about 20%, or less than about 10% as a function of the value for the reference/comparator antibody.

A polypeptide, antibody, polynucleotide, vector, cell, or composition which is "isolated" is a polypeptide, antibody, polynucleotide, vector, cell, or composition which is in a form not found in nature. Isolated polypeptides, antibodies, polynucleotides, vectors, cell or compositions include those which have been purified to a degree that they are no longer in a form in which they are found in nature. An antibody, polynucleotide, vector, cell, or composition which is isolated is substantially pure.

As used herein, "substantially pure" refers to material which is at least 50% pure (i.e., free from contaminants), at least 90% pure, at least 95% pure, at least 98% pure, or at least 99% pure.

The term "immunoconjugate" or "conjugate" as used herein refers to a compound or a derivative thereof that is linked to a cell binding agent (i.e., an anti-FOLRl antibody or fragment thereof) and is defined by a generic formula: C-L-A, wherein C = cytotoxin, L = linker, and A = anti-FOLRl antibody or antibody fragment. Immunoconjugates can also be defined by the generic formula in reverse order: A-L-C.

The term "IMGN853" refers to the immunoconjugate described herein containing the huMov19 antibody, the sulfoSPDB linker, and the DM4 maytansinoid. The huMov19 antibody contains a variable heavy chain with the amino acid sequence of SEQ ID NO:3 and a variable light chain with the amino acid sequence of SEQ ID NO: 5.

A "linker" is any chemical moiety that is capable of linking a compound, usually a drug, such as a maytansinoid, to a cell-binding agent such as an anti FOLR1 antibody or a fragment thereof in a stable, covalent manner. Linkers can be susceptible to or be substantially resistant to acid-induced cleavage, light-induced cleavage, peptidase-induced cleavage, esterase-induced cleavage, and disulfide bond cleavage, at conditions under which the compound or the antibody remains active. Suitable linkers are well known in the art and include, for example, disulfide groups, thioether groups, acid labile groups, photolabile groups, peptidase labile groups and esterase labile groups. Linkers also include charged linkers, and hydrophilic forms thereof as described herein and know in the art.

The terms "cancer" and "cancerous" refer to or describe the physiological condition in mammals in which a population of cells are characterized by unregulated cell growth. Examples of cancer include, but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancers. The cancer can be a cancer that expresses FOLR1.

"Tumor" and "neoplasm" refer to any mass of tissue that result from excessive cell growth or proliferation, either benign (noncancerous) or malignant (cancerous) including pre-cancerous lesions.

The terms "cancer cell," "tumor cell," and grammatical equivalents refer to the total population of cells derived from a tumor or a pre-cancerous lesion, including both non-tumorigenic cells, which comprise the bulk of the tumor cell population, and tumorigenic stem cells (cancer stem cells). As used herein, the term "tumor cell" will be modified by the term "non-tumorigenic" when referring solely to those tumor cells lacking the capacity to renew and differentiate to distinguish those tumor cells from cancer stem cells.

The term "subject" refers to any animal (e.g., a mammal), including, but not limited to humans, non-human primates, rodents, and the like, which is to be the recipient of a particular treatment. Typically, the terms "subject" and "patient" are used interchangeably herein in reference to a human subject.

The term "ideal body weight" (IBW) refers to a size descriptor that is unrelated to total body weight. IBW is an estimate of weight corrected for sex and height, and optionally frame size. IBW can be calculated, for example, using

the formulas IBW = 0.9H-88 (for males) and IBW = 0.9H-92 (for females), wherein H=height in cm.

The term "lean body weight" (LBW) refers to a size descriptor that can account for fractional fat mass ($FM_{frac}$). LBW is equal to total body weight minus the product of $FM_{frac}$ and weight. LBW can be calculated, for example, using the formulas LBW = 1.10 x weight in kg - 128([weight in kg]$^2$ / [100 x height in meters]$^2$) (for males) and LBW = 1.07 x weight in kg - 148([weight in kg]$^2$ / [100 x height in meters]$^2$) (for females).

The term "adjusted ideal body weight" (AIBW) or "adjusted body weight" (ADJ) refers to a size descriptor that accounts for sex, total body weight, and height. AIBW and ADJ are used interchangeably throughout the specification. AIBW (ADJ) can be calculated, for example, using the formula ADJ = IBW + 0.4(weight in kg - IBW).

IBW, LBW, and AIBW (ADJ) are discussed in more detail in Green and Duffull, British Journal of Clinical Pharmacology 58: 119-133 (2004).

Administration "in combination with" one or more further therapeutic agents includes simultaneous (concurrent) and consecutive administration in any order.

The term "pharmaceutical formulation" refers to a preparation which is in such form as to permit the biological activity of the active ingredient to be effective, and which contains no additional components which are unacceptably toxic to a subject to which the formulation would be administered. The formulation can be sterile.

An "effective amount" of an antibody or immunoconjugate as disclosed herein is an amount sufficient to carry out a specifically stated purpose. An "effective amount" can be determined empirically and in a routine manner, in relation to the stated purpose.

The term "therapeutically effective amount" refers to an amount of an antibody or other drug effective to "treat" a disease or disorder in a subject or mammal. In the case of cancer, the therapeutically effective amount of the drug can reduce the number of cancer cells; reduce the tumor size; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP), a decrease in CA125 in the case of ovarian cancer, or any combination thereof.

See the definition herein of "treating." To the extent the drug can prevent growth and/or kill existing cancer cells, it can be cytostatic and/or cytotoxic. Identification of increased FOLR1 levels may allow for administration of decreased amounts of the FOLR1-targeting therapeutic to achieve the same therapeutic effect as seen with higher dosages. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "respond favorably" generally refers to causing a beneficial state in a subject. With respect to cancer treatment, the term refers to providing a therapeutic effect on the subject. Positive therapeutic effects in cancer can be measured in a number of ways (*See*, W.A. Weber, J. Nucl. Med. 50:1S-10S (2009)). For example, tumor growth inhibition, molecular marker expression, serum marker expression, and molecular imaging techniques can all be used to assess therapeutic efficacy of an anti-cancer therapeutic. With respect to tumor growth inhibition, according to NCI standards, a T/C $\leq$ 42% is the minimum level of anti-tumor activity. A T/C <10% is considered a high anti-tumor activity level, with T/C (%) = Median tumor volume of the treated / Median tumor volume of the control x 100. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP), a decrease in CA125 in the case of ovarian cancer or any combination thereof.

PFS, DFS, and OS can be measured by standards set by the National Cancer Institute and the U.S. Food and Drug Administration for the approval of new drugs. See Johnson et al, (2003) J. Clin. Oncol. 21(7):1404-1411.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

"Time to Tumor Progression" (TTP) is defined as the time from enrollment to disease progression. TTP is generally measured using the RECIST 1.1 criteria.

A "complete response" or "complete remission" or "CR" indicates the disappearance of all signs of tumor or cancer in response to treatment. This does not always mean the cancer has been cured.

A "partial response" or "PR" refers to a decrease in the size or volume of one or more tumors or lesions, or in the extent of cancer in the body, in response to treatment.

"Stable disease" refers to disease without progression or relapse. In stable disease there is neither sufficient

tumor shrinkage to qualify for partial response nor sufficient tumor increase to qualify as progressive disease.

"Progressive disease" refers to the appearance of one more new lesions or tumors and/or the unequivocal progression of existing non-target lesions. Progressive disease can also revert to a tumor growth of more than 20 percent since treatment began, either due to an increases in mass or in spread of the tumor.

"Disease free survival" (DFS) refers to the length of time during and after treatment that the patient remains free of disease.

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period of time, such as one year, five years, etc., e.g., from the time of diagnosis or treatment.

A "decrease in CA125 levels" can be assessed according to the Gynecologic Cancer Intergroup (GCIG) guidelines. For example, CA125 levels can be measured prior to treatment to establish a baseline CA125 level. CA125 levels can be measured one or more times during or after treatment, and a reduction in the CA125 levels over time as compared to the baseline level is considered a decrease in CA125 levels.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. Thus, those in need of treatment include those already diagnosed with or suspected of having the disorder. In certain embodiments, a subject is successfully "treated" for cancer according to the treatment of the present invention if the patient shows one or more of the following: a reduction in the number of or complete absence of cancer cells; a reduction in the tumor size; inhibition of or an absence of cancer cell infiltration into peripheral organs including, for example, the spread of cancer into soft tissue and bone; inhibition of or an absence of tumor metastasis; inhibition or an absence of tumor growth; relief of one or more symptoms associated with the specific cancer; reduced morbidity and mortality; improvement in quality of life; reduction in tumorigenicity, tumorigenic frequency, or tumorigenic capacity, of a tumor; reduction in the number or frequency of cancer stem cells in a tumor; differentiation of tumorigenic cells to a non-tumorigenic state; increased progression-free survival (PFS), disease-free survival (DFS), or overall survival (OS), complete response (CR), partial response (PR), stable disease (SD), a decrease in progressive disease (PD), a reduced time to progression (TTP), a decrease in CA125 in the case of ovarian cancer, or any combination thereof.

Prophylactic or preventative measures refer to therapeutic measures that prevent and/or slow the development of a targeted pathologic condition or disorder. Thus, those in need of prophylactic or preventative measures include those prone to have the disorder and those in whom the disorder is to be prevented.

The terms "pre-treat" and "pre-treatment" refer to therapeutic measures that occur prior to the administration of an anti-FOLR1 therapeutic. For example, as described in more detail herein, a prophylactic such as a steroid can administered within about a week, about five days, about three days, about two days, or about one day or 24 hours prior to the administration of the anti-FOLR1 therapeutic. The prophylactic can also be administered prior to the anti-FOLR1 therapeutic on the same day as the anti-FOLR1 therapeutic.

The term "maximum concentration" (Cmax) refers to the highest concentration of drug in the blood that is measured after a dose of the drug.

The term "area-under-the-curve" (AUC) refers to the overall amount of drug in the bloodstream after a dose of the drug. The AUC can be defined over a particular time period. Thus, for example, the $AUC_{0-\infty}$ refers to the overall amount of drug in the bloodstream for an infinite time after a dose of the drug. In another example, the $AUC_{0-24}$ refers to the overall amount of drug in the bloodstream for a period of 24 hours after a dose of the drug. In another example, the $AUC_{0-168}$ refers to the overall amount of drug in the bloodstream for a period of 168 hours (or 1 week) after a dose of the drug.

The "apparent volume of distribution at steady state" ($V_{ss}$) refers to the ratio of the total amount of drug in the body to the concentration of the drug in the plasma, or the "apparent" volume necessary to contain the entire amount of a drug, if the drug in the entire body were in the same concentration as in the plasma.

A "chemotherapeutic agent" is a chemical compound useful in the treatment of cancer, regardless of mechanism of action. Chemotherapeutic agents include, for example, antagonists of CD20 such as Rituximab and cyclophosphamide, doxorubicin, vincristine, predinisone, fludarabine, etoposide, methotrexate, lenalidomide, chlorambucil, bentamustine and/or modified versions of such chemotherapeutics.

The terms "polypeptide," "peptide," and "protein" are used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. It is understood that, because the polypeptides of this invention are based upon antibodies, in certain embodiments, the polypeptides can occur as single chains or associated chains.

The terms "identical" or percent "identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm described in Karlin et al, 1990, Proc. Natl. Acad. Sci., 87:2264-2268, as modified in Karlin et al., 1993, Proc. Natl. Acad. Sci., 90:5873-5877, and incorporated into the NBLAST and XBLAST programs (Altschul et al., 1991, Nucleic Acids Res., 25:3389-3402).Gapped BLAST can be used as described in Altschul et al., 1997, Nucleic Acids Res. 25:3389-3402. BLAST-2, WU-BLAST-2 (Altschul et al., 1996, Methods in Enzymology, 266:460-480), ALIGN, ALIGN-2 (Genentech, South San Francisco, California) or Megalign (DNASTAR) are additional publicly available software programs that can be used to align sequences. The percent identity between two nucleotide sequences may be determined using the GAP program in GCG software (e.g., using a NWSgapdna.CMP matrix and a gap weight of 40, 50, 60, 70, or 90 and a length weight of 1, 2, 3, 4, 5, or 6). The GAP program in the GCG software package, which incorporates the algorithm of Needleman and Wunsch (J. Mol. Biol. (48):444-453 (1970)) can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, the percent identity between nucleotide or amino acid sequences may be determined using the algorithm of Myers and Miller (CABIOS, 4:11-17 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. The default parameters of the alignment software may be used. The percentage identity "X" of a first amino acid sequence to a second sequence amino acid may be calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be longer than the percent identity of the second sequence to the first sequence.

As a non-limiting example, whether any particular polynucleotide has a certain percentage sequence identity (e.g., is at least 80% identical, at least 85% identical, at least 90% identical, or at least 95%, 96%, 97%, 98%, or 99% identical) to a reference sequence can be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2: 482 489 (1981), to find the best segment of homology between two sequences. When using Bestfit or any other sequence alignment program to determine whether a particular sequence is, for instance, 95% identical to a reference sequence, the parameters are set such that the percentage of identity is calculated over the full length of the reference nucleotide sequence and that gaps in homology of up to 5% of the total number of nucleotides in the reference sequence are allowed.

Two nucleic acids or polypeptides are substantially identical, meaning they have at least 70%, at least 75%, at least 80%, at least 85%, at least 90%, or at least 95%, 96%, 97%, 98%, 99% nucleotide or amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and the sequences may be substantially identical over the full length of the sequences being compared, such as the coding region of a nucleotide sequence for example.

A "conservative amino acid substitution" is one in which one amino acid residue is replaced with another amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). For example, substitution of a phenylalanine for a tyrosine is a conservative substitution. Conservative substitutions in the sequences of the polypeptides and antibodies may not abrogate the binding of the polypeptide or antibody containing the amino acid sequence, to the antigen(s), i.e., the FOLR1 to which the polypeptide or antibody binds. Methods of identifying nucleotide and amino acid conservative substitutions which do not eliminate antigen binding are well- known in the art (see, e.g., Brummell et al., Biochem. 32: 1180-1 187 (1993); Kobayashi et al. Protein Eng. 12(10):879-884 (1999); and Burks et al. Proc. Natl. Acad. Sci. USA 94:.412-417 (1997)).

As used in the present disclosure and claims, the singular forms "a," "an," and "the" include plural forms unless the context clearly dictates otherwise.

It is understood that wherever embodiments are described herein with the language "comprising", otherwise

analogous embodiments described in terms of "consisting of" and/or "consisting essentially of' are also provided.

The term "and/or" as used in a phrase such as "A and/or B" herein is intended to include both "A and B," "A or B," "A," and "B." Likewise, the term "and/or" as used in a phrase such as "A, B, and/or C" is intended to encompass each of the following: A, B, and C; A, B, or C; A or C; A or B; B or C; A and C; A and B; B and C; A (alone); B (alone); and C (alone).

## II. FOLR1 binding agents

The methods described herein provide methods of administering agents (e.g., antibodies or antigen-binding fragments thereof or polypeptides) that specifically bind FOLR1 ("FOLR1 binding agents"). The FOLR1 binding agents may be antibodies, immunoconjugates or polypeptides. The amino acid and nucleotide sequences for human FOLR1 are known in the art and are also provided herein as represented by SEQ ID NO:1 and SEQ ID NO:2. Thus, the FOLR1 binding agents can bind to an epitope found within SEQ ID NO:1 . The immunoconjugate of the present invention comprises a maytansinoid and an antibody or antigen-binding fragment thereof as the FOLR1 binding agent that comprises the variable light chain (VL) complementarity determining region (CDR)-1 of SEQ ID NO: 6, the VL CDR-2 of SEQ ID NO: 7, the VL CDR-3 of SEQ ID NO: 8, the variable heavy chain (VH) CDR-1 of SEQ ID NO: 9, the VH CDR-2 of SEQ ID NO: 11, and the VH CDR-3 of SEQ ID NO: 12.

Examples of therapeutically effective anti-FOLR1 antibodies can be found in US Appl. Pub. No. US 2012/0009181. An example of a therapeutically effective anti-FOLR1 antibody is huMov19 (M9346A). The polypeptides of SEQ ID NOs: 3-5 comprise the variable domain of the heavy chain of huMov19 (M9346A), and the variable domain light chain version 1.00, the variable domain light chain version 1.60 of huMov19, respectively. In certain embodiments, the huMov19 anti-FOLR1 antibody is comprised of a variable domain heavy chain represented by SEQ ID NO:3 and a variable domain light chain represented by SEQ ID NO:5 (version 1.60 of huMov19). In certain embodiments, the huMov19 (M9346A) antibody is encoded by the plasmids deposited with the American Type Culture Collection (ATCC), located at 10801 University Boulevard, Manassas, VA 20110 on April 7, 2010 under the terms of the Budapest Treaty and having ATCC deposit nos. PTA-10772 and PTA-10773 or 10774. Examples of FOLR1 immunoconjugates useful in the therapeutic methods of the invention are provided below.

In some embodiments, the FOLR1 binding agents of the invention are humanized antibodies or antigen-binding fragments thereof. In some embodiments, the humanized antibody or fragment is a resurfaced antibody or antigen-binding fragment thereof. In other embodiments, the FOLR1 binding agent is a fully human antibody or antigen-binding fragment thereof.

In certain embodiments, the FOLR1-binding agents of the invention have one or more of the following effects: induce stable disease, inhibit proliferation of tumor cells, reduce the tumorigenicity of a tumor by reducing the frequency of cancer stem cells in the tumor, inhibit tumor growth, increase survival, trigger cell death of tumor cells, differentiate tumorigenic cells to a non-tumorigenic state, or prevent metastasis of tumor cells.

In certain embodiments, a FOLR1-binding agent of the invention that is an antibody that has antibody-dependent cellular cytotoxicity (ADCC) activity.

In some embodiments, the FOLR1-binding agents of the invention are capable of reducing tumor volume. The ability of a FOLR1-binding agent to reduce tumor volume can be assessed, for example, by measuring a %T/C value, which is the median tumor volume of treated subjects divided by the median tumor volume of the control subjects. In certain embodiments, immunoconjugates that specifically bind human FOLR1 trigger cell death via a cytotoxic agent. For example, in certain embodiments, an antibody to a human FOLR1 antibody is conjugated to a maytansinoid that is activated in tumor cells expressing the FOLR1 by protein internalization. In certain embodiments, the FOLR1-binding agents are capable of inhibiting tumor growth. In certain embodiments, the FOLR1-binding agents are capable of inhibiting tumor growth *in vivo* (e.g., in a xenograft mouse model and/or in a human having cancer).

The FOLR1 binding molecules as described herein can be antibodies or antigen binding fragments that specifically bind to FOLR1 that comprise the CDRs of huMov19 (M9346A) with up to four (i.e. 0, 1, 2, 3, or 4) conservative amino acid substitutions per CDR, e.g., wherein the antibodies or fragments do not comprise the six CDRs of murine Mov19 (i.e., SEQ ID NOs:6-9, 16, and 12). Polypeptides can comprise one of the individual variable light chains or variable heavy chains described herein. Antibodies and polypeptides can also comprise both a variable light chain and a variable heavy chain.

The FOLR1 binding molecule described herein is an antibody or antigen-binding fragment comprising the sequences of SEQ ID NOs:6-10 and the sequence of SEQ ID NO:12. According to the invention, the FOLR1 binding molecule is an antibody or antigen-binding fragment comprising the sequences of SEQ ID NOs:6-9 and the sequences of SEQ ID NOs:11 and 12

Also provided are polypeptides that comprise a polypeptide having at least about 90% sequence identity to SEQ ID NO:3, SEQ ID NO:4 or SEQ ID NO:5. In certain embodiments, the polypeptide comprises a polypeptide having at least about 95%, at least about 96%, at least about 97%, at least about 98%, or at least about 99% sequence identity

to SEQ ID NO:3, SEQ ID NO:4 or SEQ ID NO:5 Thus, in certain embodiments, the polypeptide comprises (a) a polypeptide having at least about 95% sequence identity to SEQ ID NO:3 and/or (b) a polypeptide having at least about 95% sequence identity to SEQ ID NO:4 or SEQ ID NO:5. In certain embodiments, the polypeptide comprises (a) a polypeptide having the amino acid sequence of SEQ ID NO:3; and/or (b) a polypeptide having the amino acid sequence of SEQ ID NO:4 or SEQ ID NO:5. Thereby, the polypeptide is an antibody or antigen-binding fragment thereof. In certain embodiments, the polypeptide is a murine, chimeric, or humanized antibody that specifically binds FOLR1. In certain embodiments, the polypeptide having a certain percentage of sequence identity to SEQ ID NO:3, SEQ ID NO:4 or SEQ ID NO:5 differs from SEQ ID NO:3, SEQ ID NO:4 or SEQ ID NO:5 by conservative amino acid substitutions only.

As described herein, polypeptides can comprise one of the individual light chains or heavy chains described herein. Antibodies and polypeptides can also comprise both a light chain and a heavy chain.

Monoclonal antibodies can be prepared using hybridoma methods, such as those described by Köhler and Milstein (1975) Nature 256:495. Using the hybridoma method, a mouse, hamster, or other appropriate host animal, is immunized as described above to elicit the production by lymphocytes of antibodies that will specifically bind to an immunizing antigen. Lymphocytes can also be immunized *in vitro.* Following immunization, the lymphocytes are isolated and fused with a suitable myeloma cell line using, for example, polyethylene glycol, to form hybridoma cells that can then be selected away from unfused lymphocytes and myeloma cells. Hybridomas that produce monoclonal antibodies directed specifically against a chosen antigen as determined by immunoprecipitation, immunoblotting, or by an *in vitro* binding assay (e.g. radioimmunoassay (RIA); enzyme-linked immunosorbent assay (ELISA)) can then be propagated either *in vitro* culture using standard methods (Goding, Monoclonal Antibodies: Principles and Practice, Academic Press, 1986) or *in vivo* as ascites tumors in an animal. The monoclonal antibodies can then be purified from the culture medium or ascites fluid as described for polyclonal antibodies above.

Alternatively monoclonal antibodies can also be made using recombinant DNA methods as described in U.S. Patent 4,816,567. The polynucleotides encoding a monoclonal antibody are isolated from mature B-cells or hybridoma cell, such as by RT-PCR using oligonucleotide primers that specifically amplify the genes encoding the heavy and light chains of the antibody, and their sequence is determined using conventional procedures. The isolated polynucleotides encoding the heavy and light chains are then cloned into suitable expression vectors, which when transfected into host cells such as E. coli cells, simian COS cells, Chinese hamster ovary (CHO) cells, or myeloma cells that do not otherwise produce immunoglobulin protein, monoclonal antibodies are generated by the host cells. Also, recombinant monoclonal antibodies or fragments thereof of the desired species can be isolated from phage display libraries expressing CDRs of the desired species as described (McCafferty et al., 1990, Nature, 348:552-554; Clackson et al., 1991, Nature, 352:624-628; and Marks et al., 1991, J. Mol. Biol., 222:581-597).

The polynucleotide(s) encoding a monoclonal antibody can further be modified in a number of different manners using recombinant DNA technology to generate alternative antibodies. In some embodiments, the constant domains of the light and heavy chains of, for example, a mouse monoclonal antibody can be substituted 1) for those regions of, for example, a human antibody to generate a chimeric antibody or 2) for a non-immunoglobulin polypeptide to generate a fusion antibody. In some embodiments, the constant regions are truncated or removed to generate the desired antibody fragment of a monoclonal antibody. Site-directed or high-density mutagenesis of the variable region can be used to optimize specificity, affinity, etc. of a monoclonal antibody.

In some embodiments, the monoclonal antibody against the human FOLR1 is a humanized antibody. In some embodiments, the humanized antibody is a resurfaced antibody. In certain embodiments, such antibodies are used therapeutically to reduce antigenicity and HAMA (human antimouse antibody) responses when administered to a human subject. Humanized antibodies can be produced using various techniques known in the art. In certain alternative embodiments, the antibody to FOLR1 is a human antibody.

Human antibodies can be directly prepared using various techniques known in the art. Immortalized human B lymphocytes immunized *in vitro* or isolated from an immunized individual that produce an antibody directed against a target antigen can be generated (See, e.g., Cole et al., Monoclonal Antibodies and Cancer Therapy, Alan R. Liss, p. 77 (1985); Boemer et al., 1991, J. Immunol., 147 (1):86-95; and U.S. Patent 5,750,373). Also, the human antibody can be selected from a phage library, where that phage library expresses human antibodies, as described, for example, in Vaughan et al., 1996, Nat. Biotech., 14:309-314, Sheets et al., 1998, Proc. Nat'l. Acad. Sci., 95:6157-6162, Hoogenboom and Winter, 1991, J. Mol. Biol., 227:381, and Marks et al., 1991, J. Mol. Biol., 222:581). Techniques for the generation and use of antibody phage libraries are also described in U.S. Patent Nos. 5,969,108, 6,172,197, 5,885,793, 6,521,404; 6,544,731; 6,555,313; 6,582,915; 6,593,081; 6,300,064; 6,653,068; 6,706,484; and 7,264,963; and Rothe et al., 2007, J. Mol. Bio., doi:10.1016/j.jmb.2007.12.018. Affinity maturation strategies and chain shuffling strategies (Marks et al., 1992, Bio/Technology 10:779-783) are known in the art and can be employed to generate high affinity human antibodies.

Humanized antibodies can also be made in transgenic mice containing human immunoglobulin loci that are capable upon immunization of producing the full repertoire of human antibodies in the absence of endogenous immunoglobulin production. This approach is described in U.S. Patents 5,545,807; 5,545,806; 5,569,825; 5,625,126; 5,633,425; and 5,661,016.

This invention also encompasses bispecific antibodies that specifically recognize a FOLR1. Bispecific antibodies are antibodies that are capable of specifically recognizing and binding at least two different epitopes. The different epitopes can either be within the same molecule (e.g. the same FOLR1) or on different molecules such that both, for example, the antibodies can specifically recognize and bind a FOLR1 as well as, for example, 1) an effector molecule on a leukocyte such as a T-cell receptor (e.g. CD3) or Fc receptor (e.g. CD64, CD32, or CD16) or 2) a cytotoxic agent as described in detail below.

The polypeptides can be recombinant polypeptides, natural polypeptides, or synthetic polypeptides comprising an antibody, or fragment thereof, against a human FOLR1.

The polypeptides and analogs can be further modified to contain additional chemical moieties not normally part of the protein. Those derivatized moieties can improve the solubility, the biological half life or absorption of the protein. The moieties can also reduce or eliminate any desirable side effects of the proteins and the like. An overview for those moieties can be found in REMINGTON'S PHARMACEUTICAL SCIENCES, 20th ed., Mack Publishing Co., Easton, PA (2000).

Methods known in the art for purifying antibodies and other proteins also include, for example, those described in U.S. Patent Publication No. 2008/0312425, 2008/0177048, and 2009/0187005.

**III. Immunoconjugates**

Methods for administering conjugates comprising the anti-FOLR1 antibodies, antibody fragments, and their functional equivalents as disclosed herein, linked or conjugated to a drug or prodrug (also referred to herein as immunoconjugates) are also described herein. Suitable drugs or prodrugs are known in the art. The drugs or prodrugs can be cytotoxic agents. The cytotoxic agent used in the cytotoxic conjugate of the present invention can be any compound that results in the death of a cell, or induces cell death, or in some manner decreases cell viability, and includes, for example, maytansinoids and maytansinoid analogs. Other suitable cytotoxic agents are for example benzodiazepines, taxoids, CC-1065 and CC-1065 analogs, duocarmycins and duocarmycin analogs, enediynes, such as calicheamicins, dolastatin and dolastatin analogs including auristatins, tomaymycin derivaties, leptomycin derivaties, methotrexate, cisplatin, carboplatin, daunorubicin, doxorubicin, vincristine, vinblastine, melphalan, mitomycin C, chlorambucil and morpholino doxorubicin.

Such conjugates can be prepared by using a linking group in order to link a drug or prodrug to the antibody or functional equivalent. Suitable linking groups are well known in the art and include, for example, disulfide groups, thioether groups, acid labile groups, photolabile groups, peptidase labile groups and esterase labile groups.

The drug or prodrug can, for example, be linked to the anti-FOLRl antibody or fragment thereof through a disulfide bond. The linker molecule or crosslinking agent comprises a reactive chemical group that can react with the anti-FOLR1 antibody or fragment thereof. The reactive chemical groups for reaction with the cell-binding agent can be *N*-succinimidyl esters and *N*-sulfosuccinimidyl esters. Additionally the linker molecule comprises a reactive chemical group, which can be a dithiopyridyl group that can react with the drug to form a disulfide bond. Linker molecules include, for example, *N*-succinimidyl 3-(2-pyridyldithio) propionate (SPDP) (see, e.g., Carlsson et al., Biochem. J., 173: 723-737 (1978)), *N*-succinimidyl 4-(2-pyridyldithio)butanoate (SPDB) (see, e.g., U.S. Patent No. 4,563,304), *N*-succinimidyl 4-(2-pyridyldithio)2-sulfobutanoate (sulfo-SPDB) (see US Publication No. 20090274713), *N*-succinimidyl 4-(2-pyridyldithio) pentanoate (SPP) (see, e.g., CAS Registry number 341498-08-6), 2-iminothiolane, or acetylsuccinic anhydride. For example, the antibody or cell binding agent can be modified with crosslinking reagents and the antibody or cell binding agent containing free or protected thiol groups thus derived is then reacted with a disulfide- or thiol-containing maytansinoid to produce conjugates. The conjugates can be purified by chromatography, including but not limited to HPLC, size-exclusion, adsorption, ion exchange and affinity capture, dialysis or tangential flow filtration.

In another aspect of the present invention, the anti-FOLRl antibody is linked to cytotoxic drugs via disulfide bonds and a polyethylene glycol spacer in enhancing the potency, solubility or the efficacy of the immunoconjugate. Such cleavable hydrophilic linkers are described in WO2009/0134976. The additional benefit of this linker design is the desired high monomer ratio and the minimal aggregation of the antibody-drug conjugate. Specifically contemplated in this aspect are conjugates of cell-binding agents and drugs linked via disulfide group (-S-S-) bearing polyethylene glycol spacers ($(CH_2CH_2O)_{n=1-14}$) with a narrow range of drug load of 2-8 are described that show relatively high potent biological activity toward cancer cells and have the desired biochemical properties of high conjugation yield and high monomer ratio with minimal protein aggregation.

Antibody-maytansinoid conjugates with non-cleavable linkers can also be prepared. Such crosslinkers are described in the art (see US Publication No. 20050169933) and include but are not limited to, *N*-succinimidyl 4-(maleimidomethyl) cyclohexanecarboxylate (SMCC). In some embodiments, the antibody is modified with crosslinking reagents such as succinimidyl 4-(N-maleimidomethyl)-cyclohexane-1-carboxylate (SMCC), sulfo-SMCC, maleimidobenzoyl-N-hydroxysuccinimide ester (MBS), sulfo-MBS or succinimidyl-iodoacetate, as described in the literature, to introduce 1-10 reactive groups (Yoshitake et al, Eur. J. Biochem., 101:395-399 (1979); Hashida et al, J. Applied Biochem., 56-63 (1984);

and Liu et al, Biochem., 18:690-697 (1979)). The modified antibody is then reacted with the thiol-containing maytansinoid derivative to produce a conjugate. The conjugate can be purified by gel filtration through a Sephadex G25 column or by dialysis or tangential flow filtration. The modified antibodies are treated with the thiol-containing maytansinoid (1 to 2 molar equivalent/maleimido group) and antibody-maytansinoid conjugates are purified by gel filtration through a Sephadex G-25 column, chromatography on a ceramic hydroxyapatite column, dialysis or tangential flow filtration or a combination of methods thereof. Typically, an average of 1-10 maytansinoids per antibody are linked. One method is to modify antibodies with succinimidyl 4-(N-maleimidomethyl)-cyclohexane-1-carboxylate (SMCC) to introduce maleimido groups followed by reaction of the modified antibody with a thiol-containing maytansinoid to give a thioether-linked conjugate. Again conjugates with 1 to 10 drug molecules per antibody molecule result. Maytansinoid conjugates of antibodies, antibody fragments, and other proteins are made in the same way.

In another aspect of the invention, the FOLR1 antibody is linked to the drug via a non-cleavable bond through the intermediacy of a PEG spacer. Suitable crosslinking reagents comprising hydrophilic PEG chains that form linkers between a drug and the anti-FOLR1 antibody or fragment are also well known in the art, or are commercially available (for example from Quanta Biodesign, Powell, Ohio). Suitable PEG-containing crosslinkers can also be synthesized from commercially available PEGs themselves using standard synthetic chemistry techniques known to one skilled in the art. The drugs can be reacted with bifunctional PEG-containing cross linkers to give compounds of the following formula, Z $-X_1-(-CH_2-CH_2-O-)_n-Y_p-D$, by methods described in detail in US Patent Publication 20090274713 and in WO2009/0134976, which can then react with the cell binding agent to provide a conjugate. Alternatively, the cell binding can be modified with the bifunctional PEG crosslinker to introduce a thiol-reactive group (such as a maleimide or haloacetamide) which can then be treated with a thiol-containing maytansinoid to provide a conjugate. In another method, the cell binding can be modified with the bifunctional PEG crosslinker to introduce a thiol moiety which can then be treated with a thiol-reactive maytansinoid (such as a maytansinoid bearing a maleimide or haloacetamide), to provide a conjugate.

Examples of suitable PEG-containing linkers include linkers having an *N*-succinimidyl ester or *N*-sulfosuccinimidyl ester moiety for reaction with the anti-FOLR1 antibody or fragment thereof, as well as a maleimido- or haloacetyl-based moiety for reaction with the compound. A PEG spacer can be incorporated into any crosslinker known in the art by the methods described herein.

In some embodiments, the linker is a linker containing at least one charged group as described, for example, in U.S. Patent Publication No. 2012/0282282. In some embodiments, the charged or pro-charged cross-linkers are those containing sulfonate, phosphate, carboxyl or quaternary amine substituents that significantly increase the solubility of the modified cell-binding agent and the cell-binding agent-drug conjugates, especially for monoclonal antibody-drug conjugates with 2 to 20 drugs/antibody linked. Conjugates prepared from linkers containing a pro-charged moiety would produce one or more charged moieties after the conjugate is metabolized in a cell. In some embodiments, the linker is selected from the group consisting of: N-succinimidyl 4-(2-pyridyldithio)-2-sulfopentanoate (sulfo-SPP) and N-succinimidyl 4-(2-pyridyldithio)-2-sulfobutanoate (sulfo-SPDB).

Many of the linkers disclosed herein are described in detail in U.S. Patent Publication Nos. 2005/0169933, 2009/0274713, and 2012/0282282, and in WO2009/0134976.

The present invention includes aspects wherein about 2 to about 8 drug molecules ("drug load"), for example, maytansinoid, are linked to an anti-FOLRl antibody or fragment thereof. "Drug load", as used herein, refers to the number of drug molecules (e.g., a maytansinoid) that can be attached to a cell binding agent (e.g., an anti-FOLRl antibody or fragment thereof). In one aspect, the number of drug molecules that can be attached to a cell binding agent can average from about 2 to about 8 (e.g., 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1). N2'-deacetyl-N2'-(3-mercapto-1-oxopropyl)-maytansine (DM1) and N2'-deacetyl-N2'-(4-mercapto-4-methyl-1-oxopentyl) maytansine (DM4) can be used.

Thus, in one aspect, an immunoconjugate comprises 1 maytansinoid per antibody. In another aspect, an immunoconjugate comprises 2 maytansinoids per antibody. In another aspect, an immunoconjugate comprises 3 maytansinoids per antibody. In another aspect, an immunoconjugate comprises 4 maytansinoids per antibody. In another aspect, an immunoconjugate comprises 5 maytansinoids per antibody. In another aspect, an immunoconjugate comprises 6 maytansinoids per antibody. In another aspect, an immunoconjugate comprises 7 maytansinoids per antibody. In another aspect, an immunoconjugate comprises 8 maytansinoids per antibody.

In one aspect, an immunoconjugate comprises about 1 to about 8 maytansinoids per antibody. In another aspect, an immunoconjugate comprises about 2 to about 7 maytansinoids per antibody. In another aspect, an immunoconjugate comprises about 2 to about 6 maytansinoids per antibody. In another aspect, an immunoconjugate comprises about 2 to about 5 maytansinoids per antibody. In another aspect, an immunoconjugate comprises about 3 to about 5 maytansinoids per antibody. In another aspect, an immunoconjugate comprises about 3 to about 4 maytansinoids per antibody.

In one aspect, a composition comprising immunoconjugates has an average of about 2 to about 8 (e.g., 1.9,

2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1) drug molecules (e.g., maytansinoids) attached per antibody. In one aspect, a composition comprising immunoconjugates has an average of about 1 to about 8 drug molecules (e.g., maytansinoids) per antibody. In one aspect, a composition comprising immunoconjugates has an average of about 2 to about 7 drug molecules (e.g., maytansinoids) per antibody. In one aspect, a composition comprising immunoconjugates has an average of about 2 to about 6 drug molecules (e.g., maytansinoids) per antibody. In one aspect, a composition comprising immunoconjugates has an average of about 2 to about 5 drug molecules (e.g., maytansinoids) per antibody. In one aspect, a composition comprising immunoconjugates has an average of about 3 to about 5 drug molecules (e.g., maytansinoids) per antibody. In one aspect, a composition comprising immunoconjugates has an average of about 3 to about 4 drug molecules (e.g., maytansinoids) per antibody.

In one aspect, a composition comprising immunoconjugates has an average of about 2 $\pm$ 0.5, about 3 $\pm$ 0.5, about 4 $\pm$ 0.5, about 5 $\pm$ 0.5, about 6 $\pm$ 0.5, about 7 $\pm$ 0.5, or about 8 $\pm$ 0.5 drug molecules (e.g., maytansinoids) attached per antibody. In one aspect, a composition comprising immunoconjugates has an average of about 3.5 $\pm$ 0.5 drug molecules (e.g., maytansinoids) per antibody.

The anti-FOLRI antibody or fragment thereof can be modified by reacting a bifunctional crosslinking reagent with the anti-FOLRI antibody or fragment thereof, thereby resulting in the covalent attachment of a linker molecule to the anti-FOLRI antibody or fragment thereof. As used herein, a "bifunctional crosslinking reagent" is any chemical moiety that covalently links a cell-binding agent to a drug, such as the drugs described herein. In another method, a portion of the linking moiety is provided by the drug. In this respect, the drug comprises a linking moiety that is part of a larger linker molecule that is used to join the cell-binding agent to the drug. For example, to form the maytansinoid DM1, the side chain at the C-3 hydroxyl group of maytansine is modified to have a free sulfhydryl group (SH). This thiolated form of maytansine can react with a modified cell-binding agent to form a conjugate. Therefore, the final linker is assembled from two components, one of which is provided by the crosslinking reagent, while the other is provided by the side chain from DM1.

The drug molecules can also be linked to the antibody molecules through an intermediary carrier molecule such as serum albumin.

As used herein, the expression "linked to a cell-binding agent" or "linked to an anti-FOLRI antibody or fragment" refers to the conjugate molecule comprising at least one drug derivative bound to a cell-binding agent anti-FOLRI antibody or fragment via a suitable linking group, or a precursor thereof. Exemplary linking groups are SPDB or sulfo-SPDB.

In certain embodiments, cytotoxic agents useful in the present invention are maytansinoids and maytansinoid analogs. Examples of suitable maytansinoids include esters of maytansinol and maytansinol analogs. Included are any drugs that inhibit microtubule formation and that are highly toxic to mammalian cells, as are maytansinol and maytansinol analogs.

Examples of suitable maytansinol esters include those having a modified aromatic ring and those having modifications at other positions. Such suitable maytansinoids are disclosed in U.S. Patent Nos. 4,424,219; 4,256,746; 4,294,757; 4,307,016; 4,313,946; 4,315,929; 4,331,598; 4,361,650; 4,362,663; 4,364,866; 4,450,254; 4,322,348; 4,371,533; 5,208,020; 5,416,064; 5,475,092; 5,585,499; 5,846,545; 6,333,410; 7,276,497 and 7,473,796.

In a certain embodiment, the immunoconjugates of the invention utilize the thiol-containing maytansinoid (DM1), formally termed *N*$^{2'}$-deacetyl-*N*$^{2'}$-(3-mercapto-1-oxopropyl)-maytansine, as the cytotoxic agent. DM1 is represented by the following structural formula (I):

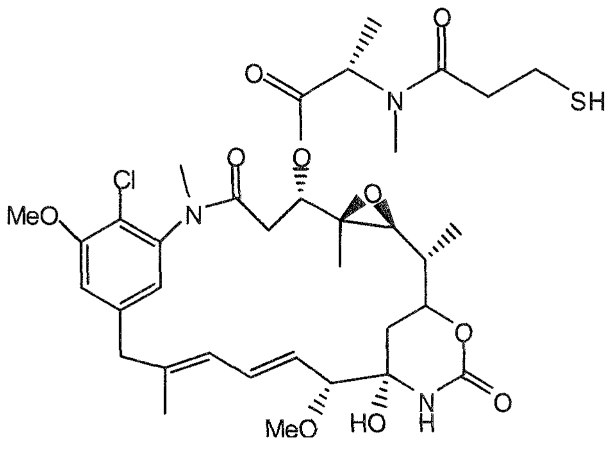

(I)

In another embodiment, the conjugates of the present invention utilize the thiol-containing maytansinoid *N*$^{2'}$-deacetyl-*N*$^{2'}$(4-methyl -4-mercapto-1-oxopentyl)-maytansine (e.g., DM4) as the cytotoxic agent. DM4 is represented by the following structural formula (II):

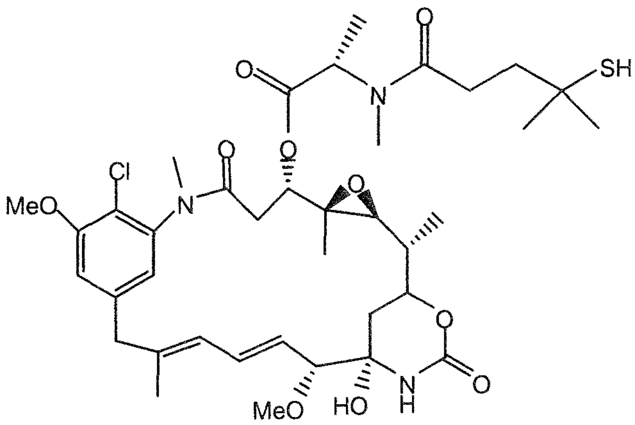

(II)

Another maytansinoid comprising a side chain that contains a sterically hindered thiol bond is *N*$^{2'}$-deacetyl-*N*-$^{2'}$(4-mercapto-1-oxopentyl)-maytansine (termed DM3), represented by the following structural formula (III):

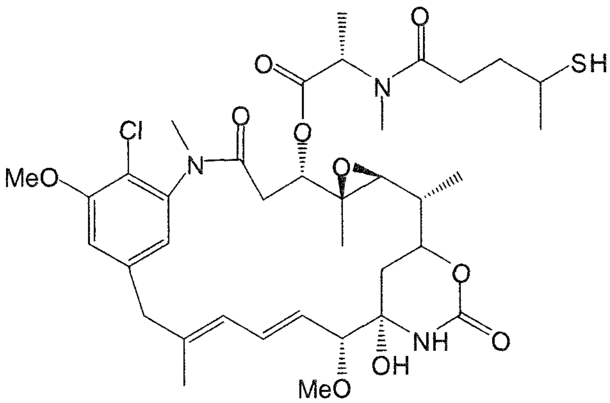

(III)

Each of the maytansinoids taught in US Patent No. 5,208,020 and 7,276,497, can also be used in the conjugate of the present invention.

Many positions on maytansinoids can serve as the position to chemically link the linking moiety. For example, the C-3 position having a hydroxyl group, the C-14 position modified with hydroxymethyl, the C-15 position modified with hydroxy and the C-20 position having a hydroxy group are all expected to be useful. In some embodiments, the C-3 position serves as the position to chemically link the linking moiety, and in some particular embodiments, the C-3 position of maytansinol serves as the position to chemically link the linking moiety.

Structural representations of some conjugates are shown below:

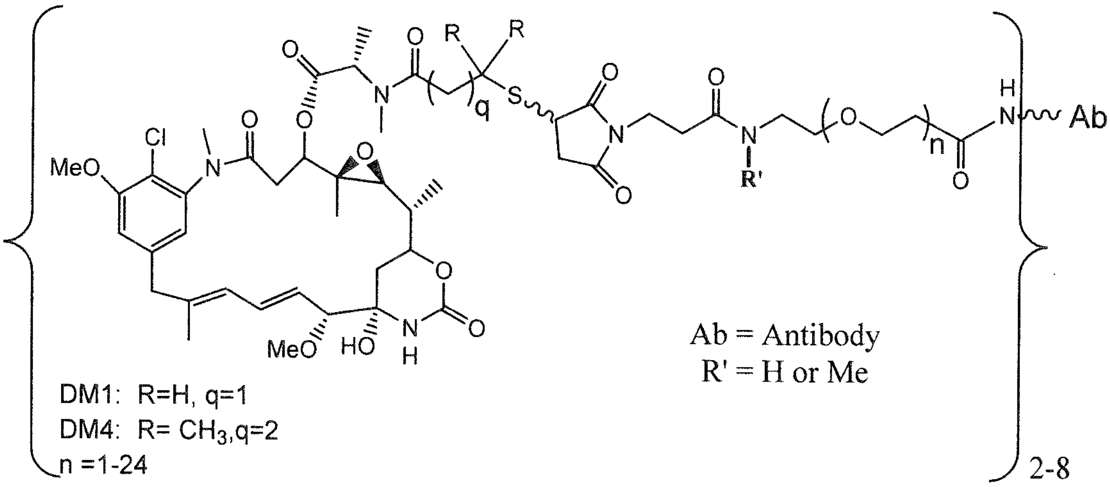

Ab = Antibody
R' = H or Me

DM1: R=H, q=1
DM4: R= $CH_3$,q=2
n =1-24

Ab-PEG-Mal-DM1/DM4

(IV)

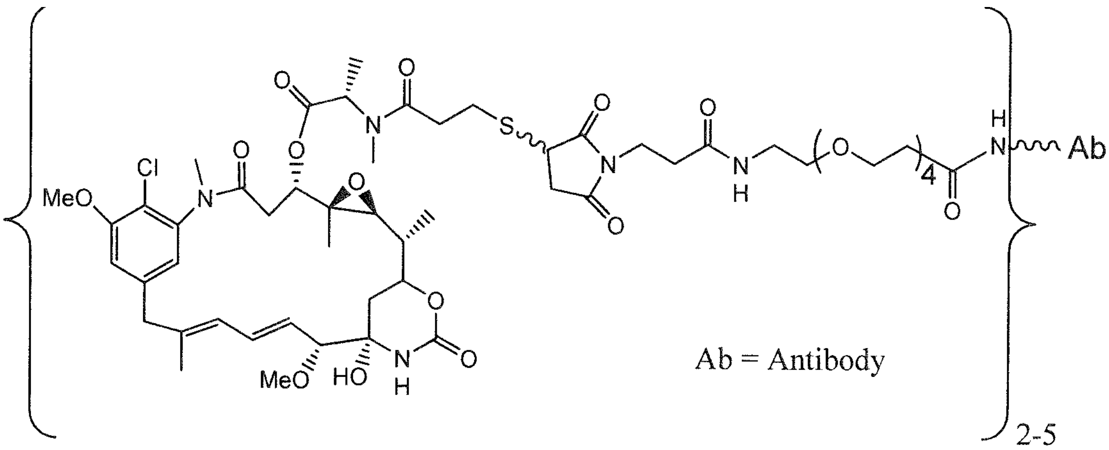

Ab = Antibody

Ab-PEG4-Mal-DM1

(V)

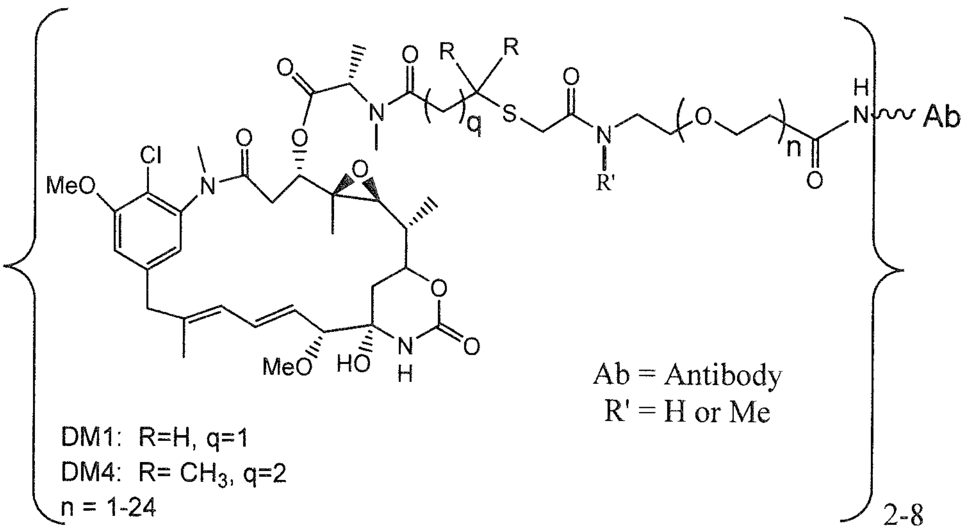

Ab = Antibody
R' = H or Me

DM1: R=H, q=1
DM4: R= $CH_3$, q=2
n = 1-24

Ab-PEG-SIA-DM1/DM4

(VI)

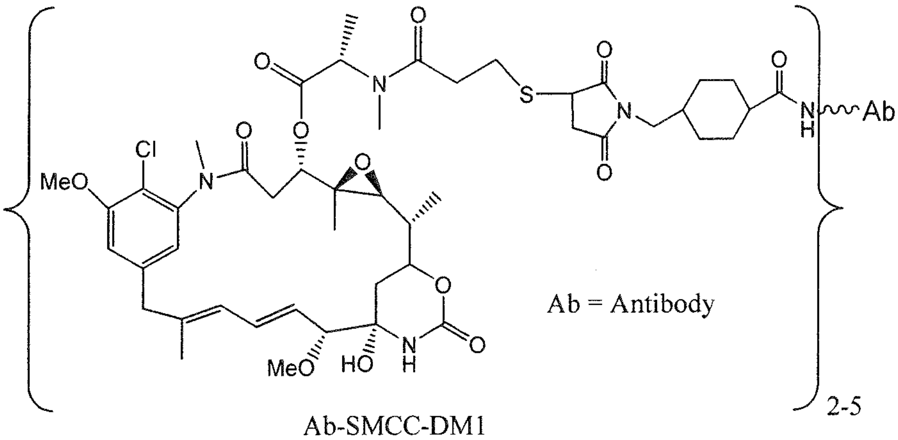


Ab-SMCC-DM1

(VII)

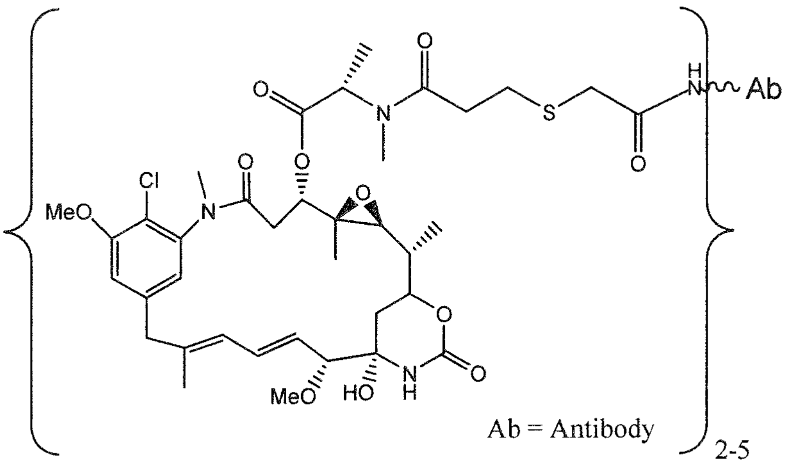


Ab-SIA-DM1

(VIII)

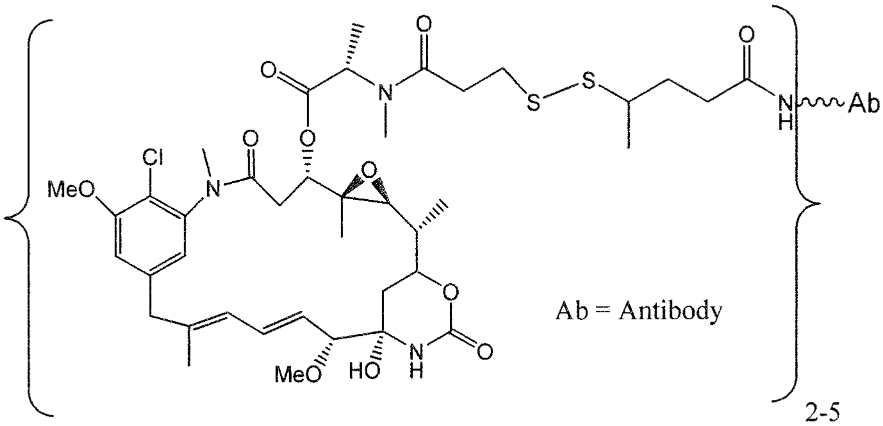


Ab-SPP-DM1

(IX)

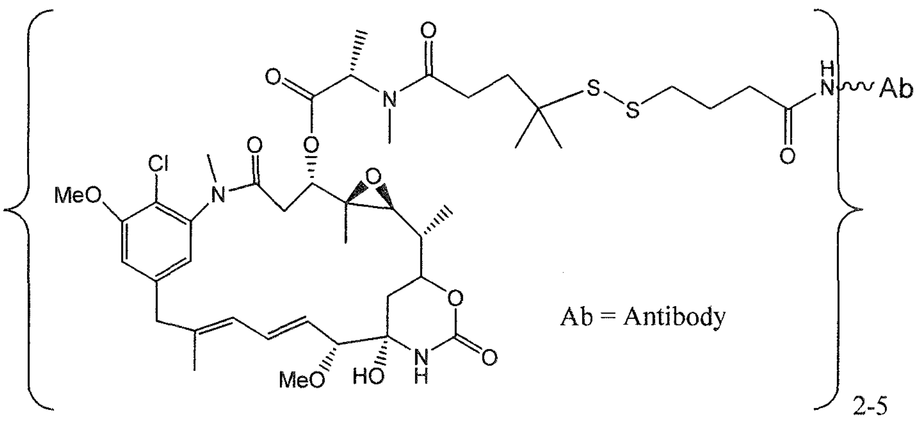

Ab-SPDB-DM4

(X)

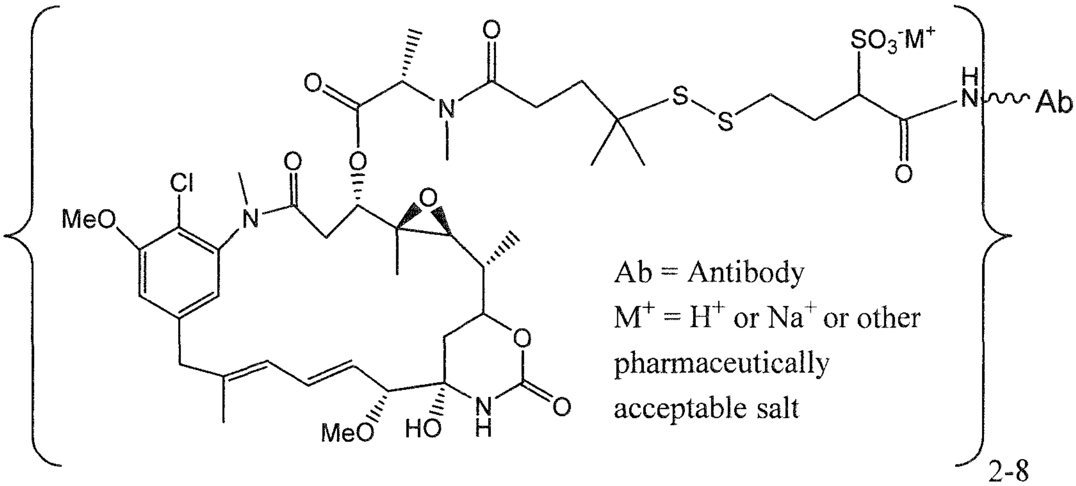

Ab-sulfo-SPDB-DM4

(XI)

Described are any stereoisomers and mixtures thereof for any compounds or conjugates depicted by any structures above.

Several descriptions for producing such antibody-maytansinoid conjugates are provided in U.S. Patent Nos. 6,333,410, 6,441,163, 6,716,821, and 7,368,565.

In general, a solution of an antibody in aqueous buffer can be incubated with a molar excess of maytansinoids having a disulfide moiety that bears a reactive group. The reaction mixture can be quenched by addition of excess amine (such as ethanolamine, taurine, etc.). The maytansinoid-antibody conjugate can then be purified by gel filtration.

The number of maytansinoid molecules bound per antibody molecule can be determined by measuring spectrophotometrically the ratio of the absorbance at 252 nm and 280 nm. The average number of maytansinoid molecules/antibody can be, for example, 1-10 or 2-5. The average number of maytansinoid molecules/antibody can be, for example about 3 to about 4. The average number of maytansinoid molecules/antibody can be about 3.5.

Conjugates of antibodies with maytansinoid or other drugs can be evaluated for their ability to suppress proliferation of various unwanted cell lines *in vitro.* For example, cell lines such as the human lymphoma cell line Daudi and the human lymphoma cell line Ramos, can easily be used for the assessment of cytotoxicity of these compounds. Cells to be evaluated can be exposed to the compounds for 4 to 5 days and the surviving fractions of cells measured in direct assays by known methods. $IC_{50}$ values can then be calculated from the results of the assays.

The immunoconjugates can be internalized into cells. The immunoconjugate, therefore, can exert a therapeutic effect when it is taken up by, or internalized, by a FOLR1-expressing cell. Thereby, the immunoconjugate comprises an antibody, antibody fragment, or polypeptide, linked to a cytotoxic agent by a cleavable linker, and the cytotoxic agent is cleaved from the antibody, antibody fragment, or polypeptide, wherein it is internalized by a FOLR1-expressing cell.

In some embodiments, the immunoconjugates are capable of reducing tumor volume. For example, in some embodiments, treatment with an immunoconjugate results in a %T/C value that is less than about 50%, less than about

45%, less than about 40%, less than about 35%, less than about 30%, less than about 25%, less than about 20%, less than about 15%, less than about 10%, or less than about 5%. In some particular embodiments, the immunoconjugates can reduce tumor size in a KB, OVCAR-3, IGROV-1, and/or OV-90 xenograft model. In some embodiments, the immunoconjugates are capable of inhibiting metastases.

## III. **Methods of administering FOLR1-binding agents**

The FOLR1-binding agents (including antibodies, immunoconjugates, and polypeptides) are useful in a variety of applications including, but not limited to, therapeutic treatment methods, such as the treatment of cancer. The agents may be useful for inhibiting tumor growth, inducing differentiation, inhibiting metastases, reducing tumor volume, and/or reducing the tumorigenicity of a tumor. The methods of use can be *in vivo* methods.

Described is that the FOLR1 -binding agents can be administered at particular dosages. For example, the FOLR1-binding agents (e.g., IMGN853) can be administered at a dose of about 0.15 mg/kg to about 7 mg/kg, wherein the kilograms of body weight are adjusted to ideal body weight (IBW), lean body weight (LBW), or adjusted ideal body weight (AIBW or ADJ). The FOLR1-binding agents (e.g., IMGN853) may be administered at a dose of about 3.0 mg/kg to about 6.0 mg/kg, wherein the kilograms of body weight are adjusted to IBW, LBW, or AIBW (ADJ). The FOLR1-binding agents (e.g., IMGN853) may be administered at a dose of about 3.3 mg/kg to about 6.0 mg/kg, wherein the kilograms of body weight are adjusted to IBW, LBW, or AIBW (ADJ). The FOLR1-binding agents (e.g., IMGN853) may be administered at about 0.15 mg/kg, wherein the kilograms of body weight are adjusted to IBW, LBW, or AIBW (ADJ). The FOLR1-binding agents (e.g., IMGN853) may be administered at about 0.5 mg/kg, wherein the kilograms of body weight are adjusted to IBW, LBW, or AIBW (ADJ). The FOLR1-binding agents (e.g., IMGN853) may be administered at about 1.0 mg/kg, wherein the kilograms of body weight are adjusted to IBW, LBW, or AIBW (ADJ). The FOLR1-binding agents (e.g., IMGN853) may be administered at about 1.1 mg/kg, wherein the kilograms of body weight are adjusted to IBW, LBW, or AIBW (ADJ). The FOLR1-binding agents (e.g., IMGN853) may be administered at about 1.5 mg/kg, wherein the kilograms of body weight are adjusted to IBW, LBW, or AIBW (ADJ). The FOLR1-binding agents (e.g., IMGN853) may be administered at about 1.8 mg/kg, wherein the kilograms of body weight are adjusted to IBW, LBW, or AIBW (ADJ). The FOLR1-binding agents (e.g., IMGN853) may be administered at about 2.0 mg/kg, wherein the kilograms of body weight are adjusted to IBW, LBW, or AIBW (ADJ). The FOLR1-binding agents (e.g., IMGN853) may be administered at about 2.5 mg/kg, wherein the kilograms of body weight are adjusted to IBW, LBW, or AIBW (ADJ). The FOLR1-binding agents (e.g., IMGN853) may be administered at about 2.8 mg/kg, wherein the kilograms of body weight are adjusted to IBW, LBW, or AIBW (ADJ). The FOLR1-binding agents (e.g., IMGN853) may be administered at about 3.0 mg/kg, wherein the kilograms of body weight are adjusted IBW, LBW, or AIBW (ADJ). The FOLR1-binding agents (e.g., IMGN853) may be administered at about 3.3 mg/kg, wherein the kilograms of body weight are adjusted to IBW, LBW, or AIBW (ADJ). The FOLR1-binding agents (e.g., IMGN853) may be administered at about 3.75 mg/kg, wherein the kilograms of body weight are adjusted to IBW, LBW, or AIBW (ADJ). The FOLR1-binding agents (e.g., IMGN853) may be administered at about 4.2 mg/kg, wherein the kilograms of body weight are adjusted to IBW, LBW, or AIBW (ADJ). The FOLR1-binding agents (e.g., IMGN853) may be administered at about 4.5 mg/kg, wherein the kilograms of body weight are adjusted to IBW, LBW, or AIBW (ADJ). The FOLR1-binding agents (e.g., IMGN853) may be administered at about 4.8 mg/kg, wherein the kilograms of body weight are adjusted to IBW, LBW, or AIBW (ADJ). The FOLR1-binding agents (e.g., IMGN853) may be administered at about 5.0 mg/kg, wherein the kilograms of body weight are adjusted to IBW, LBW, or AIBW (ADJ). The FOLR1-binding agents (e.g., IMGN853) may be administered at about 5.5 mg/kg, wherein the kilograms of body weight are adjusted to IBW, LBW, or AIBW (ADJ). The FOLR1-binding agents (e.g., IMGN853) may be administered at about 5.6 mg/kg, wherein the kilograms of body weight are adjusted to IBW, LBW, or AIBW (ADJ). The FOLR1-binding agents (e.g., IMGN853) may be administered at about 6.0 mg/kg, wherein the kilograms of body weight are adjusted to IBW, LBW, or AIBW (ADJ). The FOLR1-binding agents (e.g., IMGN853) may be administered at about 6.1 mg/kg, wherein the kilograms of body weight are adjusted to IBW, LBW, or AIBW (ADJ). The FOLR1-binding agents (e.g., IMGN853) may be administered at about 6.2 mg/kg, wherein the kilograms of body weight are adjusted to IBW, LBW, or AIBW (ADJ). The FOLR1-binding agents (e.g., IMGN853) may be administered at about 6.3 mg/kg, wherein the kilograms of body weight are adjusted to IBW, LBW, or AIBW (ADJ). The FOLR1-binding agents (e.g., IMGN853) may be administered at about 6.4 mg/kg, wherein the kilograms of body weight are adjusted to IBW, LBW, or AIBW (ADJ). The FOLR1-binding agents (e.g., IMGN853) may be administered at about 6.5 mg/kg, wherein the kilograms of body weight are adjusted to IBW, LBW, or AIBW (ADJ). The FOLR1-binding agents (e.g., IMGN853) may be administered at about 6.6 mg/kg, wherein the kilograms of body weight are adjusted to IBW, LBW, or AIBW (ADJ). The FOLR1-binding agents (e.g., IMGN853) may be administered at about 6.7 mg/kg, wherein the kilograms of body weight are adjusted to IBW, LBW, or AIBW (ADJ). The FOLR1-binding agents (e.g., IMGN853) may be administered at about 6.8 mg/kg, wherein the kilograms of body weight are adjusted to IBW, LBW, or AIBW (ADJ). The FOLR1-binding agents (e.g., IMGN853) may be administered at about 6.9 mg/kg, wherein the kilograms of body weight are adjusted to IBW, LBW, or AIBW (ADJ). The FOLR1-binding agents (e.g., IMGN853) may be administered at about 7.0 mg/kg,

wherein the kilograms of body weight are adjusted to IBW, LBW, or AIBW (ADJ). The kilograms of body weight may be adjusted to AIBW (ADJ). In the context of the present invention, the immunoconjugate is administered at a dose of 6 milligrams (mg) per kilogram (kg) of adjusted ideal body weight (AIBW).

Furthermore, the FOLR1-binding agents can be administered at particular dose interval. For example, the FOLR1-binding agents can be administered from about four times a week to about once every four weeks. Thus, in some embodiments, the FOLR1-binding agents are administered about once every three weeks. In some embodiments, the FOLR1-binding agents are administered about once every two and a half weeks. In some embodiments, the FOLR1-binding agents are administered about once every two weeks. In some embodiments, the FOLR1-binding agents are administered about once every ten days. In some embodiments, the FOLR1-binding agents are administered about once every week.

The FOLR1-binding agents can also be administered in an about 3-week (i.e. about 21-day) cycle. For example, the FOLR1-binding agents can be administered twice in about 3 weeks. Thus, in some embodiments, the FOLR1-binding agents can be administered at about days 1 and 8 of a 21-day cycle. In other embodiments, the FOLR1-binding agents can be administered three times in about 3 weeks. Thus, in some embodiments, the FOLR1-binding agents can be administered at about days 1, 8, and 15 of a 21-day cycle.

The FOLR1-binding agents can also be administered in an about 4-week (i.e. about 28-day) cycle. For example, the FOLR1-binding agents can be administered three times in about 4 weeks. Thus, in some embodiments, the FOLR1-binding agents can be administered at about days 1, 8, and 15 of a 28-day cycle.

Described is that the FOLR1-binding agents can be administered at particular dosages. For example, the FOLR1-binding agents (e.g., IMGN853) can be administered at a dose of about 0.15 mg/kg to about 7 mg/kg once a week for three weeks on a four-week schedule. The FOLR1-binding agents (e.g., IMGN853) may be administered at a dose of about 3.0 mg/kg to about 6.0 mg/kg once a week for three weeks on a four-week schedule. The FOLR1-binding agents (e.g., IMGN853) may be administered at a dose of about 3.3 mg/kg to about 6.0 mg/kg once a week for three weeks on a four-week schedule. The FOLR1-binding agents (e.g., IMGN853) may be administered at about 0.15 mg/kg once a week for three weeks on a four-week schedule. The FOLR1-binding agents (e.g., IMGN853) may be administered at about 0.5 mg/kg once a week for three weeks on a four-week schedule. The FOLR1-binding agents (e.g., IMGN853) may be administered at about 1.0 mg/kg once a week for three weeks on a four-week schedule. The FOLR1-binding agents (e.g., IMGN853) may be administered at about 1.1 mg/kg once a week for three weeks on a four-week schedule. The FOLR1-binding agents (e.g., IMGN853) may be administered at about 1.5 mg/kg once a week for three weeks on a four-week schedule. The FOLR1-binding agents (e.g., IMGN853) may be administered at about 1.8 mg/kg once a week for three weeks on a four-week schedule. The FOLR1-binding agents (e.g., IMGN853) may be administered at about 2.0 mg/kg once a week for three weeks on a four-week schedule. In some embodiments, the FOLR1-binding agents (e.g., IMGN853) are administered at about 2.5 mg/kg once a week for three weeks on a four-week schedule. The FOLR1-binding agents (e.g., IMGN853) may be administered at about 2.8 mg/kg once a week for three weeks on a four-week schedule. The FOLR1-binding agents (e.g., IMGN853) may be administered at about 3.0 mg/kg once a week for three weeks on a four-week schedule. The FOLR1-binding agents (e.g., IMGN853) may be administered at about 3.3 mg/kg once a week for three weeks on a four-week schedule. The FOLR1-binding agents (e.g., IMGN853) may be administered at about 3.75 mg/kg once a week for three weeks on a four-week schedule.. The FOLR1-binding agents (e.g., IMGN853) may be administered at about 4.2 mg/kg once a week for three weeks on a four-week schedule. The FOLR1-binding agents (e.g., IMGN853) may be administered at about 4.5 mg/kg once a week for three weeks on a four-week schedule. The FOLR1-binding agents (e.g., IMGN853) may be administered at about 4.8 mg/kg once a week for three weeks on a four-week schedule. The FOLR1-binding agents (e.g., IMGN853) may be administered at about 5.0 mg/kg once a week for three weeks on a four-week schedule. The FOLR1-binding agents (e.g., IMGN853) may be administered at about 5.5 mg/kg once a week for three weeks on a four-week schedule. The FOLR1-binding agents (e.g., IMGN853) may be administered at about 5.6 mg/kg once a week for three weeks on a four-week schedule. The FOLR1-binding agents (e.g., IMGN853) may be administered at about 6.0 mg/kg once a week for three weeks on a four-week schedule. The FOLR1-binding agents (e.g., IMGN853) may be administered at about 6.1 mg/kg once a week for three weeks on a four-week schedule. The FOLR1-binding agents (e.g., IMGN853) may be administered at about 6.2 mg/kg once a week for three weeks on a four-week schedule. The FOLR1-binding agents (e.g., IMGN853) may be administered at about 6.3 mg/kg once a week for three weeks on a four-week schedule. The FOLR1-binding agents (e.g., IMGN853) may be administered at about 6.4 mg/kg once a week for three weeks on a four-week schedule. The FOLR1-binding agents (e.g., IMGN853) may be administered at about 6.5 mg/kg once a week for three weeks on a four-week schedule. The FOLR1-binding agents (e.g., IMGN853) may be administered at about 6.6 mg/kg once a week for three weeks on a four-week schedule. The FOLR1-binding agents (e.g., IMGN853) may be administered at about 6.7 mg/kg once a week for three weeks on a four-week schedule. The FOLR1-binding agents (e.g., IMGN853) may be administered at about 6.8 mg/kg once a week for three weeks on a four-week schedule. The FOLR1-binding agents (e.g., IMGN853) may be administered at about 6.9 mg/kg once a week for three weeks on a four-week schedule. The FOLR1-binding agents (e.g., IMGN853) may be administered at about 7.0 mg/kg once a week for three weeks on

a four-week schedule. In an embodiment of the invention, the FOLR1-binding agents of the present invention (e.g., IMGN853) are administered at 6.0 mg/kg once a week for three weeks on a four-week schedule.

Described is that the FOLR1-binding agents (e.g., IMGN853) can be administered at a dose of about 0.15 mg/kg to about 7 mg/kg once a week for three weeks on a four-week schedule, wherein the kilograms of body weight are adjusted to ideal body weight (IBW), lean body weight (LBW), or adjusted ideal body weight (AIBW or ADJ). The FOLR1-binding agents (e.g., IMGN853) may be administered at a dose of about 3.0 mg/kg to about 6.0 mg/kg once a week for three weeks on a four-week schedule, wherein the kilograms of body weight are adjusted to IBW, LBW, or AIBW (ADJ). The FOLR1-binding agents (e.g., IMGN853) may be administered at a dose of about 3.3 mg/kg to about 6.0 mg/kg once a week for three weeks on a four-week schedule, wherein the kilograms of body weight are adjusted to IBW, LBW, or AIBW (ADJ). The FOLR1-binding agents (e.g., IMGN853) may be administered at about 0.15 mg/kg once a week for three weeks on a four-week schedule, wherein the kilograms of body weight are adjusted to IBW, LBW, or AIBW (ADJ). The FOLR1-binding agents (e.g., IMGN853) may be administered at about 0.5 mg/kg once a week for three weeks on a four-week schedule, wherein the kilograms of body weight are adjusted to IBW, LBW, or AIBW (ADJ). The FOLR1-binding agents (e.g., IMGN853) may be administered at about 1.0 mg/kg once a week for three weeks on a four-week schedule, wherein the kilograms of body weight are adjusted to IBW, LBW, or AIBW (ADJ). The FOLR1-binding agents (e.g., IMGN853) may be administered at about 1.1 mg/kg once a week for three weeks on a four-week schedule, wherein the kilograms of body weight are adjusted to IBW, LBW, or AIBW (ADJ). The FOLR1-binding agents (e.g., IMGN853) may be administered at about 1.5 mg/kg once a week for three weeks on a four-week schedule, wherein the kilograms of body weight are adjusted to IBW, LBW, or AIBW (ADJ). The FOLR1-binding agents (e.g., IMGN853) may be administered at about 1.8 mg/kg once a week for three weeks on a four-week schedule, wherein the kilograms of body weight are adjusted to IBW, LBW, or AIBW (ADJ). The FOLR1-binding agents (e.g., IMGN853) may be administered at about 2.0 mg/kg once a week for three weeks on a four-week schedule, wherein the kilograms of body weight are adjusted to IBW, LBW, or AIBW (ADJ). The FOLR1-binding agents (e.g., IMGN853) may be administered at about 2.5 mg/kg once a week for three weeks on a four-week schedule, wherein the kilograms of body weight are adjusted to IBW, LBW, or AIBW (ADJ). The FOLR1-binding agents (e.g., IMGN853) may be administered at about 2.8 mg/kg once a week for three weeks on a four-week schedule, wherein the kilograms of body weight are adjusted to IBW, LBW, or AIBW (ADJ). The FOLR1-binding agents (e.g., IMGN853) may be administered at about 3.0 mg/kg once a week for three weeks on a four-week schedule, wherein the kilograms of body weight are adjusted IBW, LBW, or AIBW (ADJ). The FOLR1-binding agents (e.g., IMGN853) may be administered at about 3.3 mg/kg once a week for three weeks on a four-week schedule, wherein the kilograms of body weight are adjusted to IBW, LBW, or AIBW (ADJ). The FOLR1-binding agents (e.g., IMGN853) may be administered at about 3.75 mg/kg once a week for three weeks on a four-week schedule, wherein the kilograms of body weight are adjusted to IBW, LBW, or AIBW (ADJ). The FOLR1-binding agents (e.g., IMGN853) may be administered at about 4.2 mg/kg once a week for three weeks on a four-week schedule, wherein the kilograms of body weight are adjusted to IBW, LBW, or AIBW (ADJ). The FOLR1-binding agents (e.g., IMGN853) may be administered at about 4.5 mg/kg once a week for three weeks on a four-week schedule, wherein the kilograms of body weight are adjusted to IBW, LBW, or AIBW (ADJ). The FOLR1-binding agents (e.g., IMGN853) may be administered at about 4.8 mg/kg once a week for three weeks on a four-week schedule, wherein the kilograms of body weight are adjusted to IBW, LBW, or AIBW (ADJ). The FOLR1-binding agents (e.g., IMGN853) may be administered at about 5.0 mg/kg once a week for three weeks on a four-week schedule, wherein the kilograms of body weight are adjusted to IBW, LBW, or AIBW (ADJ). The FOLR1-binding agents (e.g., IMGN853) may be administered at about 5.5 mg/kg once a week for three weeks on a four-week schedule, wherein the kilograms of body weight are adjusted to IBW, LBW, or AIBW (ADJ). The FOLR1-binding agents (e.g., IMGN853) may be administered at about 5.6 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.0 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.1 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.2 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.3 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.4 mg/kg once a week for three weeks on a four-week schedule, wherein the kilograms of body weight are adjusted to IBW, LBW, or AIBW (ADJ). The FOLR1 -binding agents (e.g., IMGN853) may be administered at about 6.5 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.6 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.7 mg/kg once a week for three weeks on a four-week schedule, wherein the kilograms of body weight are adjusted to IBW, LBW, or AIBW (ADJ).

The FOLR1-binding agents (e.g., IMGN853) may be administered at about 6.8 mg/kg once a week for three weeks on a four-week schedule, wherein the kilograms of body weight are adjusted to IBW, LBW, or AIBW (ADJ). The FOLR1-binding agents (e.g., IMGN853) may be administered at about 6.9 mg/kg once a week for three weeks on a four-week schedule, wherein the kilograms of body weight are adjusted to IBW, LBW, or AIBW (ADJ). The FOLR1-binding agents (e.g., IMGN853) may be administered at about 7.0 mg/kg once a week for three weeks on a four-week schedule, wherein the kilograms of body weight are adjusted to IBW, LBW, or AIBW (ADJ). The kilograms of body weight may be adjusted to AIBW (ADJ).

In some embodiments, the FOLR1-binding agents of the present invention can be administered at a dose that results in a particular Cmax. For example, in some embodiments, the FOLR1-binding agents are administered at a dose that results in a Cmax of about 110 to about 160 μg/mL. In some embodiments, the FOLR1-binding agents are administered at a dose that results in a Cmax of about 110 to about 150 μg/mL. In some embodiments, the FOLR1-binding agents are administered at a dose that results in a Cmax of about 110 to about 140 μg/mL. In some embodiments, the FOLR1-binding agents are administered at a dose that results in a Cmax of about 120 to about 160 μg/mL. In some embodiments, the FOLR1-binding agents are administered at a dose that results in a Cmax of about 120 to about 150 μg/mL. In some embodiments, the FOLR1-binding agents are administered at a dose that results in a Cmax of about 120 to about 140 μg/mL. In some embodiments, the FOLR1-binding agents are administered at a dose that results in a Cmax of about 90 to about 160 μg/mL. In some embodiments, the FOLR1-binding agents are administered at a dose that results in a Cmax of about 90 to about 150 μg/mL. In some embodiments, the FOLR1-binding agents are administered at a dose that results in a Cmax of about 90 to about 140 μg/mL. In some embodiments, the FOLR1-binding agents are administered at a dose that results in a Cmax of about 100 to about 160 μg/mL. In some embodiments, the FOLR1-binding agents are administered at a dose that results in a Cmax of about 100 to about 150 μg/mL. In some embodiments, the FOLR1-binding agents are administered at a dose that results in a Cmax of about 100 to about 140 μg/mL.

In certain embodiments, the FOLR1-binding agents of the present invention can be administered at a dose that results in a particular AUC. For example, in certain embodiments, the FOLR1-binding agents are administered at a dose that results in a $AUC_{0-24}$ of no more than 2785 hr•μg/mL. In certain embodiments, the FOLR1-binding agents are administered at a dose that results in a $AUC_{0-24}$ of no more than 2741 hr•μg/mL. In certain embodiments, the FOLR1-binding agents are administered at a dose that results in a $AUC_{0-24}$ of no more than 2700 hr•μg/mL. In some embodiments, the administration produces an $AUC_{0-24}$ of about 1000-3500 hr•μg/mL. In some embodiments, the administration produces an $AUC_{0-24}$ of about 1000-3000 hr•μg/mL. In some embodiments, the administration produces an $AUC_{0-24}$ of about 1000-2785 hr•μg/mL. In some embodiments, the administration produces an $AUC_{0-24}$ of about 1000-2741 hr•μg/mL. In some embodiments, the administration produces an $AUC_{0-24}$ of about 1000-2700 hr•μg/mL. In some embodiments, the administration produces an $AUC_{0-24}$ of about 1000-2500 hr•μg/mL. In some embodiments, the administration produces an $AUC_{0-24}$ of no more than 1500-3500 hr•μg/mL. In some embodiments, the administration produces an $AUC_{0-24}$ of no more than 1500-3000 hr•μg/mL. In some embodiments, the administration produces an $AUC_{0-24}$ of no more than 1500-2785 hr•μg/mL. In some embodiments, the administration produces an $AUC_{0-24}$ of no more than 1500-2741 hr•μg/mL. In some embodiments, the administration produces an $AUC_{0-24}$ of no more than 1500-2700 hr•μg/mL. In some embodiments, the administration produces an $AUC_{0-24}$ of about 1500-2500 hr•μg/mL.

In certain embodiments, the disease treated with the FOLR1-binding agent or antagonist (e.g., an anti-FOLRl antibody) of the present invention is a specific cancer. Thereby, the cancer is characterized by FOLR1 expressing cells to which the FOLR1-binding agent (e.g., antibody) binds. In certain embodiments, a tumor overexpresses the human FOLR1.

The present invention provides for treatment of cancer comprising administering a therapeutically effective amount of a FOLR1-binding agent of the present invention to a subject (e.g., a subject in need of treatment). Cancers that can be treated by FOLR1-binding agents as described include, but are not limited to, neoplasms, tumors, metastases, or any disease or disorder characterized by uncontrolled cell growth. The cancer can be a primary or metastatic cancer. Cancers that are treated by the treatment according to the invention include ovarian cancer, endometrial cancer, uterine cancer and cancer of the peritoneum. In certain embodiments, the cancer is ovarian cancer. In certain embodiments, the cancer is endometrial cancer. Specific examples of cancers that can be treated by FOLR1-binding agents as described include, but are not limited to ovarian cancer, lung cancer, colorectal cancer, pancreatic cancer, liver cancer, breast cancer, brain cancer, kidney cancer, prostate cancer, gastrointestinal cancer, melanoma, cervical cancer, bladder cancer, glioblastoma, endometrial cancer, and head and neck cancer. The cancer may be ovarian cancer. The cancer may be endometrial cancer. The cancer may be lung cancer. The lung cancer may be non small cell lung cancer. The non-small cell lung cancer may be adenocarcinoma of the lung.

According to the invention, the cancer is a cancer that expresses FOLR1 (polypeptide or nucleic acid). In some embodiments, the FOLR1-binding agent is administered to a patient with an increased expression level of FOLR1, for example, as described in U.S. Published Application No. 2012/0282175 or International Published Application No. WO 2012/135675. Thus, in some embodiments, the FOLR1 expression is measured by immunohistochemistry (IHC) and given a staining intensity score and/or a staining uniformity score by comparison to controls (e.g., calibrated controls)

exhibiting defined scores (e.g. an intensity score of 3 is given to the test sample if the intensity is comparable to the level 3 calibrated control or an intensity of 2 is given to the test sample if the intensity is comparable to the level 2 calibrated control). A staining uniformity that is heterogeneous or homogeneous is also indicative of increased FOLR1 expression. The staining intensity and staining uniformity scores can be used alone or in combination (e.g., 2 homo, 2 hetero, 3 homo, 3 hetero, etc.). In another example, an increase in FOLR1 expression can be determined by detection of an increase of at least 2-fold, at least 3-fold, or at least 5-fold) relative to control values (e.g., expression level in a tissue or cell from a subject without cancer or with a cancer that does not have elevated FOLR1 values).

In some embodiments, the cancer is a cancer that expresses FOLR1 at a level of 1 hetero or higher by IHC. In some embodiments, the cancer is a cancer that expresses FOLR1 at a level of 2 hetero or higher by IHC. In some embodiments, the cancer is a cancer that expresses FOLR1 at a level of 3 hetero or higher by IHC. Described is that the cancer may be a lung cancer that expresses FOLR1 at a level of 2 hetero or higher by IHC. Described is that the cancer may be a lung cancer that expresses FOLR1 at a level of 3 hetero or higher by IHC. In some embodiments, the cancer is a ovarian cancer that expresses FOLR1 at a level of 2 hetero or higher by IHC. In some embodiments, the cancer is a ovarian cancer that expresses FOLR1 at a level of 3 hetero or higher by IHC. In some embodiments, the cancer is an endometrioid cancer that expresses FOLR1 at a level of 1 hetero or higher by IHC. In some embodiments, the cancer is a endometrioid cancer that expresses FOLR1 at a level of 2 hetero or higher by IHC.

In certain embodiments, the treatment for inhibiting tumor growth comprises administering to a subject a therapeutically effective amount of the FOLR1-binding agent of the invention. In certain embodiments, the subject is a human. In certain embodiments, the subject has a tumor or has had a tumor removed.

In addition, the invention provides a treatment of reducing the tumorigenicity of a tumor in a subject, comprising administering a therapeutically effective amount of the FOLR1-binding agent of the invention to the subject. Described is that a tumor may comprise cancer stem cells. The frequency of cancer stem cells in the tumor may be reduced by administration of the agent.

Described are pharmaceutical compositions comprising one or more of the FOLR1-binding agents described herein. The pharmaceutical compositions may further comprise a pharmaceutically acceptable vehicle. Described is that these pharmaceutical compositions find use in inhibiting tumor growth and treating cancer in human patients.

In certain embodiments, formulations are prepared for storage and use by combining a purified antibody of the present invention with a pharmaceutically acceptable vehicle (e.g. carrier, excipient) (Remington, The Science and Practice of Pharmacy 20th Edition Mack Publishing, 2000). Suitable pharmaceutically acceptable vehicles include, but are not limited to, nontoxic buffers such as phosphate, citrate, and other organic acids; salts such as sodium chloride; antioxidants including ascorbic acid and methionine; preservatives (e.g. octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3-pentanol; and m-cresol); low molecular weight polypeptides (e.g. less than about 10 amino acid residues); proteins such as serum albumin, gelatin, or immunoglobulins; hydrophilic polymers such as polyvinylpyrrolidone; amino acids such as glycine, glutamine, asparagine, histidine, arginine, or lysine; carbohydrates such as monosaccharides, disaccharides, glucose, mannose, or dextrins; chelating agents such as EDTA; sugars such as sucrose, mannitol, trehalose or sorbitol; salt-forming counter-ions such as sodium; metal complexes (e.g. Zn-protein complexes); and non-ionic surfactants such as TWEEN or polyethylene glycol (PEG).

The pharmaceutical compositions described herein can be administered in any number of ways for either local or systemic treatment. Administration can be topical (such as to mucous membranes including vaginal and rectal delivery) such as transdermal patches, ointments, lotions, creams, gels, drops, suppositories, sprays, liquids and powders; pulmonary (e.g., by inhalation or insufflation of powders or aerosols, including by nebulizer; intratracheal, intranasal, epidermal and transdermal); oral; or parenteral including intravenous, intraarterial, subcutaneous, intraperitoneal or intramuscular injection or infusion; or intracranial (e.g., intrathecal or intraventricular) administration. In some particular embodiments, the administration is intravenous.

An antibody or immunoconjugate can be combined in a pharmaceutical combination formulation, or dosing regimen as combination therapy, with a second compound. In some embodiments, the second compound is a steroid. In some embodiments, the treatment encompasses administration of a steroid and an immunoconjugate that results in a reduction of headaches as compared to administration of the immunoconjugate alone.

The steroid can be administered at the same time as the immunoconjugate, prior to the administration of the immunoconjugate, and/or after the administration of the immunoconjugate. In some embodiments, the steroid is administered within about a week, about five days, about three days, about two days, or about one day or 24 hours prior to the administration of the immunoconjugate. In some embodiments, the steroid is administered within one day of the administration of the immunoconjugate. In some embodiments, the steroid is administered multiple times. In some embodiments, the steroid is administered about one day prior to the administration of the immunoconjugate and on the same day as the administration of the immunoconjugate. The steroid can be administered via any number of ways, including for example, topical, pulmonary, oral, parenteral, or intracranial administration. In some embodiments, the administration is oral. In some embodiments, the administration is intravenous. In some embodiments, the administration

is both oral and intravenous.

An antibody or immunoconjugate can also be combined in a pharmaceutical combination formulation, or dosing regimen as combination therapy, with an analgesic, or other medications that prevent or treat headaches. For example, acetaminophin and/or dephenhydramine can be administered in addition to the administration of the antibody or immunoconjugate. The analgesic can be administered prior to, at the same time, or after the administration of the immunoconjugate and can be via any appropriate administration route. In some embodiments, the analgesic is administered orally.

In some embodiments, the treatment comprises administration of a first compound that is an antibody or immunoconjugate of the present invention, a second compound that is a steroid, and a third compound that is an analgesic. In some embodiments, the treatment comprises administration of a first compound that is IMGN388, a second compound that is dexamethasone, and a third compound that is acetaminophin and/or diphenydramine.

An antibody or immunoconjugate can be combined in a pharmaceutical combination formulation, or dosing regimen as combination therapy, with a second compound having anti-cancer properties. The second compound of the pharmaceutical combination formulation or dosing regimen can have complementary activities to the ADC of the combination such that they do not adversely affect each other. Pharmaceutical compositions comprising the FOLR1-binding agent and the second anti-cancer agent are also described.

Embodiments of the present disclosure can be further defined by reference to the following examples, which describe in detail preparation of certain antibodies of the present disclosure and methods for using antibodies of the present disclosure..

## Examples

It is understood that the examples and embodiments described herein are for illustrative purposes only.

### Example 1

IMGN853 Dosing Trial in Human Cancer Patients

IMGN853 is an antibody-drug conjugate (ADC) comprising a folate receptor 1 (FOLR1)-binding antibody and the potent maytansinoid, DM4. IMGN853 has been previously described in International Published Application Nos. WO 2011/106528, WO 2012/135675, and WO 2012/138749, and U.S. Published Application Nos. 2012/0009181, 2012/0282175, and 2012/0282282. IMGN853 is huMov19-sSPDB-DM4, and the huMov19 antibody contains a variable heavy chain with the amino acid sequence of SEQ ID NO:3 and a variable light chain with the amino acid sequence of SEQ ID NO: 5. FOLR1 protein is expressed at elevated levels on many solid tumors, particularly epithelial ovarian cancer (EOC), endometrial cancer, non-small cell lung cancer (NSCLC), and clear-cell renal cell cancer.

A study to determine the maximum tolerated dose (MTD) and recommended phase 2 dose (RP2D) as well as to evaluate the safety, pharmacokinetics (PK), pharmacodynamics (PD), and efficacy of IMGN853 was initiated. The study includes two components: an accelerated dose titration component, where the IMGN853 immunoconjugate was administered to patients with any type of FOLR1-expressing refractory solid tumors including epithelial ovarian cancer (EOC) and other FOLR1-positive solid tumors, and a dose expansion component.

For the accelerated titration portion of the study, IMGN853 was given intravenously (IV) on Day 1 of each 21-day (3 week) cycle. Twenty-nine patients have been enrolled across seven dose levels ranging from 0.15 to 7.0 mg/kg IMGN853 in the accelerated portion of the clinical trial and there are safety data currently available for 23 patients. There were no study drug-related AEs of any grade reported in patients treated in the first 4 dose cohorts. At doses up to 5.0 mg/kg, IMGN853 related AEs have been mild to moderate. At the 5.0 and 7.0 mg/kg dose levels, 4 of 10 and 5 of 5 patients, respectively, have reported ocular toxicity.

Table 1: Enrollment by Tumor Type

| **Enrollment and FOLR1 Expression by Tumor Type<br>N=29** | | | | | | |
|---|---|---|---|---|---|---|
| **Diagnosis** | **FOLR1 Expression Level** | | | | | |
| | 2 Hetero | 2 Homo | 3 Hetero | 3 Homo | Other | Totals |
| Ovarian Cancer | **2** | **2** | **7** | **4** | **1** | **16** |
| Serous | 1[1] | | 6[1] | 3 | | 10 |
| Transitional Cell | | | 1[2] | | | 1 |
| Clear Cell | 1 | 1 | | 1 | | 3 |

(continued)

| Enrollment and FOLR1 Expression by Tumor Type N=29 | | | | | | |
|---|---|---|---|---|---|---|
| Diagnosis | FOLR1 Expression Level | | | | | |
| | 2 Hetero | 2 Homo | 3 Hetero | 3 Homo | Other | Totals |
| Carcinosarcoma | | 1 | | | **1**[4] | 1 |
| Endometrial | **2** | **0** | **5** | **1** | **0** | **8** |
| Serous | | | 3[3] | 1 | | 4 |
| Endometrioid | 2 | | | | | 2 |
| Adenosquamous | | | 1 | | | 1 |
| Mixed | | | 1 | | | 1 |
| NSCLC Adenocarcinoma | **1** | | | | | **1** |
| Renal Cell | **0** | **2** | | | **2**[5] | **4** |
| Clear Cell | | 2 | | | 2[5] | 4 |

[1]CA125 response in 1 patient each at 3.3 mg/kg, 5.0 mg/kg, and 7.0 mg/kg
[2]Confirmed partial response
[3]Unconfirmed partial response and confirmed CA125 response in 1 patient at 5.0 mg/kg
[4]Focal
[5]Negative

Drug exposure was measured in 23 patients and found to generally increase linearly, with a half-life at doses ≥ 2.0 mg/kg of approximately 5 days. One patient with serous endometrial cancer also had a CA125 response and an unconfirmed partial response at 5 mg/kg. Three patients with ovarian cancer have reported confirmed CA125 response (one each at 7 mg/kg, 5 mg/kg and one at 3.3 mg/kg). Patients receiving IMGN853 at doses greater than or equal to 5.0 mg/kg received dexamethasone, 10 mg IV (or similar steroid equivalent), 30 to 60 minutes prior to anti-FOLRI immunoconjugate (e.g., IMGN853) administration.

The pharmacokinetic (PK) parameters are reported for Cycle 1 (first cycle of dosing for each patient only) of the IMGN853 Phase 1 trial. (Figures 1A and B.) The clearance of IMGN853 is shown to be rapid at low doses (CL= 1.1 mL/hr/kg) with a half life of approximately 35.4 hours or 1.5 days. The clearance decreases (CL= 0.4 mL/her/kg) at the higher doses, and the half-life increases to about 4 days or about 5 days at doses ≥ 2.0 mg/kg. The exposure (AUC) and the Cmax are shown to generally increase at the higher doses as well.

At the 7.0 mg/kg dose, all 5 patients experienced ocular toxicity. One patient was reported with Grade 3, dose-limiting punctate keratitis and Grade 2 blurred vision that were deemed definitely related to study treatment. Additionally, there was one patient each with Grade 3, Grade 2, and Grade 1 blurred vision; all events were deemed possibly or definitely related to IMGN853 treatment. As a result, the maximum tolerated dose on this schedule of administration (i.e., once every three weeks) was deemed to have been exceeded at the 7.0 mg/kg dose level, and all patients remaining at the 7.0 mg/kg dose level were dose reduced to the previous dose level (5.0 mg/kg) and 7 additional patients were evaluated at the 5 mg/kg dose. Together with the 3 patients treated previously, 10 patients were treated at a dose of 5 mg/kg. Of the total of 10 patients at the 5 mg/kg level, 3 had blurred vision, including 1 patient with grade 3 blurred vision, and 2 patients had corneal changes. Other related grade 3 adverse events included elevated alkaline phosphatase and grade 3 hypophosphatemia. Additional patients were enrolled to the 3.3 mg/kg dose level to further explore and confirm the safety profile seen with the 3 patients originally assigned to this dose. Safety review of the additional 6 patients currently being treated at the 3.3 mg/kg is ongoing and IMGN853 is well tolerated. To date, three of the nine patients treated at the 3.3 mg/kg dose level have reported IMGN853-related AEs, including grade 2 peripheral neuropathy (1 patient), grade 2 nausea, fatigue and AST elevation (1 patient), and 1 patient with grade 2 vomiting.

Once the MTD is defined, the study will proceed to the dose expansion phase. Three expansion cohorts will evaluate patients with FOLR1 protein positive (1) platinum resistant epithelial ovarian cancer; (2) relapsed or refractory epithelial ovarian cancer, and (3) relapsed or refractory non small cell lung cancer (NSCLC). Cohorts 2 and 3 will have IMGN853 PD assessment by pre-and post-dose tumor biopsy and/or by FLT-PET imaging, respectively. IMGN853 will be administered at a dose of at least 3.3 mg/kg and may include doses of 5.0 mg/kg or as high as 6.0 mg/kg or even 7.0 mg/kg. Initially IMGN853 should be administered at a rate of 1 mg/min; after 30 minutes, the rate can be increased to 3 mg/min if well tolerated. If well tolerated after 30 minutes at 3 mg/min, the rate may be increased to 5 mg/min.

Subsequent infusions can be delivered at the tolerated rate.

For all IMGN853 dosing at 3.3 mg/kg or higher, prophylactic steroid treatment will be included using the protocols described in Example 2 (e.g., steroid treatment is included at 10 mg dexamethasone IV (or similar steroid equivalent) 30 to 60 minutes prior to IMGN853 administration is required and prophylactic diphenhydramine HCl and acetaminophen is recommended prior to IMGN853 administration). Cycles are repeated until (i) the patient's disease worsens, (ii) the patient experiences unacceptable toxicity, (iii) the patient withdraws consent, (iv) the patient develops a comorbid condition that would preclude further study treatment or (v) the patient is discontinues due to non-compliance or administrative reasons.

Responses are assessed using RECIST and Gynecologic Cancer Intergroup (GCIG) criteria (as appropriate).

Example 2

IMGN853 Steroid-Based Prophylaxis for Infusion Reaction

In order to decrease the likelihood of infusion reaction, any of the following steroid-based prophylaxis protocols can be used.

(1) Patients receive dexamethasone, 10 mg IV (or similar steroid equivalent), 30 to 60 minutes prior to anti-FOLRl immunoconjugate (e.g., IMGN853) administration.

(2) Patients receive dexamethasone, 10 mg IV (or similar steroid equivalent) and diphenhydramine HCl (25-50 mg IV or PO), with or without acetaminophen (325-650 mg IV or PO), 30 to 60 minutes prior to anti-FOLRl immunoconjugate (e.g., IMGN853) administration. This prophylactic protocol is recommended and at the discretion of each investigator.

(3) Patients receive dexamethasone 8 mg (or similar steroid equivalent) by mouth BID on the day prior to administration of anti-FOLRl immunoconjugate (e.g., IMGN853). On the day of administration of anti-FOLRl immunoconjugate (e.g., IMGN853), 30-60 mins prior to anti-FOLRl immunoconjugate (e.g., IMGN853) administration, patients receive dexamethasone, 10 mg IV (or similar steroid equivalent), diphenhydramine HCl (25-50 mg IV or PO), with or without acetaminophen (325-650 mg IV or PO)

(4) Within 24 hours prior to infusion steroids (e.g., dexamethasone) are administered orally.

Example 3

Relationship of IMGN853 Exposure with Ocular Toxicity

For each patient treated with the IMGN853 protocol described in Examples 1 and 2, the plasma concentration of IMGN853 was measured at various time points across each cycle, beginning at end of infusion and continuing to day 21. Pharmacokinetic (PK) parameter analysis identified an apparent association between Cmax and the occurrence of ocular toxicity, which is characterized by corneal deposits and loss of visual acuity. The statistically significant correlation was also observed with early exposure levels as measured by area under the curve in the first 24 hrs ($AUC_{0-24}$). (See Figures 2A-2C.)

In the 3.3 to 7.0 mg/kg cohorts, ocular toxicity was observed in 9/10 patients with Cmax values at or above 147.7 μg/ml, indicated by the dotted line in Figure 2A. No patients with Cmax values below 147.7 μg/ml developed ocular toxicity. All (9/9) patients with an $AUC_{0-24}$ at or above 2785 hr*μg/ml, indicated by the dotted line in Figure 2B, developed ocular toxicity, whereas none below had any eye toxicity. Efficacy signals were observed at doses ≥ 3.3 mg/kg and did not correlate with reports of ocular toxicity. The lowest Cmax value in patients that have had signals of activity was 91.25 μg/ml.

After 24 patients were treated with either 3.3 mg/kg, 5.0 mg/kg, or 7.0 mg/kg IMGN853, a Cmax value above threshold correlated with (reversible) ocular toxicity using Fisher's exact test, p = 0.00004. An $AUC_{0-24}$ value above the threshold level of 2,741 hr*μg/ml also correlated with (reversible) ocular toxicity using Fisher's exact test, p = 0.00001. Based on these results and calculations using nominal time and concentration values, it was determined that patients having a Cmax greater than about 150 μg/ml or an $AUC_{0-24}$ value greater than 2785 hr*μg/ml were most likely to have an increased rate of ocular toxicity, and patients having signals of activity had a Cmax level of at least about 90 μg/mg. Strategies were developed to modify the dosing in order to reduce the variability seen within each dose level and to achieve a Cmax level for optimal efficacy and minimal toxicity at each patient weight.

Example 4

IMGN853 Alternate Dosing Approaches

As described in Example 3, above, a correlation between Cmax values over 150 µg/ml or $AUC_{0-24}$ values over 2785 hr*µg/ml and incidence of ocular toxicity was observed across all dose levels. In addition, initial PK parameter analysis demonstrated that although Cmax increased proportionally with the dose of IMGN853, there was a significant variation in Cmax, $AUC_{0-24}$ and volume of distribution within dose levels (Figure 3).

The variation in Cmax was particularly striking in the 5 mg/kg cohort, where PK was analyzed in 10 patients. There was no apparent intra-patient Cmax variability across cycles, and infusion times were similar between patients and were not associated with Cmax variation. Covariate analysis demonstrated a correlation between weight and Cmax. (Figure 4).

Volume of distribution ($V_{ss}$) is indicative of plasma volume for biologies and does not increase linearly with weight. When Cmax values were normalized by Vss, the variation was reduced. These data suggest that exploring alternate dosing approaches other than based on total body weight could produce more uniform dosing across cohorts. To this end, Cmax values were estimated using alternative dosing calculations for all patients treated in the 3.3 (n=3), 5.0 (n=10), and 7 mg/kg (n=5) dose groups. Calculated Cmax values were normalized to a 5 mg/kg dose level and compared to Cmax values observed from total body weight (TBW) dosing. Body surface area (BSA) was also considered; however, Cmax values based on BSA dosing decreased the Cmax, but the variability was only minimally impacted and a positive correlation between weight and Cmax was still observed though to a lesser extent. Three additional alternate formulas were evaluated: (1) Ideal Body Weight (IBW), Lean Body Weight (LBW), and Adjusted Body Weight (ADJ). The formula for each of IBW, LBW, ADJ, and BSA is provided below:

*Ideal Body Weight (IBW)*

$$\text{IBW (male)} = 0.9\text{H-}88$$

$$\text{IBW (fem)} = 0.9\text{H-}92$$

(where H=height in cm)

*Lean Body Weight (LBW)*

$$\text{Men} = 1.10 \text{ x weight in kg} - 128([\text{weight in kg}]^2/[100 \text{ x height in meters}]^2)$$

$$\text{Women} = 1.07 \text{ x weight in kg} - 148([\text{weight in kg}]^2/[100 \text{ x height in meters}]^2)$$

*Adjusted Ideal Body Weight (AIBW or ADJ)*

$$\text{IBW} + 0.4(\text{Actual weight in kg} - \text{IBW})$$

*Body Surface Area (BSA) - Mosteller Formula*

$$\text{BSA (m}^2) = (\text{Height(cm) x Weight(kg) / 3600})^{1/2}$$

*Body Surface Area (BSA) - Boyd Formula*

$$\text{BSA (m}^2) = (0.0003207 \text{ x Height(cm)}^{0.3} \text{ x Weight(grams)}^{(0.7285 - (0.0188 \text{ x LOG(grams)})})]$$

Average Cmax values were 93.06, 82.72, 110.77 and 137.46 µg/ml for IBW, LBW, AIBW (ADJ) and TBW, respectively. Additionally, all three alternate metrics reduced the standard deviation in Cmax (21.7, 20.5, 22.9 vs. 33.7

μg/ml for TBW). (See Figure 5.)

As mentioned above, a positive correlation was observed for TBW versus Cmax. Correlation analysis of the IBW and LBW versus weight plots demonstrated a negative correlation versus weight. Dosing by AIBW (ADJ) body weight had the least body weight dependence (see Figure 6), similar to BSA but with less PK variability.

Dosing by IBW, LBW, or AIBW (ADJ) results in less weight dependence compared to TBW dosing. Based on the current data, dosing by AIBW (ADJ) weight results in the least variance in Cmax.

$AUC_{0-24}$ values were observed in 24 patients who received 3.3, 5, or 7 mg/kg of IMGN853 based on total body weight (see Figure 7 "TBW Actual"). In addition, $AUC_{0-24}$ values were also observed in 7 patients who received 5 mg/kg of IMGN853 based on adjusted ideal body weight (see Figure 7 "5 ADJ Actual"). These actual values obtained with the 24 patients were compared with the projected values that would have been obtained with those same patients if they had all been treated with 5 mg/kg based on total body weight (Figure 7 "TBW 5 mg/kg") or if they had all been treated with 5, 5.4, or 6 mg/kg based on adjusted ideal body weight (Figure 7 "ADJ 5 mg/kg," "ADJ 5.4 mg/kg," and "ADJ 6 mg/kg.") As shown in the table in Figure 7, administering 5 mg/kg IMGN853 based on AIBW (ADJ) minimizes the number of patients who are projected to exceed the threshold level of $AUC_{0-24}$ 2741 hr*μg/ml associated with ocular toxicity. In addition, only 14% of the 7 patients who received 5 mg/kg IMGN853 based on AIBW (ADJ) reached $AUC_{0-24}$ levels above 2741 hr*μg/ml, whereas 38% of the patients who received 3.3, 5, or 7 mg/kg of IMGN853 based on TBW exceeded this level. The original analysis of $AUC_{0-24}$ was calculated using nominal time and concentration values and resulted in a value of 2785 hr*μg/ml. When recalculated using actual time, the result was a slight modification to the $AUC_{0-24}$ values and a determination of a threshold value of 2741 hr*μg/ml.

Patients were then treated with either 5 mg/kg based on adjusted ideal body weight, 6 mg/kg based on adjusted ideal body weight, 5 mg/kg based on total body weight, or 7 mg/kg based on total body weight. The observed $AUC_{0-24}$ values in these patients are shown in Figure 8 ("Acutal"), and these values are compared with the projected values ("Proj") that would have been obtained if all of these patients had been treated with 5 mg/kg based on adjusted ideal body weight, 6 mg/kg based on adjusted ideal body weight, 5 mg/kg based on total body weight, or 7 mg/kg based on total body weight. Dosing based on adjusted ideal body weight decreased the variability in early exposure levels and decreased ocular adverse events as shown in Table 2 below. In particular, only one patient receiving 5 mg/kg based on adjusted ideal body weight experienced Grade 1 visual disturbance and three patients receiving 6 mg/kg based on adjusted ideal body weight experienced Grade 1-2 ocular toxicity. In contrast, doses of 5 mg/kg or more based on total body weight were associated with more patients having ocular adverse events and with the occurrence of Grade 3 ocular adverse events.

Table 2: Ocular Adverse Events (OAE) Reported with Dosing based on Adjusted Ideal Body Weight (All Reversible)

| IMGN853 (mg/kg adjusted ideal body weight) | Cohort Size | Patients with Any Grade OAE | OAEs | | DLTs |
|---|---|---|---|---|---|
| | | | Grade 1-2 | Grade 3[1] | |
| 5.0 | 7 | 1 | 1[2] | 0 | 0 |
| 6.0 | 7 | 3 | 5[3] | 0 | 0 |

[1]There were no Grade 4 adverse events.
[2]One patient reported visual disturbance.
[3]Grade 2 blurred vision and punctate keratitis in 1 patient; Grade 2 retinopathy in one patient; and Grade 1 blurred vision and floaters in 1 patient.

Example 5

<u>IMGN853 Alternate Schedules</u>

A population PK model based on rich and sparse (peak and through) concentration-time profiles of IMGN853 following Q3W dosing (escalating dose) was developed. The PK of IMGN853 as stated above appears to be linear over the dose range studied.

This model was used to simulate steady-state exposure to IMGN853 for the following candidate regimens:

1 to 2.5 mg/kg QW (10 dose levels at 0.15 mg/kg increments)
2 to 5 mg/kg Q2W (10 dose levels at 0.3 mg/kg increments)
1 to 2.5 mg/kg QWx4 then Q2Wx4 (10 dose levels at 0.15 mg/kg increments)
1 to 2.5 mg/kg QWx3 in 4W (10 dose levels at 0.15 mg/kg increments)

The QWx4 then Q2Wx4 dosing regimen resulted in the least accumulation (i.e., accumulation index of 1) over time, while the QW dosing regimen resulted in the highest accumulation (i.e., accumulation index of 1.97). QWx3 in 4W allowed overall exposure increases up to ~3-fold while limiting Cmax to levels below that observed with ocular toxicity (Table 3).

Table 3

| Schedule | Dose (mg/kg) | Cmax (μg/ml) | Cmin (μg/ml) | Cavg (μg/ml) | $AUC_{12W}$ (h*mg/ml) |
|---|---|---|---|---|---|
| Q3W | 4.2 | 112 | 7.8 | 30 | 32 |
| Q2W | 4.1 | 119 | 18 | 44 | 84 |
| QWx3 in 4W | 3.3 | 119 | 20 | 52 | 105 |

The population PK model was also used to simulate a hypothetical population of 500 patients via Monte Carlo resampling. Descriptive statistics were derived in order to determine a dosing regimen with the percentage of subjects with $C_{max}$ < 150 μg/ml to optimize the safety profile of IMGN853. For the QWx3 in 4W regimen, the 1.5, 2.0 and 2.5 mg/kg dose levels resulted in 99%, 95% and 90% of the population with a $C_{max}$ < 150 μg/ml, respectively.

Example 6

*In Vivo* Antitumor Activity and Predicted Pharmacokinetics of Multi-dose IMGN853

The plasma concentrations of intact IMGN853 conjugate administered intravenously at a dose of 10 mg/kg in female CD-1 mice was determined by ELISA at various time points post injection. Pharmacokinetic (PK) analyses were performed using the standard algorithms of the non-compartmental pharmacokinetic analysis program (201), WinNonlin, Professional version 6.1 (Pharsight, Mountain View, CA). The maximal concentration (Cmax), the total area under the concentration-time curve ($AUC_{0-\infty}$), the half-life ($t_{1/2}$) in terminal elimination phase, the total blood clearance (CL) and the volume of distribution at steady-state (Vss) were estimated. The values of the first order rate constant for determining conjugate $t_{1/2}$ were evaluated by using the concentration data from 1 to 28 days post-administration. The values of the first order rate constant for determining antibody $t_{1/2}$ were evaluated by using the concentration data from 1 to 28 days post-administration. Based on the measured values generated at the 10 mg/kg dose, PK simulations were performed with WinNonlin using various dose levels, with both single and multi-dose schedules. The resulting parameters were evaluated in comparison to the anti-tumor activity of IMGN853 at various dose levels and schedules in NCI-H2110 (non-small cell lung cancer, NSCLC) xenografts in female SCID mice.

When IMGN853 was administered as a single injection, dose-dependent antitumor activity was observed in the NCI-H2110 model. All dose levels (2.8, 5.6, and 8.5 mg/kg) were highly active with T/C values <10%, yet there were an increase in numbers of complete tumor regressions (CR) with increased IMGN853 dose. The predicted plasma PK parameters also showed a dose-dependent increase in Cmax (maximal plasma concentration), Cavg (average plasma concentration), and exposure ($AUC_{0-540}$ hrs). Thus, the activity of single doses of IMGN853 was shown to be dose-dependent and predictable based on plasma PK parameters, as shown in Figure 9.

In contrast to the single-dose activity, multi-dose schedules of IMGN853 show that activity does not depend on Cmax (Figure 10). IMGN853 administered at a dose of 2.8 mg/kg x 3, either daily or every 3 days schedule (total dose of 8.4 mg/kg) had similar activity to single-dose IMGN853 at 8.5 mg/kg. Interestingly, total exposure (AUC) and average plasma concentration (Cavg) of conjugate was comparable among the treatment groups, while the Cmax was highest in the 8.5 mg/kg single-dose group. The activity observed with multi-dose schedules suggests that this method of dosing has greater activity, as there were tumor-free animals remaining at the end of the study, versus no tumor-free animals in the single high dose group.

Additional IMGN853 dose levels and schedules were evaluated for activity against NCI-H2110 xenografts, with results consistently demonstrating that multi-dose administration is equally active or has better activity than single-dose IMGN853. The predicted average plasma concentration of IMGN853 administered as a single-dose of 5.6 mg/kg IMGN853 was comparable to that of a 1.4 mg/kg daily for 3 days. Despite having a lower total dose (4.2 mg/kg), Cmax and exposure ($AUC_{0-540}$) the 1.4 mg/kg qd x3 had comparable antitumor activity in vivo (Figure 11). These results suggest that maintenance of a certain minimal plasma concentration is critical for activity.

A weekly schedule of IMGN853 with matched total dose to a single high dose of IMGN853 were also found to have comparable activity in vivo (Figure 12). Again, maintenance of an average plasma concentration above a minimum threshold was required for activity, with the single-dose IMGN853 (8.5 mg/kg) resulting in a slightly higher Cavg and AUC but with comparable overall activity. The key difference in predicted pharmacokinetic parameters with single versus

multi-dose schedule is the dramatic reduction in Cmax. The weekly dosing Cmax is predicted to be almost 60% lower than the Cmax of single-dose IMGN853. As there is no apparent activity benefit of reaching a higher Cmax, avoiding high plasma concentrations can be beneficial in reducing toxicity.

It is to be appreciated that the Detailed Description section, and not the Summary and Abstract sections, is intended to be used to interpret the claims. The Summary and Abstract sections sets forth one or more, but not all, exemplary embodiments of the present invention as contemplated by the inventor(s), and thus, are not intended to limit the present invention and the appended claims in any way.

The present invention has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

It is to be understood that the phraseology or terminology herein is for the purpose of description such that the terminology or phraseology of the present specification is to be interpreted by the skilled artisan in light of the teachings and guidance.

The breadth and scope of the present invention should be defined only in accordance with the claims.

**SEQUENCES**

SEQ ID NO:1 - human folate receptor 1

MAQRMTTQLLLLLVWVAVVGEAQTRIAWARTELLNVCMNAKHHKEKPGPEDKLHEQ
CRPWRKNACCSTNTSQEAHKDVSYLYRFNWNHCGEMAPACKRHFIQDTCLYECSPNLG
PWIQQVDQSWRKERVLNVPLCKEDCEQWWEDCRTSYTCKSNWHKGWNWTSGFNKCA
VGAACQPFHFYFPTPTVLCNEIWTHSYKVSNYSRGSGRCIQMWFDPAQGNPNEEVARFY
AAAMSGAGPWAAWPFLLSLALMLLWLLS

SEQ ID NO:2 - human folate receptor 1 nucleic acid sequence

atggctcagcggatgacaacacagctgctgctccttctagtgtgggtggctgtagtaggggaggctcagacaaggattgcatgggccagga
ctgagcttctcaatgtctgcatgaacgccaagcaccacaaggaaaagccaggccccgaggacaagttgcatgagcagtgtcgaccctgga
ggaagaatgcctgctgttctaccaacaccagccaggaagcccataaggatgtttcctacctatatagattcaactggaaccactgtggagag
atggcacctgcctgcaaacggcatttcatccaggacacctgcctctacgagtgctcccccaacttggggccctggatccagcaggtggatc
agagctggcgcaaagagcgggtactgaacgtgcccctgtgcaaagaggactgtgagcaatggtgggaagattgtcgcacctcctacacct
gcaagagcaactggcacaagggctggaactggacttcagggtttaacaagtgcgcagtgggagctgcctgccaacctttccatttctacttc
cccacacccactgttctgtgcaatgaaatctggactcactcctacaaggtcagcaactacagccgagggagtggccgctgcatccagatgtg
gttcgacccagcccagggcaaccccaatgaggaggtggcgaggttctatgctgcagccatgagtggggctgggccctgggcagcctggc
ctttcctgcttagcctggccctaatgctgctgtggctgctcagc

SEQ ID NO:3 - huMov19 vHC

QVQLVQSGAEVVKPGASVKISCKASGYTFTGYFMNWVKQSPGQSLEWIGRIHPYDGDT
FYNQKFQGKATLTVDKSSNTAHMELLSLTSEDFAVYYCTRYDGSRAMDYWGQGTTVT
VSS

SEQ ID NO:4 - huMov19 vLCv1.00

DIVLTQSPLSLAVSLGQPAIISCKASQSVSFAGTSLMHWYHQKPGQQPRLLIYRASNLEA
GVPDRFSGSGSKTDFTLNISPVEAEDAATYYCQQSREYPYTFGGGTKLEIKR

SEQ ID NO:5 - huMov19 vLCv1.60

DIVLTQSPLSLAVSLGQPAIISCKASQSVSFAGTSLMHWYHQKPGQQPRLLIYRASNLEA
GVPDRFSGSGSKTDFTLTISPVEAEDAATYYCQQSREYPYTFGGGTKLEIKR

SEQ ID NO:6 - huMov19 vLC CDR1
KASQSVSFAGTSLMH

SEQ ID NO:7 - huMov19 vLC CDR2
RASNLEA

SEQ ID NO:8 - huMov19 vLC CDR3
QQSREYPYT

SEQ ID NO:9 - huMov19 vHC CDR1
GYFMN

SEQ ID NO:10 - huMov19 vHC CDR2 - Kabat Defined
RIHPYDGDTFYNQKFQG

SEQ ID NO:11 - huMov19 vHC CDR2 - Abm Defined
RIHPYDGDTF

SEQ ID NO:12 - huMov19 vHC CDR3
YDGSRAMDY

SEQ ID NO:13 - huMov19 HC amino acid sequence

QVQLVQSGAEVVKPGASVKISCKASGYTFTGYFMNWVKQSPGQSLEWIGRIHPYDGDT
FYNQKFQGKATLTVDKSSNTAHMELLSLTSEDFAVYYCTRYDGSRAMDYWGQGTTVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK

SEQ ID NO:14 - huMov19 LCv1.00

DIVLTQSPLSLAVSLGQPAIISCKASQSVSFAGTSLMHWYHQKPGQQPRLLIYRASNLEA
GVPDRFSGSGSKTDFTLNISPVEAEDAATYYCQQSREYPYTFGGGTKLEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC

SEQ ID NO:15 - huMov19 LCv1.60

DIVLTQSPLSLAVSLGQPAIISCKASQSVSFAGTSLMHWYHQKPGQQPRLLIYRASNLEA
GVPDRFSGSGSKTDFTLTISPVEAEDAATYYCQQSREYPYTFGGGTKLEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC

SEQ ID NO:16 – muMov19 vHC CDR2 – Kabat Defined
RIHPYDGDTFYNQNFKD

## Claims

1. An immunoconjugate which binds to FOLR1 polypeptide for use in the treatment of a FOLR1-expressing cancer, wherein the immunoconjugate comprises a maytansinoid and an antibody or antigen-binding fragment thereof that comprises the variable light chain (VL) complementarity determining region (CDR)-1 of SEQ ID NO: 6, the VL CDR-2 of SEQ ID NO: 7, the VL CDR-3 of SEQ ID NO: 8, the variable heavy chain (VH) CDR-1 of SEQ ID NO: 9, the VH CDR-2 of SEQ ID NO: 11, and the VH CDR-3 of SEQ ID NO: 12, wherein the cancer is ovarian cancer, cancer of the peritoneum, endometrial cancer, or uterine cancer, preferably, wherein the cancer is a FOLR1-expressing ovarian cancer, and wherein the immunoconjugate is administered at a dose of 6 milligrams (mg) per kilogram (kg) of adjusted ideal body weight (AIBW).

2. The immunoconjugate of claim 1, wherein the antibody or antigen-binding fragment thereof comprises a variable heavy chain domain comprising SEQ ID NO: 3 and a variable light chain domain comprising SEQ ID NO: 4 or SEQ ID NO: 5.

3. The immunoconjugate of claims 1 or 2, wherein the immunoconjugate comprises 1-10 maytansinoid molecules, more preferably 2-5 maytansinoid molecules, and most preferably 3-4 maytansinoid molecules.

4. The immunoconjugate of any one of claims 1-3, wherein the maytansinoid is DM4.

5. The immunoconjugate of any one of claims 1-4, wherein the immunoconjugate comprises the linker sulfo-SPDB.

6. The immunoconjugate of any one of claims 1-3, wherein the antibody comprises (i) a heavy chain comprising the same amino acid sequence as the amino acid sequence of the heavy chain encoded by the plasmid deposited with the American Type Culture Collection (ATCC) as PTA-10772 and (ii) a light chain comprising the same amino acid sequence as the amino acid sequence of the light chain encoded by the plasmid deposited with the ATCC as PTA-10774, wherein the maytansinoid is DM4, and wherein the DM4 is linked to the antibody by sulfo-SPDB.

7. The immunoconjugate of any one of claims 1-3, wherein the antibody comprises (i) a heavy chain comprising the amino acid sequence of SEQ ID NO: 13 and (ii) a light chain comprising the amino acid sequence of SEQ ID NO: 15, wherein the maytansinoid is DM4, and wherein the DM4 is linked to the antibody by sulfo-SPDB.

8. The immunoconjugate of any one of claims 1-7, wherein the immunoconjugate is formulated for IV administration or parenteral administration.

9. The immunoconjugate of any one of claims 1-8, wherein the cancer is ovarian cancer or cancer of the peritoneum, optionally wherein the ovarian cancer is epithelial ovarian cancer or wherein the ovarian cancer is platinum resistant, relapsed, or refractory.

10. An immunoconjugate which binds to FOLR1 polypeptide for use in the treatment of a FOLR1-expressing ovarian cancer, wherein the immunoconjugate comprises DM4 and an antibody comprising (i) a heavy chain comprising the same amino acid sequence as the amino acid sequence of the heavy chain encoded by the plasmid deposited with the American Type Culture Collection (ATCC) as PTA-10772 and (ii) a light chain comprising the same amino acid sequence as the amino acid sequence of the light chain encoded by the plasmid deposited with the ATCC as PTA-10774, wherein the DM4 is linked to the antibody by sulfo-SPDB, wherein the immunoconjugate is formulated for IV administration, and wherein the immunoconjugate is administered at a dose of 6 milligrams (mg) per kilogram

(kg) of adjusted ideal body weight (AIBW).

**11.** An immunoconjugate which binds to FOLR1 polypeptide for use in the treatment of a FOLR1-expressing cancer of the peritoneum, wherein the immunoconjugate comprises DM4 and an antibody comprising (i) a heavy chain comprising the same amino acid sequence as the amino acid sequence of the heavy chain encoded by the plasmid deposited with the American Type Culture Collection (ATCC) as PTA-10772 and (ii) a light chain comprising the same amino acid sequence as the amino acid sequence of the light chain encoded by the plasmid deposited with the ATCC as PTA-10774, wherein the DM4 is linked to the antibody by sulfo-SPDB, wherein the immunoconjugate is formulated for IV administration, and wherein the immunoconjugate is administered at a dose of 6 milligrams (mg) per kilogram (kg) of adjusted ideal body weight (AIBW).

**12.** The immunoconjugate of any one of claims 1-11, wherein a sample obtained from the patient exhibits FOLR1 expression as measured by immunohistochemistry (IHC).

**13.** The immunoconjugate of 12, wherein the sample has a staining intensity of at least 2 hetero, at least 2 homo, at least 3 hetero, or at least 3 homo.

**14.** The immunoconjugate of any one of claims 1-13, wherein the immunoconjugate is administered once every three weeks or once every four weeks.

**15.** The immunoconjugate of claim 10 or 11, wherein the immunoconjugate comprises 1-10 maytansinoid molecules per antibody, more preferably 2-5 maytansinoid molecules per antibody, and most preferably 3-4 maytansinoid molecules per antibody.

**Patentansprüche**

**1.** Immunkonjugat, das an FOLR1-Polypeptid bindet, zur Verwendung bei der Behandlung eines FOLR1-exprimierenden Krebses, wobei das Immunkonjugat ein Maytansinoid und einen Antikörper oder ein Antigenbindendes Fragment davon umfasst, der oder das die variable leichte Kette (VL)-komplementaritätsbestimmende Region (CDR)-1 von SEQ ID NR: 6, die VL-CDR-2 von SEQ ID NR: 7, die VL-CDR-3 von SEQ ID NR: 8, die variable schwere Kette (VH)-CDR-1 von SEQ ID NR: 9, die VH-CDR-2 von SEQ ID NR: 11 und die VH-CDR-3 von SEQ ID NR: 12 umfasst, wobei der Krebs Eierstockkrebs, Krebs des Peritoneums, Endometriumkrebs oder Gebärmutterkrebs ist, vorzugsweise wobei der Krebs ein FOLR1-exprimierender Eierstockkrebs ist und wobei das Immunkonjugat in einer Dosis von 6 Milligramm (mg) pro Kilogramm (kg) von angepasstem idealem Körpergewicht (AIBW) verabreicht wird.

**2.** Immunkonjugat nach Anspruch 1, wobei der Antikörper oder das Antigenbindende Fragment davon eine variable schwere Kette-Domäne, umfassend SEQ ID NR: 3, und eine variable leichte Kette-Domäne, umfassend SEQ ID NR: 4 oder SEQ ID NR: 5, umfasst.

**3.** Immunkonjugat nach Anspruch 1 oder 2, wobei das Immunkonjugat 1-10 Maytansinoid-Moleküle, stärker bevorzugt 2-5 Maytansionoid-Moleküle und am meisten bevorzugt 3-4 Maytansionoid-Moleküle umfasst.

**4.** Immunkonjugat nach einem beliebigen der Ansprüche 1-3, wobei das Maytansinoid DM4 ist.

**5.** Immunkonjugat nach einem beliebigen der Ansprüche 1-4, wobei das Immunkonjugat den Linker Sulfo-SPDB umfasst.

**6.** Immunkonjugat nach einem beliebigen der Ansprüche 1-3, wobei der Antikörper (i) eine schwere Kette, umfassend die gleiche Aminosäuresequenz wie die Aminosäuresequenz der schweren Kette, die durch das Plasmid kodiert wird, das bei der American Type Culture Collection (ATCC) als PTA-10772 hinterlegt ist, und (ii) eine leichte Kette, umfassend die gleiche Aminosäuresequenz wie die Aminosäuresequenz der leichten Kette, die durch das Plasmid kodiert wird, das bei der ATCC als PTA-10774 hinterlegt ist, umfasst, wobei das Maytansinoid DM4 ist und wobei das DM4 mit dem Antikörper durch Sulfo-SPDB verknüpft ist.

**7.** Immunkonjugat nach einem beliebigen der Ansprüche 1-3, wobei der Antikörper (i) eine schwere Kette, umfassend die Aminosäuresequenz von SEQ ID NR: 13, und (ii) eine leichte Kette, umfassend die Aminosäuresequenz von SEQ ID NR: 15, umfasst, wobei das Maytansinoid DM4 ist und wobei das DM4 mit dem Antikörper durch Sulfo-SPDB verknüpft ist.

8. Immunkonjugat nach einem beliebigen der Ansprüche 1-7, wobei das Immunkonjugat für IV-Verabreichung oder parenterale Verabreichung formuliert ist.

9. Immunkonjugat nach einem beliebigen der Ansprüche 1-8, wobei der Krebs Eierstockkrebs oder Krebs des Peritoneums ist, optional wobei der Eierstockkrebs epithelialer Eierstockkrebs ist oder wobei der Eierstockkrebs Platinresistent, rückfällig oder refraktorisch ist.

10. Immunkonjugat, das an FOLR1-Polypeptid bindet, zur Verwendung bei der Behandlung eines FOLR1-exprimierenden Eierstockkrebses, wobei das Immunkonjugat DM4 und einen Antikörper, umfassend (i) eine schwere Kette, umfassend die gleiche Aminosäuresequenz wie die Aminosäuresequenz der schweren Kette, die durch das Plasmid kodiert wird, das bei der American Type Culture Collection (ATCC) als PTA-10772 hinterlegt ist, und (ii) eine leichte Kette, umfassend die gleiche Aminosäuresequenz wie die Aminosäuresequenz der leichten Kette, die durch das Plasmid kodiert wird, das bei der ATCC als PTA-10774 hinterlegt ist, umfasst, wobei das DM4 mit dem Antikörper durch Sulfo-SPDB verknüpft ist, wobei das Immunkonjugat für IV-Verabreichung formuliert ist und wobei das Immunkonjugat in einer Dosis von 6 Milligramm (mg) pro Kilogramm (kg) von angepasstem idealem Körpergewicht (AIBW) verabreicht wird.

11. Immunkonjugat, das an das FOLR1-Polypeptid bindet, zur Verwendung bei der Behandlung eines FOLR1-exprimierenden Krebses des Peritoneums, wobei das Immunkonjugat DM4 und einen Antikörper, umfassend (i) eine schwere Kette, umfassend die gleiche Aminosäuresequenz wie die Aminosäuresequenz der schweren Kette, die durch das Plasmid kodiert wird, das bei der American Type Culture Collection (ATCC) als PTA-10772 hinterlegt ist, und (ii) eine leichte Kette, umfassend die gleiche Aminosäuresequenz wie die Aminosäuresequenz der leichten Kette, die durch das Plasmid kodiert wird, das bei der ATCC als PTA-10774 hinterlegt ist, umfasst, wobei das DM4 mit dem Antikörper durch Sulfo-SPDB verknüpft ist, wobei das Immunkonjugat für IV-Verabreichung formuliert ist und wobei das Immunkonjugat in einer Dosis von 6 Milligramm (mg) pro Kilogramm (kg) von angepasstem idealem Körpergewicht (AIBW) verabreicht wird.

12. Immunkonjugat nach einem beliebigen der Ansprüche 1-11, wobei eine Probe, die von dem Patienten erhalten wird, FOLR1-Expression zeigt, wie durch Immunhistochemie (IHC) gemessen.

13. Immunkonjugat nach 12, wobei die Probe eine Färbungsintensität von mindestens 2 Hetero, mindestens 2 Homo, mindestens 3 Hetero oder mindestens 3 Homo aufweist.

14. Immunkonjugat nach einem beliebigen der Ansprüche 1-13, wobei das Immunkonjugat einmal alle drei Wochen oder einmal alle vier Wochen verabreicht wird.

15. Immunkonjugat nach Anspruch 10 oder 11, wobei das Immunkonjugat 1-10 Maytansinoid-Moleküle pro Antikörper, stärker bevorzugt 2-5 Maytansinoid-Moleküle pro Antikörper und am stärksten bevorzugt 3-4 Maytansinoid-Moleküle pro Antikörper umfasst.

**Revendications**

1. Immunoconjugué qui se lie au polypeptide FOLR1 pour utilisation dans le traitement d'un cancer exprimant FOLR1, où l'immunoconjugué comprend un maytansinoïde et un anticorps ou un de ses fragments de liaison à un antigène qui comprend la région de détermination de complémentarité (CDR) -1 de la chaîne légère variable (VL) de la SEQ ID n° 6, la CDR-2 de VL de la SEQ ID n° 7, la CDR-3 de VL de la SEQ ID n° 8, la CDR-1 de chaîne lourde variable (VH) de la SEQ ID n° 9, la CDR-2 de VH de la SEQ ID n° 11 et la CDR-3 de VH de la SEQ ID n° 12, où le cancer est le cancer des ovaires, le cancer du péritoine, le cancer endométrial ou le cancer utérin, de préférence, où le cancer est un cancer des ovaires exprimant FOLR1, et où l'immunoconjugué est administré à une dose de 6 milligrammes (mg) par kilogramme (kg) de poids corporel idéal ajusté (PCIA).

2. Immunoconjugué selon la revendication 1, dans lequel l'anticorps ou un de ses fragments de liaison à un antigène comprend un domaine de chaîne lourde variable comprenant la SEQ ID n° 3 et un domaine de chaîne légère variable comprenant la SEQ ID n° 4 ou la SEQ ID n° 5.

3. Immunoconjugué selon les revendications 1 ou 2, où l'immunoconjugué comprend 1 à 10 molécule de maytansinoïde, plus préférablement 2 à 5 molécules de maytansinoïde et le plus préférablement 3 à 4 molécules de maytansinoïde.

4. Immunoconjugué selon l'une quelconque des revendications 1 à 3, dans lequel le maytansinoïde est le DM4.

5. Immunoconjugué selon l'une quelconque des revendications 1 à 4, où l'immunoconjugué comprend le lieur sulfo-SPDB.

6. Immunoconjugué selon l'une quelconque des revendications 1 à 3, dans lequel l'anticorps comprend (i) une chaîne lourde comprenant la même séquence d'acides aminés que la séquence d'acides aminés de la chaîne lourde codée par le plasmide déposé à l'American Type Culture Collection (ATCC) sous le nom de PTA-10772 et (ii) une chaîne légère comprenant la même séquence d'acides aminés que la séquence d'acides aminés de la chaîne légère codée par le plasmide déposé à l'ATCC sous le nom de PTA-10774, où le maytansinoïde est le DM4, et où le DM4 est lié à l'anticorps par le sulfo-SPDB.

7. Immunoconjugué selon l'une quelconque des revendications 1 à 3, dans lequel l'anticorps comprend (i) une chaîne lourde comprenant la séquence d'acides aminés de la SEQ ID n° 13 et (ii) une chaîne légère comprenant la séquence d'acides aminés de la SEQ ID n° 15, où le maytansinoïde est le DM4, et où le DM4 est lié à l'anticorps par le sulfo-SPDB.

8. Immunoconjugué selon l'une quelconque des revendications 1 à 7, où l'immunoconjugué est formulé pour une administration IV ou une administration parentérale.

9. Immunoconjugué selon l'une quelconque des revendications 1 à 8, dans lequel le cancer est le cancer des ovaires ou le cancer du péritoine, éventuellement où le cancer des ovaires est le cancer des ovaires épithélial ou où le cancer des ovaires est résistant au platine, en rechute ou réfractaire.

10. Immunoconjugué qui se lie au polypeptide FOLR1 pour utilisation dans le traitement d'un cancer des ovaires exprimant FOLR1, où l'immunoconjugué comprend le DM4 et un anticorps comprenant (i) une chaîne lourde comprenant la même séquence d'acides aminés que la séquence d'acides aminés de la chaîne lourde codée par le plasmide déposé à l'American Type Culture Collection (ATCC) sous le nom de PTA-10772 et (ii) une chaîne légère comprenant la même séquence d'acides aminés que la séquence d'acides aminés de la chaîne légère codée par le plasmide déposé à l'ATCC sous le nom de PTA-10774, où le DM4 est lié à l'anticorps par le sulfo-SPDB, où l'immunoconjugué est formulé pour l'administration IV, et où l'immunoconjugué est administré à une dose de 6 milligrammes (mg) par kilogramme (kg) de poids corporel idéal ajusté (PCIA).

11. Immunoconjugué qui se lie au polypeptide FOLR1 pour utilisation dans le traitement du cancer du péritoine exprimant FOLR1, où l'immunoconjugué comprend le DM4 et un anticorps comprenant (i) une chaîne lourde comprenant la même séquence d'acides aminés que la séquence d'acides aminés de la chaîne lourde codée par le plasmide déposé à l'American Type Culture Collection (ATCC) sous le nom de PTA-10772 et (ii) une chaîne légère comprenant la même séquence d'acides aminés que la séquence d'acides aminés de la chaîne légère codée par le plasmide déposé à l'ATCC sous le nom de PTA-10774, où le DM4 est lié à l'anticorps par le sulfo-SPDB, où l'immunoconjugué est formulé pour l'administration IV, et où l'immunoconjugué est administré à une dose de 6 milligrammes (mg) par kilogramme (kg) de poids corporel idéal ajusté (PCIA).

12. Immunoconjugué selon l'une quelconque des revendications 1 à 11, dans lequel un échantillon obtenu du patient présente une expression de FOLR1 telle que mesurée par immunohistochimie (IHC).

13. Immunoconjugué selon la revendication 12, dans lequel l'échantillon a une intensité de coloration d'au moins 2 hétéro, d'au moins 2 homo, d'au moins 3 hétéro ou d'au moins 3 homo.

14. Immunoconjugué selon l'une quelconque des revendications 1 à 13, où l'immunoconjugué est administré une fois toutes les trois semaines ou une fois toutes les quatre semaines.

15. Immunoconjugué selon la revendication 10 ou 11, où l'immunoconjugué comprend 1 à 10 molécules de maytansinoïde par anticorps, plus préférablement 2 à 5 molécules de maytansinoïde par anticorps et le plus préférablement 3 à 4 molécules de maytansinoïde par anticorps.

**Figure 1A**

IMGN853 Pharmacokinetic Results

| Dose (mg/kg) | 0.15 (n=2) | 0.5 (n=1) | 1.0 (n=1) | 2.0 (n=1) | 3.3 (n=3) | 5.0 (n=3) | 7.0 (n=4) |
|---|---|---|---|---|---|---|---|
| Cmax (ug/mL) | 2.9 | 10.5 | 22.1 | 65.7 | 96.6 (16.4) | 108 (32.7) | 179 (21.8) |
| Half-life (hr) | 35.4 | 41.2 | 70.1 | 69.9 | 99.5 (15.7) | 105 (4.4) | 87.6 (11.5) |
| Half-life (d) | 1.5 | 1.7 | 2.9 | 2.9 | 4.1 (0.65) | 4.4 (1.0) | 3.6 (0.5) |
| $AUC_{(0-inf)}$ (hr*ug/mL) | 150.9 | 596.6 | 1779 | 6505 | 12188 (2581) | 12708(2112) | 17559(2850) |
| $AUC_{(0-168)}$(hr*ug/mL) | 104.6 | 496.6 | 1678 | 5330 | 8178 (1129) | 8254 (1771) | 12177(1621) |
| CL (mL/hr/kg) | 1.1 | 0.8 | 0.6 | 0.3 | 0.3 (0.06) | 0.4 (0.07) | 0.4 (0.06) |
| Vss (mL/kg) | 51.8 | 46.7 | 54.9 | 28.2 | 38.6 ( 5.2) | 61.2 (16.1) | 52.8 (8.3) |

**Figure 1B**

# IMGN853 Pharmacokinetic Results

| Cohort Dose (mg/kg) | N | $C_{Max}$ (µg/ml) | $T_{1/2}$ (days) | Cl (ml/hr/kg) | $V_{ss}$ (ml/kg) | $AUC_{0_\infty}$ (hr*µg/ml) |
|---|---|---|---|---|---|---|
| **0.15** | 2 | 2.90 (± 0.47) | 1.5 (± 0.2) | 1.08 (± 0.38) | 53.2 (± 11.2) | 149 (± 52) |
| **0.5** | 1 | 10.55 | 3.2 | 0.58 | 59.2 | 865 |
| **1** | 1 | 22.09 | 3.4 | 0.56 | 57.0 | 1,777 |
| **2** | 1 | 65.73 | 5.0 | 0.30 | 32.4 | 6,757 |
| **3.3** | 3 | 96.64 (± 16.39) | 4.7 (± 0.3) | 0.26 (± 0.05) | 36.82 (± 5.7) | 12,915 (± 2,056) |
| **5** | 10 | 141.90 (± 41.63) | 4.9 (± 1.2) | 0.35 (± 0.08) | 51.9 (± 17.0) | 15,079 (± 3,740) |
| **7** | 5 | 172.98 (± 23.58) | 6.2 (± 1.3) | 0.36 (± 0.08) | 65.7 (± 8.9) | 20,146 (± 4,099) |

Figure 2A-C

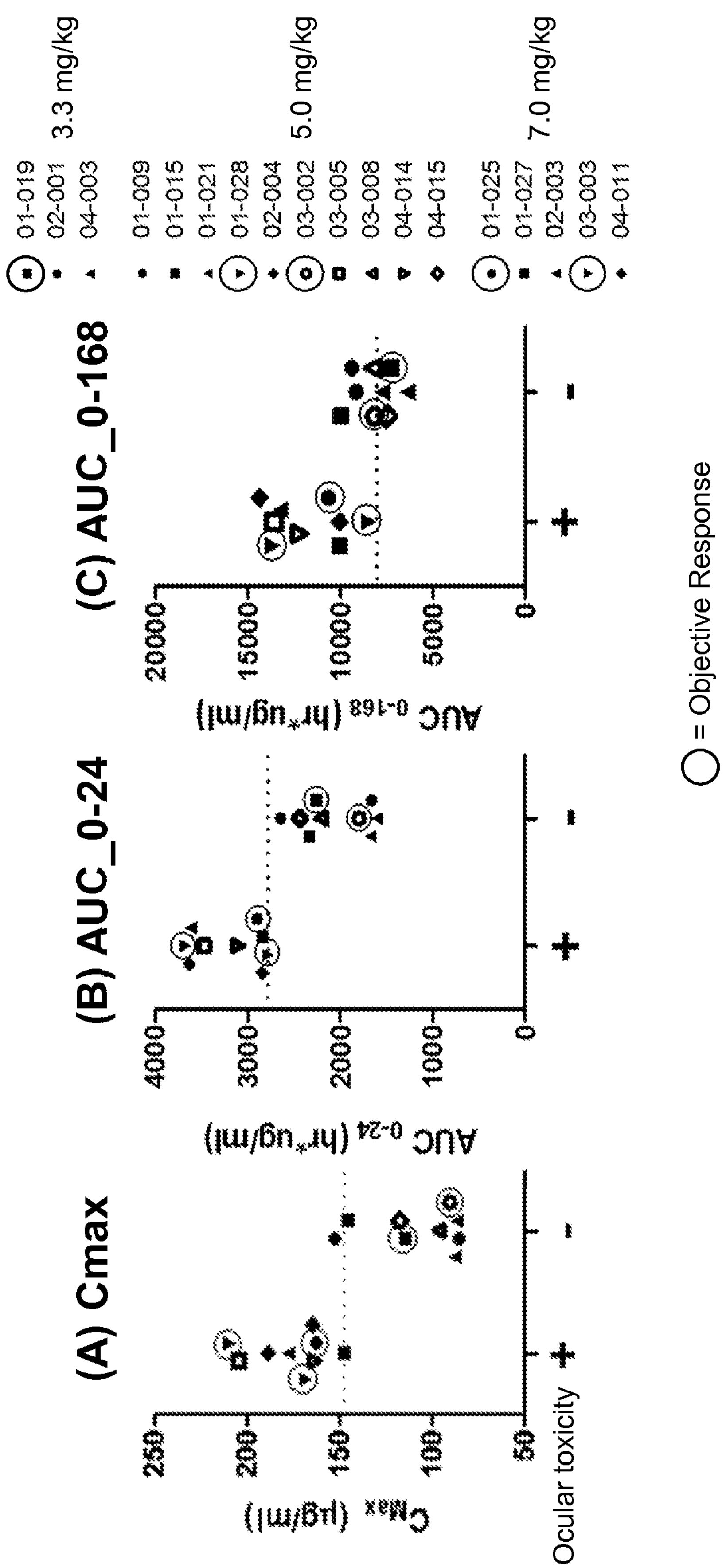
(A) Cmax
(B) AUC_0-24
(C) AUC_0-168
Cmax (µg/ml)
AUC 0-24 (hr*ug/ml)
AUC 0-168 (hr*ug/ml)
250
200
150
100
50
4000
3000
2000
1000
0
20000
15000
10000
5000
0
Ocular toxicity
+
-
01-019
02-001
04-003
3.3 mg/kg
01-009
01-015
01-021
01-028
02-004
03-002
03-005
03-008
04-014
04-015
5.0 mg/kg
01-025
01-027
02-003
03-003
04-011
7.0 mg/kg
= Objective Response

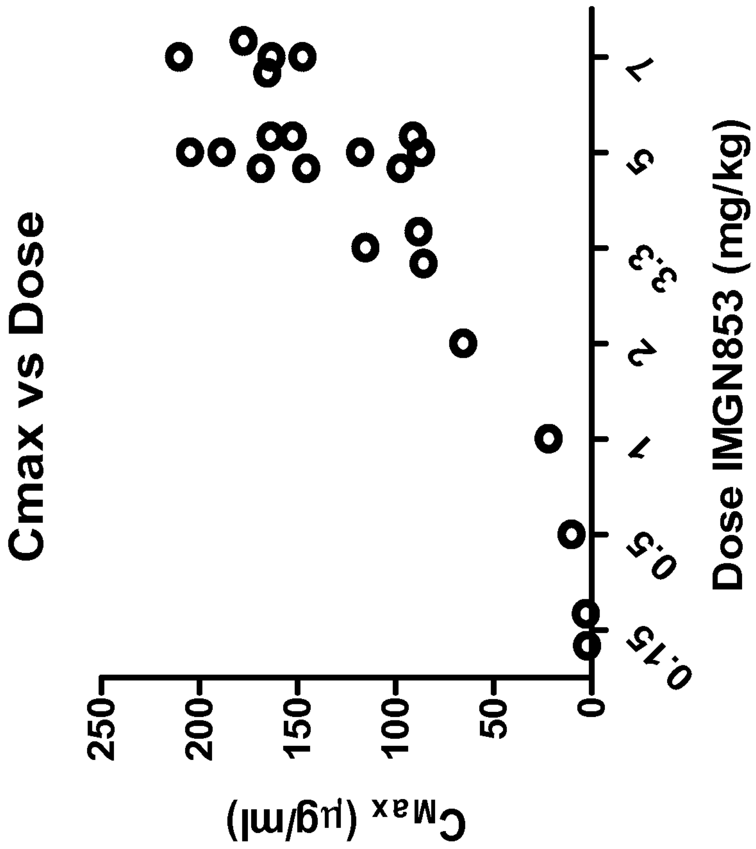
Cmax vs Dose
Cmax (µg/ml)
250
200
150
100
50
0
0.15
0.5
1
2
3.3
5
7
Dose IMGN853 (mg/kg)

Figure 3

Figure 4

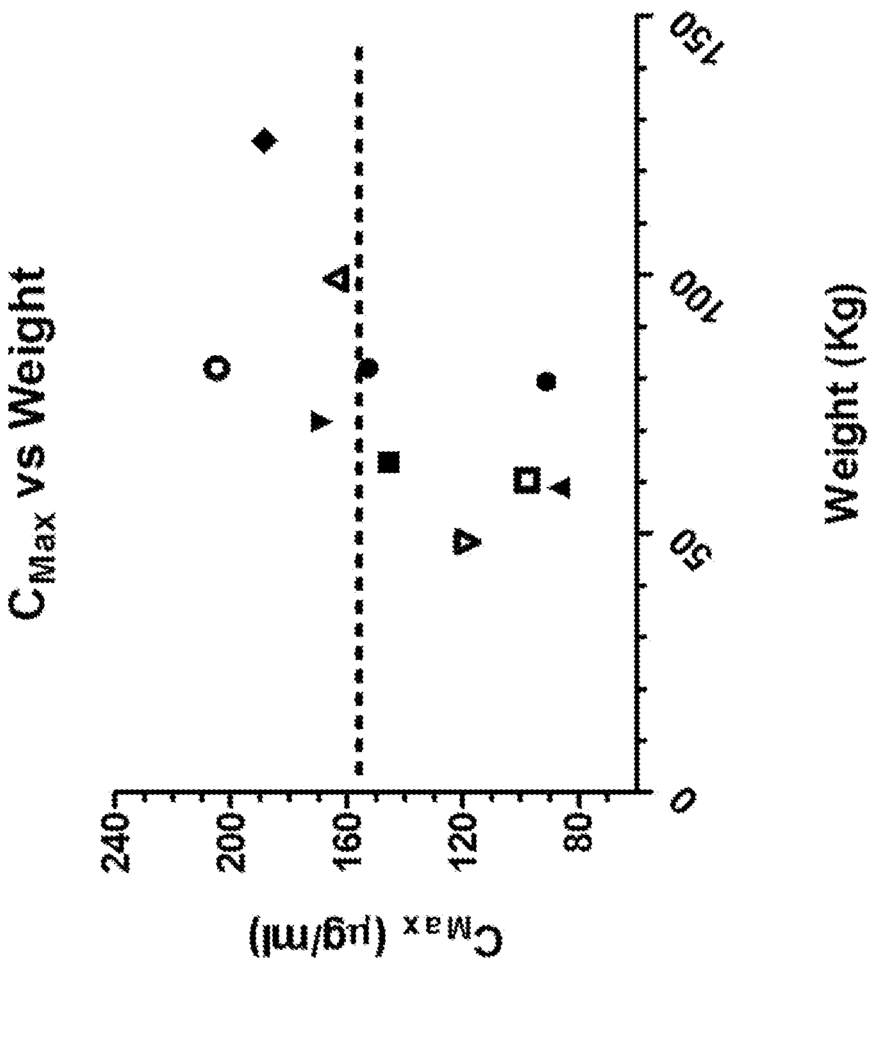
C Max vs Weight
C Max (µg/ml)
240
200
160
120
80
0
50
100
150
Weight (Kg)

Figure 5

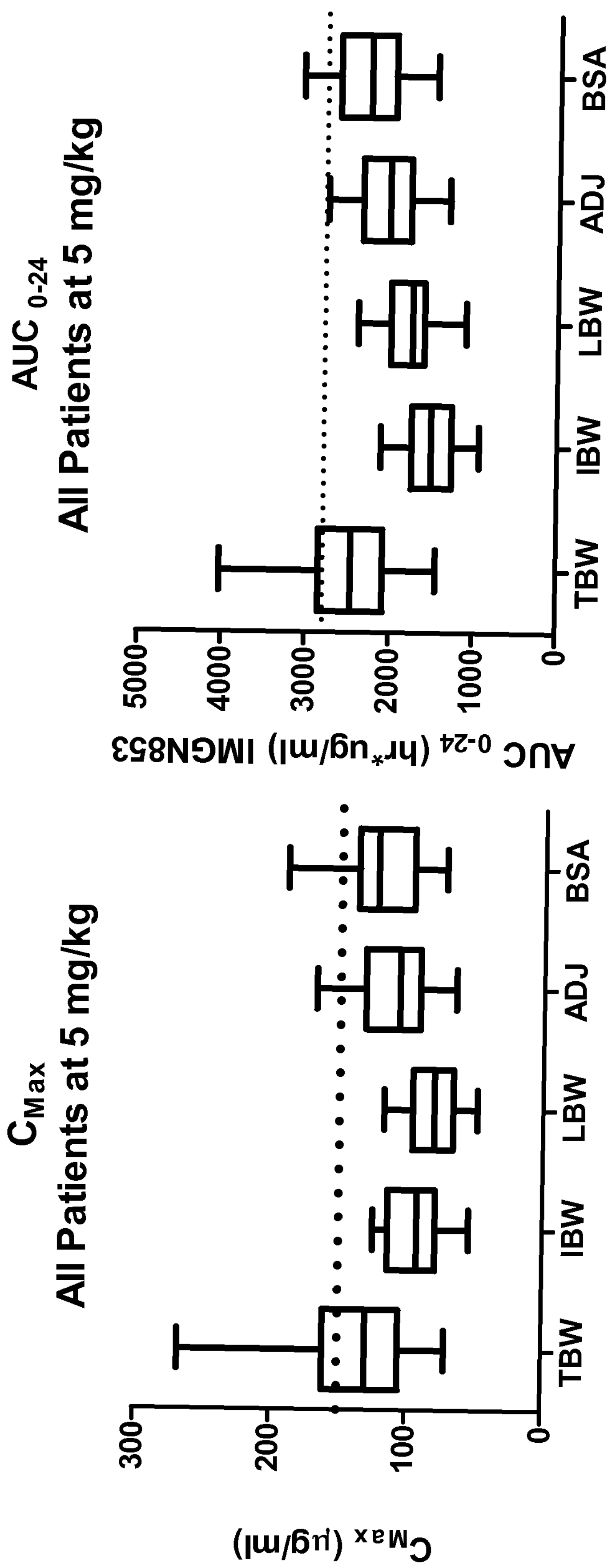
$C_{Max}$
All Patients at 5 mg/kg
$C_{Max}$ (µg/ml)
300
200
100
0
TBW
IBW
LBW
ADJ
BSA
$AUC_{0-24}$
All Patients at 5 mg/kg
$AUC_{0-24}$ (hr*ug/ml) IMGN853
5000
4000
3000
2000
1000
0
TBW
IBW
LBW
ADJ
BSA

# Figure 6

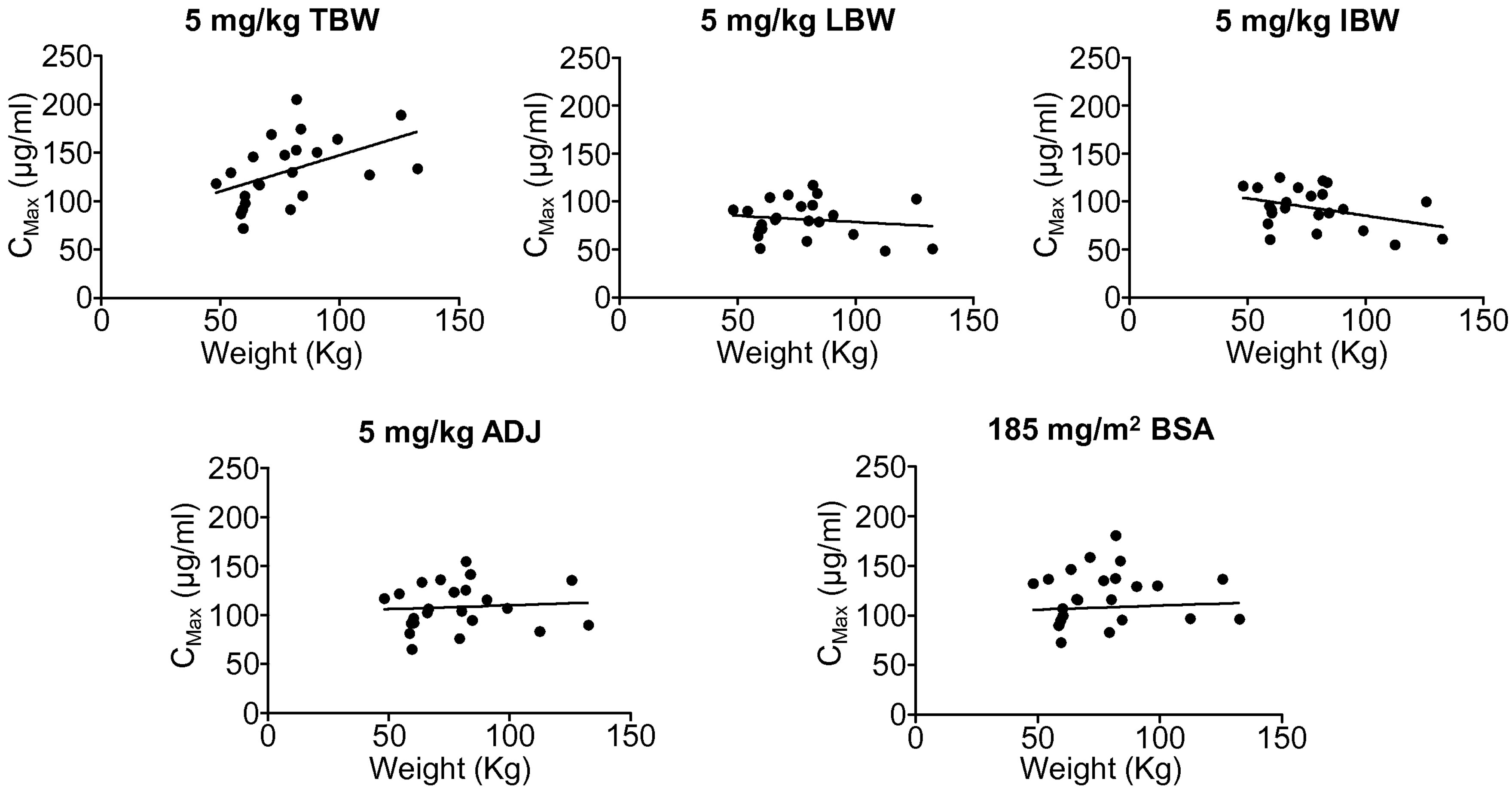
5 mg/kg TBW
5 mg/kg LBW
5 mg/kg IBW
5 mg/kg ADJ
185 mg/m2 BSA
CMax (µg/ml)
0
50
100
150
200
250
Weight (Kg)
0
50
100
150

# Figure 7

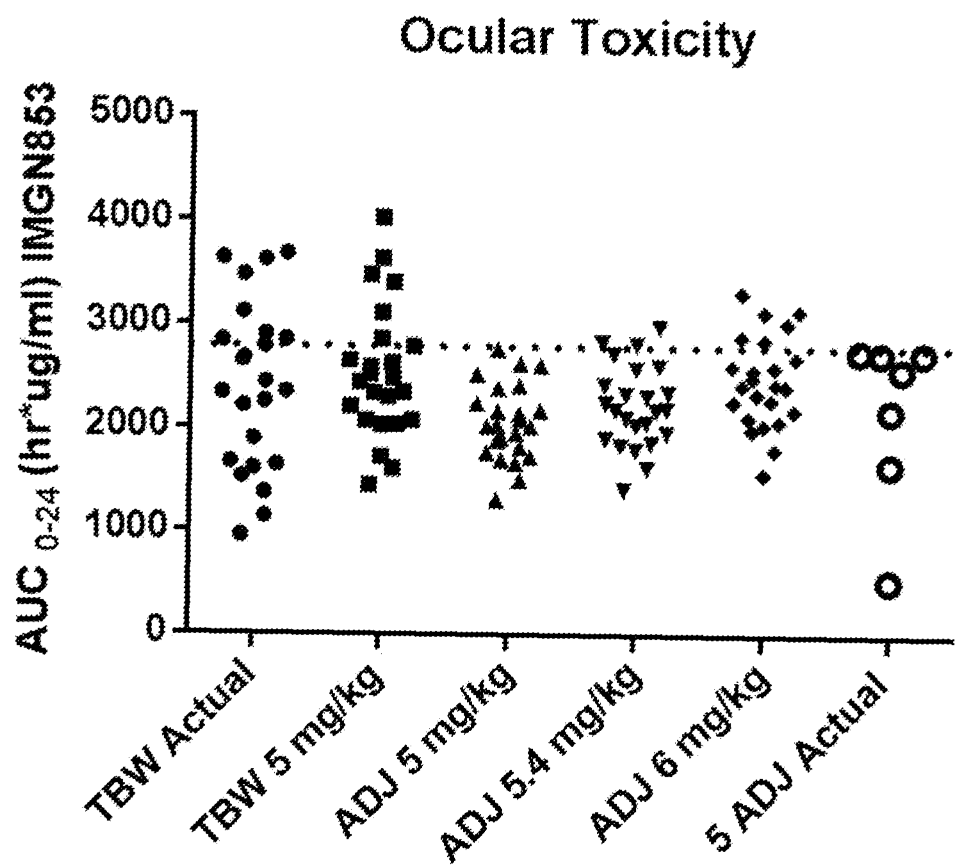


| | | | Percent of Patients Above Threshold | | | | | |
|---|---|---|---|---|---|---|---|---|
| Readout | PK Parameter | Threshold (hr*ug/ml) | TBW Actual | TBW 5 mg/kg | ADJ 5 mg/kg | ADJ 5.4 mg/kg | ADJ 6 mg/kg | 5 ADJ Actual |
| Ocular Toxicity | AUC_0-24 | >2741 | 38% | 29% | 0% | 13% | 25% | 14%* |

* - Patient @ 2749 hr*ug/ml

Figure 8

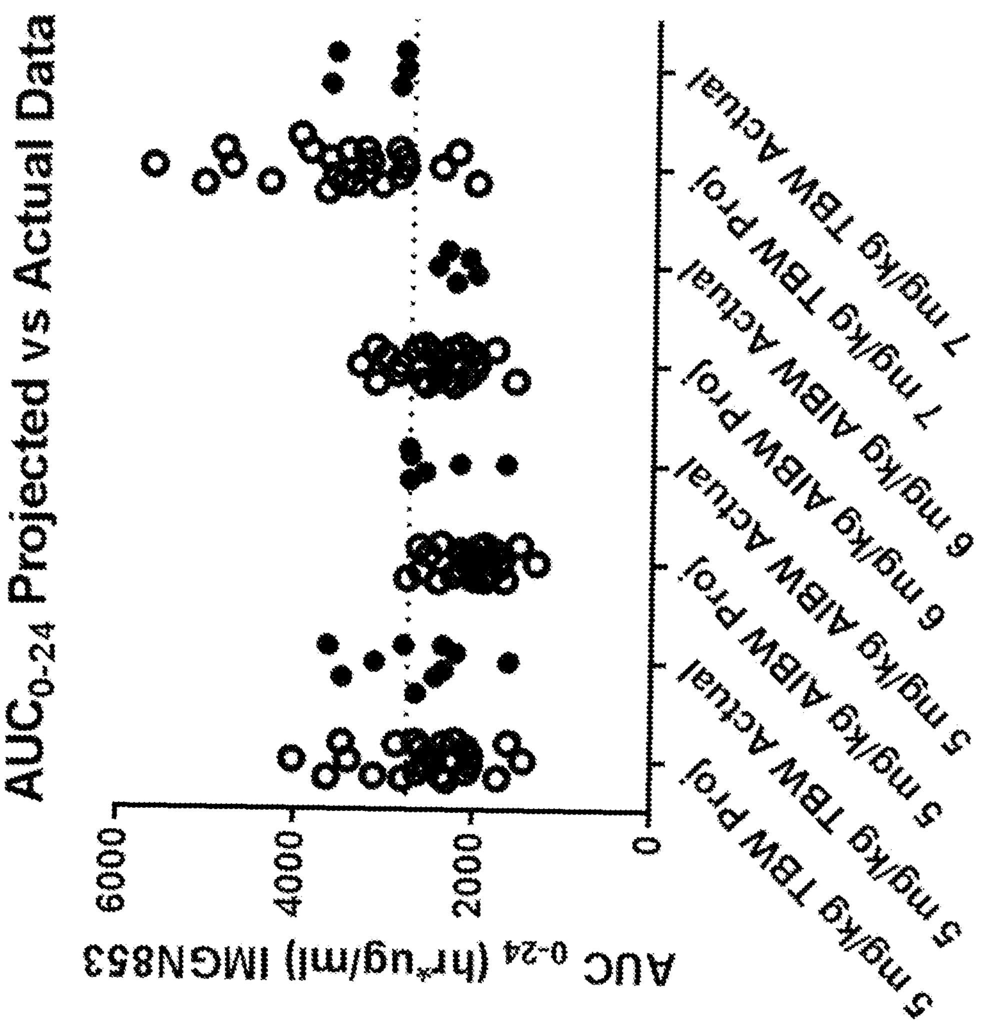
AUC$_{0-24}$ Projected vs Actual Data
AUC 0-24 (hr*ug/ml) IMGN853
6000
4000
2000
0
5 mg/kg TBW Proj
5 mg/kg TBW Actual
5 mg/kg AIBW Proj
5 mg/kg AIBW Actual
6 mg/kg AIBW Proj
6 mg/kg AIBW Actual
7 mg/kg AIBW Proj
7 mg/kg TBW Actual
7 mg/kg TBW Proj
7 mg/kg TBW Actual

**Figure 9**

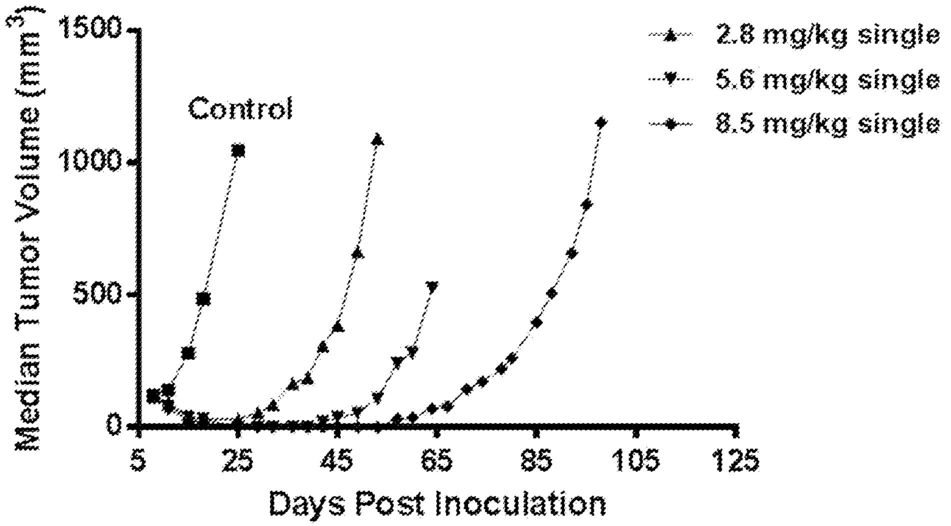


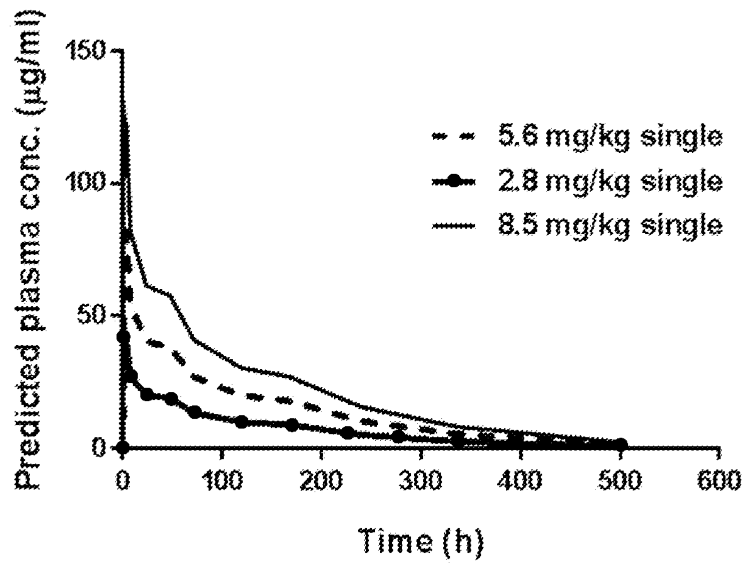


| Dose (mg/kg) | Schedule | T/C (%) | Log cell kill | CR | Cmax (µg/ml) | Cavg (µg/ml) | AUC 0-540 (hr*µg/ml) |
|---|---|---|---|---|---|---|---|
| 2.8 | Single | 3 | 1.5 | 2/6 | 43 | 7 | 3740 |
| 5.6 | Single | 0 | 2.7 | 4/6 | 86 | 15 | 7481 |
| 8.5 | Single | 0 | 3.9 | 6/6 | 131 | 23 | 11354 |

# Figure 10

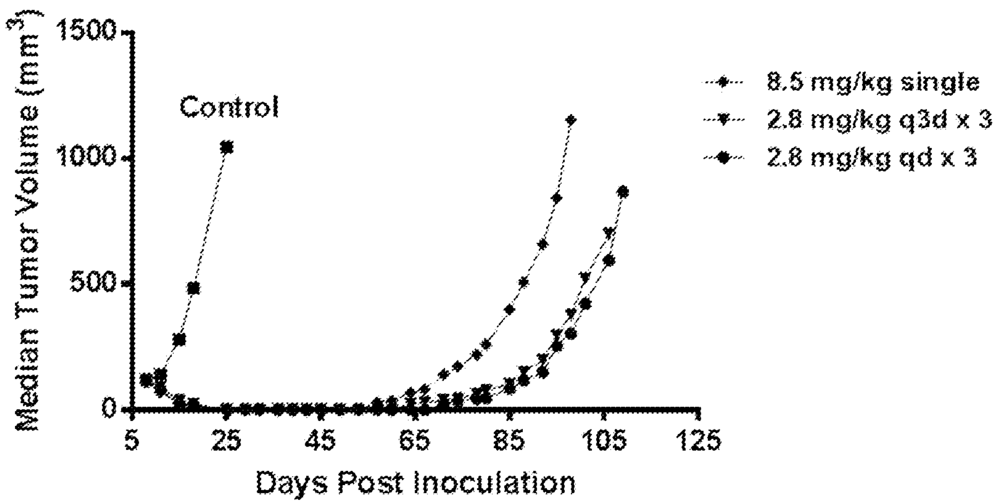


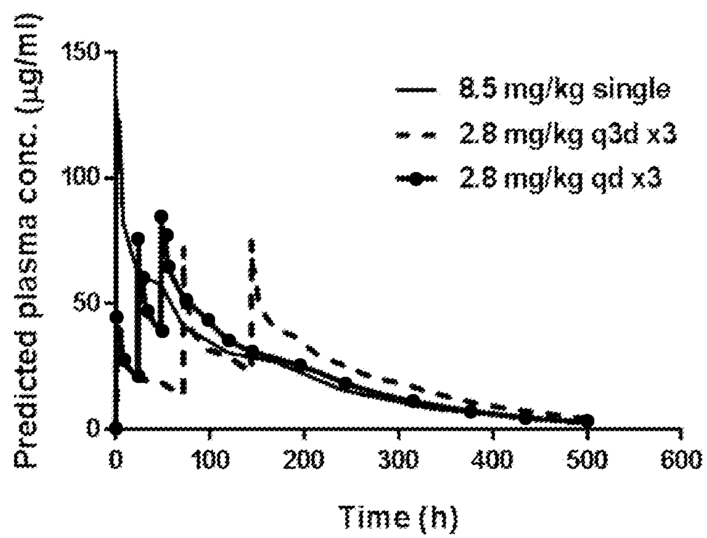


| Dose (mg/kg) | Schedule | Total dose (mg/kg) | T/C (%) | Log cell kill | CR | Tumor-free (Day 120) | Cmax (μg/ml) | Cavg (μg/ml) | AUC 0-540 (hr*μg/ml) |
|---|---|---|---|---|---|---|---|---|---|
| 8.5 | Single | 8.5 | 0 | 3.9 | 6/6 | 0/6 | 131 | 23 | 11354 |
| 2.8 | qd x 3 | 8.4 | 0 | 4.6 | 6/6 | 1/6 | 85 | 28 | 10961 |
| 2.8 | q3d x 3 | 8.4 | 0 | 4.1 | 6/6 | 2/6 | 75 | 21 | 10762 |

# Figure 11

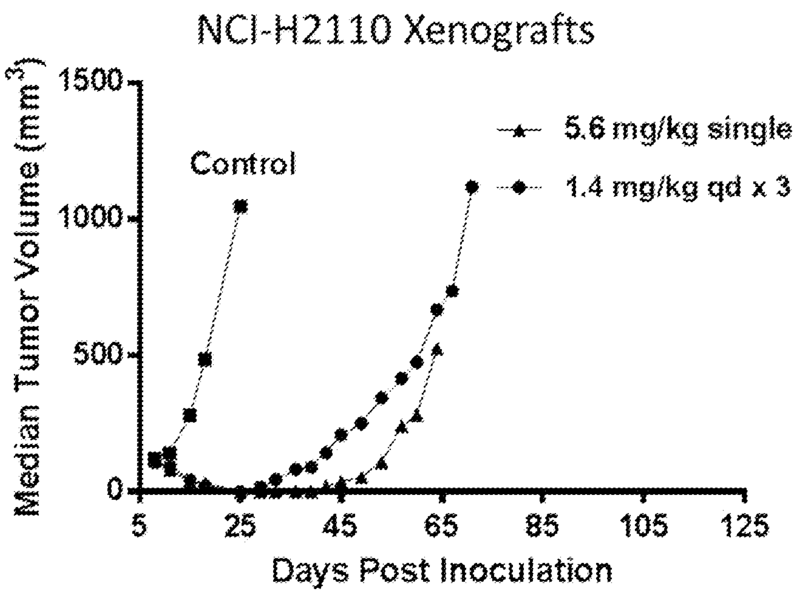


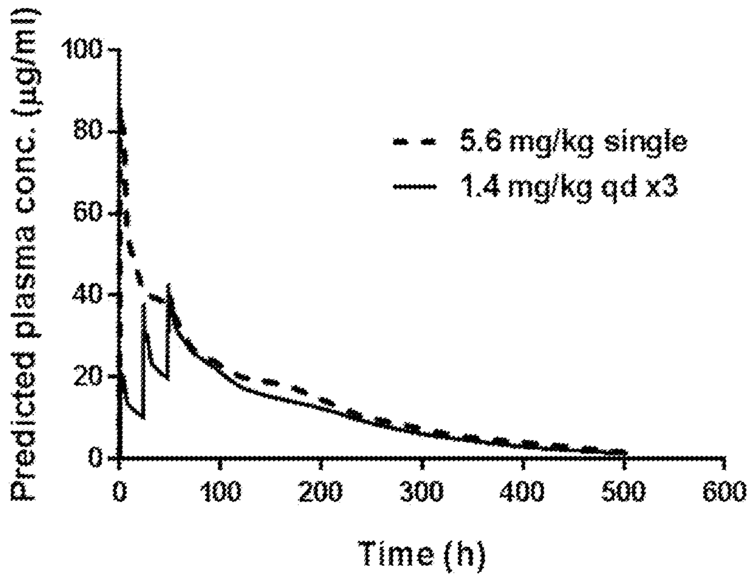


| Dose (mg/kg) | Schedule | Total Dose (mg/kg) | T/C (%) | Log cell kill | CR | Cmax (µg/ml) | Cavg (µg/ml) | AUC 0-540 (hr*µg/ml) |
|---|---|---|---|---|---|---|---|---|
| 5.6 | Single | 5.6 | 0 | 2.7 | 4/6 | 86 | 15 | 7481 |
| 1.4 | qd x 3 | 4.2 | 0 | 2.5 | 4/6 | 43 | 14 | 5480 |

# Figure 12

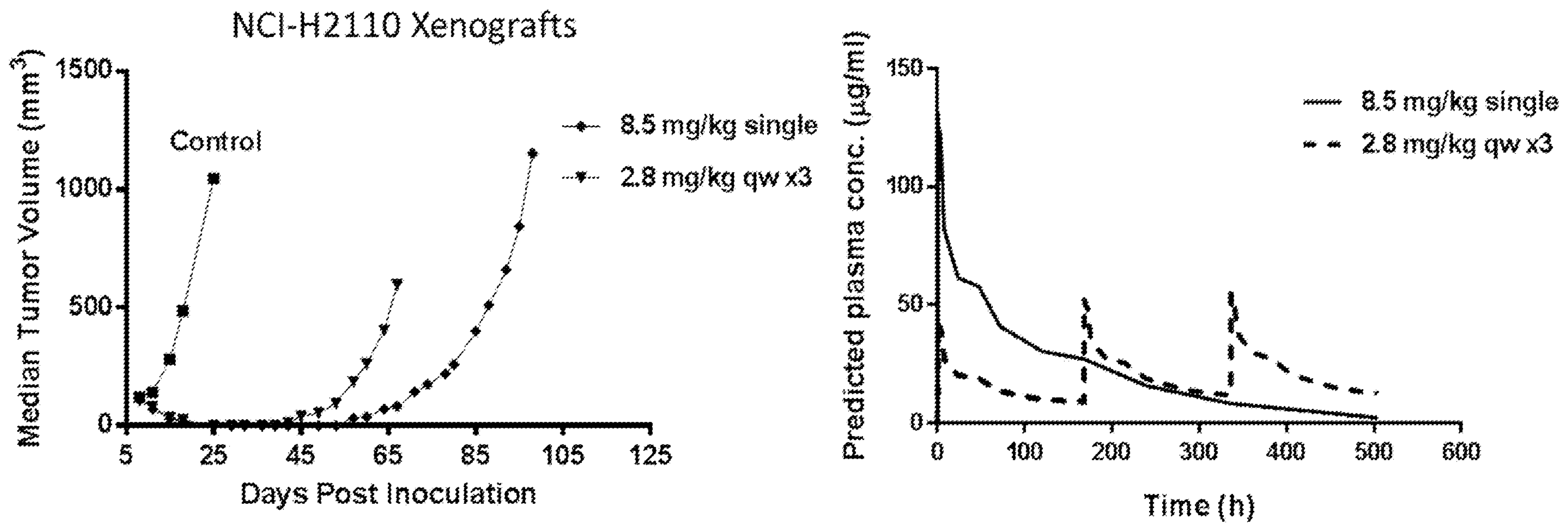


| Dose (mg/kg) | Schedule | Total Dose (mg/kg) | T/C (%) | Log cell kill | CR | Cmax (µg/ml) | Cavg (µg/ml) | AUC 0-540 (hr*µg/ml) |
|---|---|---|---|---|---|---|---|---|
| 8.5 | Single | 8.5 | 0 | 3.9 | 6/6 | 131 | 23 | 11354 |
| 2.8 | qw x 3 | 8.4 | 0 | 3.0 | 6/6 | 55 | 15 | 9614 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3896111 A
- US 2012009181 A1
- WO 2012135675 A2
- US 5225539 A
- US 20120009181 A
- US 4816567 A
- US 5750373 A
- US 5969108 A
- US 6172197 B
- US 5885793 A
- US 6521404 B
- US 6544731 B
- US 6555313 B
- US 6582915 B
- US 6593081 B
- US 6300064 B
- US 6653068 B
- US 6706484 B
- US 7264963 B
- US 5545807 A
- US 5545806 A
- US 5569825 A
- US 5625126 A
- US 5633425 A
- US 5661016 A
- US 20080312425 A
- US 20080177048 A
- US 20090187005 A
- US 4563304 A
- US 20090274713 A
- WO 20090134976 A
- US 20050169933 A
- US 20120282282 A
- US 4424219 A
- US 4256746 A
- US 4294757 A
- US 4307016 A
- US 4313946 A
- US 4315929 A
- US 4331598 A
- US 4361650 A
- US 4362663 A
- US 4364866 A
- US 4450254 A
- US 4322348 A
- US 4371533 A
- US 5208020 A
- US 5416064 A
- US 5475092 A
- US 5585499 A
- US 5846545 A
- US 6333410 B
- US 7276497 B
- US 7473796 B
- US 6441163 B
- US 6716821 B
- US 7368565 B
- US 20120282175 A
- WO 2012135675 A
- WO 2011106528 A
- WO 2012138749 A
- WO 61888365 A
- WO 61888337 A
- WO 61948363 A
- WO 62004815 A


### Non-patent literature cited in the description


- **JEMAL et al.** *Cancer J. Clin.,* 2003, vol. 53, 5-26
- **WEITMAN SD et al.** *Cancer Res,* 1992, vol. 52, 3396-3401
- **ANTONY AC.** *Annu Rev Nutr,* 1996, vol. 16, 501-521
- **KALLI KR et al.** *Gynecol Oncol,* 2008, vol. 108, 619-626
- **GRUENIGEN V et al.** *Cancer,* 2008, vol. 112, 2221-2227
- **AYHAN A et al.** *Am J Obstet Gynecol,* 2007, vol. 196 (81), e81-86
- **HARRY VN et al.** *Obstet Gynecol Surv,* 2009, vol. 64, 548-560
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **KABAT et al.** Sequences of Proteins of Immunological Interest. National Institutes of Health, 1991
- **AL-LAZIKANI et al.** *J. Molec. Biol.,* 1997, vol. 273, 927-948

- **KABAT et al.** Sequences of Immunological Interest. Public Health Service, National Institutes of Health, 1991
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **GREEN ; DUFFULL.** *British Journal of Clinical Pharmacology,* 2004, vol. 58, 119-133
- **W.A. WEBER.** *J. Nucl. Med.,* 2009, vol. 50, 1S-10S
- **JOHNSON et al.** *J. Clin. Oncol.,* 2003, vol. 21 (7), 1404-1411
- **KARLIN et al.** *Proc. Natl. Acad. Sci.,* 1990, vol. 87, 2264-2268
- **KARLIN et al.** *Proc. Natl. Acad. Sci.,* 1993, vol. 90, 5873-5877
- **ALTSCHUL et al.** *Nucleic Acids Res.,* 1991, vol. 25, 3389-3402
- **ALTSCHUL et al.** *Nucleic Acids Res.,* 1997, vol. 25, 3389-3402
- **ALTSCHUL et al.** *Methods in Enzymology,* 1996, vol. 266, 460-480
- **NEEDLEMAN ; WUNSCH.** *J. Mol. Biol.,* 1970, vol. 48, 444-453
- **MYERS ; MILLER.** *CABIOS,* 1989, vol. 4, 11-17
- **SMITH ; WATERMAN.** *Advances in Applied Mathematics,* 1981, vol. 2, 482-489
- **BRUMMELL et al.** *Biochem.,* 1993, vol. 32, 1180-1 187
- **KOBAYASHI et al.** *Protein Eng.,* 1999, vol. 12 (10), 879-884
- **BURKS et al.** *Proc. Natl. Acad. Sci. USA,* 1997, vol. 94, 412-417
- **KÖHLER ; MILSTEIN.** *Nature,* 1975, vol. 256, 495
- **GODING.** Monoclonal Antibodies: Principles and Practice. Academic Press, 1986
- **MCCAFFERTY et al.** *Nature,* 1990, vol. 348, 552-554
- **CLACKSON et al.** *Nature,* 1991, vol. 352, 624-628
- **MARKS et al.** *J. Mol. Biol.,* 1991, vol. 222, 581-597
- **COLE et al.** Monoclonal Antibodies and Cancer Therapy. Alan R. Liss, 1985, 77
- **BOEMER et al.** *J. Immunol.,* 1991, vol. 147 (1), 86-95
- **VAUGHAN et al.** *Nat. Biotech.,* 1996, vol. 14, 309-314
- **SHEETS et al.** *Proc. Nat'l. Acad. Sci.,* 1998, vol. 95, 6157-6162
- **HOOGENBOOM ; WINTER.** *J. Mol. Biol.,* 1991, vol. 227, 381
- **MARKS et al.** *J. Mol. Biol.,* 1991, vol. 222, 581
- **ROTHE et al.** *J. Mol. Bio.,* 2007
- **MARKS et al.** *Bio/Technology,* 1992, vol. 10, 779-783
- REMINGTON'S PHARMACEUTICAL SCIENCES. Mack Publishing Co, 2000
- **CARLSSON et al.** *Biochem. J,* 1978, vol. 173, 723-737
- *CHEMICAL ABSTRACTS,* 341498-08-6
- **YOSHITAKE et al.** *Eur. J. Biochem.,* 1979, vol. 101, 395-399
- **HASHIDA et al.** *J. Applied Biochem.,* 1984, 56-63
- **LIU et al.** *Biochem.,* 1979, vol. 18, 690-697
- Remington, The Science and Practice of Pharmacy. Mack Publishing, 2000

SEQUENCE LISTING

<110> IMMUNOGEN, INC.
LUTZ, ROBERT J
PONTE, JOSE

<120> ANTI-FOLR1 IMMUNOCONJUGATE DOSING REGIMENS

<130> 2921.055PC04/EKS/CLD

<150> 61/888,365
<151> 2013-10-08

<150> 61/888,337
<151> 2013-10-08

<150> 61/948,363
<151> 2014-03-05

<150> 62/004,815
<151> 2014-05-29

<160> 16

<170> PatentIn version 3.5

<210> 1
<211> 257
<212> PRT
<213> Homo sapiens

<400> 1

```
Met Ala Gln Arg Met Thr Thr Gln Leu Leu Leu Leu Leu Val Trp Val
1               5                   10                  15

Ala Val Val Gly Glu Ala Gln Thr Arg Ile Ala Trp Ala Arg Thr Glu
            20                  25                  30

Leu Leu Asn Val Cys Met Asn Ala Lys His His Lys Glu Lys Pro Gly
        35                  40                  45

Pro Glu Asp Lys Leu His Glu Gln Cys Arg Pro Trp Arg Lys Asn Ala
    50                  55                  60

Cys Cys Ser Thr Asn Thr Ser Gln Glu Ala His Lys Asp Val Ser Tyr
65                  70                  75                  80

Leu Tyr Arg Phe Asn Trp Asn His Cys Gly Glu Met Ala Pro Ala Cys
                85                  90                  95

Lys Arg His Phe Ile Gln Asp Thr Cys Leu Tyr Glu Cys Ser Pro Asn
            100                 105                 110

Leu Gly Pro Trp Ile Gln Gln Val Asp Gln Ser Trp Arg Lys Glu Arg
        115                 120                 125
```

```
Val Leu Asn Val Pro Leu Cys Lys Glu Asp Cys Glu Gln Trp Trp Glu
    130                 135                 140

Asp Cys Arg Thr Ser Tyr Thr Cys Lys Ser Asn Trp His Lys Gly Trp
145                 150                 155                 160

Asn Trp Thr Ser Gly Phe Asn Lys Cys Ala Val Gly Ala Ala Cys Gln
                165                 170                 175

Pro Phe His Phe Tyr Phe Pro Thr Pro Thr Val Leu Cys Asn Glu Ile
            180                 185                 190

Trp Thr His Ser Tyr Lys Val Ser Asn Tyr Ser Arg Gly Ser Gly Arg
        195                 200                 205

Cys Ile Gln Met Trp Phe Asp Pro Ala Gln Gly Asn Pro Asn Glu Glu
    210                 215                 220

Val Ala Arg Phe Tyr Ala Ala Ala Met Ser Gly Ala Gly Pro Trp Ala
225                 230                 235                 240

Ala Trp Pro Phe Leu Leu Ser Leu Ala Leu Met Leu Leu Trp Leu Leu
                245                 250                 255

Ser
```

<210> 2
<211> 771
<212> DNA
<213> Homo sapiens

<400> 2

```
atggctcagc ggatgacaac acagctgctg ctccttctag tgtgggtggc tgtagtaggg      60
gaggctcaga caaggattgc atgggccagg actgagcttc tcaatgtctg catgaacgcc     120
aagcaccaca aggaaaagcc aggccccgag gacaagttgc atgagcagtg tcgaccctgg     180
aggaagaatg cctgctgttc taccaacacc agccaggaag cccataagga tgtttcctac     240
ctatatagat tcaactggaa ccactgtgga gagatggcac ctgcctgcaa acggcatttc     300
atccaggaca cctgcctcta cgagtgctcc cccaacttgg ggccctggat ccagcaggtg     360
gatcagagct ggcgcaaaga gcgggtactg aacgtgcccc tgtgcaaaga ggactgtgag     420
caatggtggg aagattgtcg cacctcctac acctgcaaga gcaactggca caagggctgg     480
aactggactt cagggtttaa caagtgcgca gtgggagctg cctgccaacc tttccatttc     540
tacttcccca cacccactgt tctgtgcaat gaaatctgga ctcactccta caaggtcagc     600
```

aactacagcc gagggagtgg ccgctgcatc cagatgtggt tcgacccagc ccagggcaac 660

cccaatgagg aggtggcgag gttctatgct gcagccatga gtggggctgg gccctgggca 720

gcctggcctt tcctgcttag cctggcccta atgctgctgt ggctgctcag c 771

<210> 3
<211> 118
<212> PRT
<213> Artificial Sequence

<220>
<223> huMov19 vHC

<400> 3

Gln Val Gln Leu Val Gln Ser Gly Ala Glu Val Val Lys Pro Gly Ala
1 5 10 15

Ser Val Lys Ile Ser Cys Lys Ala Ser Gly Tyr Thr Phe Thr Gly Tyr
20 25 30

Phe Met Asn Trp Val Lys Gln Ser Pro Gly Gln Ser Leu Glu Trp Ile
35 40 45

Gly Arg Ile His Pro Tyr Asp Gly Asp Thr Phe Tyr Asn Gln Lys Phe
50 55 60

Gln Gly Lys Ala Thr Leu Thr Val Asp Lys Ser Ser Asn Thr Ala His
65 70 75 80

Met Glu Leu Leu Ser Leu Thr Ser Glu Asp Phe Ala Val Tyr Tyr Cys
85 90 95

Thr Arg Tyr Asp Gly Ser Arg Ala Met Asp Tyr Trp Gly Gln Gly Thr
100 105 110

Thr Val Thr Val Ser Ser
115

<210> 4
<211> 112
<212> PRT
<213> Artificial Sequence

<220>
<223> huMov19 vLCv1.00

<400> 4

Asp Ile Val Leu Thr Gln Ser Pro Leu Ser Leu Ala Val Ser Leu Gly
1 5 10 15

```
Gln Pro Ala Ile Ile Ser Cys Lys Ala Ser Gln Ser Val Ser Phe Ala
            20                  25                  30

Gly Thr Ser Leu Met His Trp Tyr His Gln Lys Pro Gly Gln Gln Pro
        35                  40                  45

Arg Leu Leu Ile Tyr Arg Ala Ser Asn Leu Glu Ala Gly Val Pro Asp
    50                  55                  60

Arg Phe Ser Gly Ser Gly Ser Lys Thr Asp Phe Thr Leu Asn Ile Ser
65                  70                  75                  80

Pro Val Glu Ala Glu Asp Ala Ala Thr Tyr Tyr Cys Gln Gln Ser Arg
                85                  90                  95

Glu Tyr Pro Tyr Thr Phe Gly Gly Gly Thr Lys Leu Glu Ile Lys Arg
            100                 105                 110
```

<210> 5
<211> 112
<212> PRT
<213> Artificial Sequence

<220>
<223> huMov19 vLCv1.60

<400> 5

```
Asp Ile Val Leu Thr Gln Ser Pro Leu Ser Leu Ala Val Ser Leu Gly
1               5                   10                  15

Gln Pro Ala Ile Ile Ser Cys Lys Ala Ser Gln Ser Val Ser Phe Ala
            20                  25                  30

Gly Thr Ser Leu Met His Trp Tyr His Gln Lys Pro Gly Gln Gln Pro
        35                  40                  45

Arg Leu Leu Ile Tyr Arg Ala Ser Asn Leu Glu Ala Gly Val Pro Asp
    50                  55                  60

Arg Phe Ser Gly Ser Gly Ser Lys Thr Asp Phe Thr Leu Thr Ile Ser
65                  70                  75                  80

Pro Val Glu Ala Glu Asp Ala Ala Thr Tyr Tyr Cys Gln Gln Ser Arg
                85                  90                  95

Glu Tyr Pro Tyr Thr Phe Gly Gly Gly Thr Lys Leu Glu Ile Lys Arg
            100                 105                 110
```

<210> 6
<211> 15
<212> PRT
<213> Artificial Sequence

<220>
<223> huMov19 vLC CDR1

<400> 6

```
Lys Ala Ser Gln Ser Val Ser Phe Ala Gly Thr Ser Leu Met His
1               5                   10                  15
```

<210> 7
<211> 7
<212> PRT
<213> Artificial Sequence

<220>
<223> huMov19 vLC CDR2

<400> 7

```
Arg Ala Ser Asn Leu Glu Ala
1               5
```

<210> 8
<211> 9
<212> PRT
<213> Artificial Sequence

<220>
<223> huMov19 vLC CDR3

<400> 8

```
Gln Gln Ser Arg Glu Tyr Pro Tyr Thr
1               5
```

<210> 9
<211> 5
<212> PRT
<213> Artificial Sequence

<220>
<223> huMov19 vHC CDR1

<400> 9

```
Gly Tyr Phe Met Asn
1               5
```

<210> 10
<211> 17
<212> PRT
<213> Artificial Sequence

<220>

<223> huMov19 vHC CDR2 - Kabat Defined

<400> 10

```
Arg Ile His Pro Tyr Asp Gly Asp Thr Phe Tyr Asn Gln Lys Phe Gln
1               5                   10                  15

Gly
```

<210> 11
<211> 10
<212> PRT
<213> Artificial Sequence

<220>
<223> huMovl9 vHC CDR2 - Abm Defined

<400> 11

```
Arg Ile His Pro Tyr Asp Gly Asp Thr Phe
1               5                   10
```

<210> 12
<211> 9
<212> PRT
<213> Artificial Sequence

<220>
<223> huMov19 vHC CDR3

<400> 12

```
Tyr Asp Gly Ser Arg Ala Met Asp Tyr
1               5
```

<210> 13
<211> 448
<212> PRT
<213> Artificial Sequence

<220>
<223> huMov19 HC

<400> 13

```
Gln Val Gln Leu Val Gln Ser Gly Ala Glu Val Val Lys Pro Gly Ala
1               5                   10                  15

Ser Val Lys Ile Ser Cys Lys Ala Ser Gly Tyr Thr Phe Thr Gly Tyr
            20                  25                  30

Phe Met Asn Trp Val Lys Gln Ser Pro Gly Gln Ser Leu Glu Trp Ile
        35                  40                  45

Gly Arg Ile His Pro Tyr Asp Gly Asp Thr Phe Tyr Asn Gln Lys Phe
    50                  55                  60
```

```
Gln Gly Lys Ala Thr Leu Thr Val Asp Lys Ser Ser Asn Thr Ala His
65                  70                  75                  80

Met Glu Leu Leu Ser Leu Thr Ser Glu Asp Phe Ala Val Tyr Tyr Cys
                85                  90                  95

Thr Arg Tyr Asp Gly Ser Arg Ala Met Asp Tyr Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
```

305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Tyr Thr Leu
340 345 350

Pro Pro Ser Arg Asp Glu Leu Thr Lys Asn Gln Val Ser Leu Thr Cys
355 360 365

Leu Val Lys Gly Phe Tyr Pro Ser Asp Ile Ala Val Glu Trp Glu Ser
370 375 380

Asn Gly Gln Pro Glu Asn Asn Tyr Lys Thr Thr Pro Pro Val Leu Asp
385 390 395 400

Ser Asp Gly Ser Phe Phe Leu Tyr Ser Lys Leu Thr Val Asp Lys Ser
405 410 415

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro Gly Lys
435 440 445

<210> 14
<211> 218
<212> PRT
<213> Artificial Sequence

<220>
<223> huMov19 LCv1.00

<400> 14

```
Asp Ile Val Leu Thr Gln Ser Pro Leu Ser Leu Ala Val Ser Leu Gly
1               5                   10                  15

Gln Pro Ala Ile Ile Ser Cys Lys Ala Ser Gln Ser Val Ser Phe Ala
            20                  25                  30

Gly Thr Ser Leu Met His Trp Tyr His Gln Lys Pro Gly Gln Gln Pro
        35                  40                  45

Arg Leu Leu Ile Tyr Arg Ala Ser Asn Leu Glu Ala Gly Val Pro Asp
    50                  55                  60

Arg Phe Ser Gly Ser Gly Ser Lys Thr Asp Phe Thr Leu Asn Ile Ser
65                  70                  75                  80

Pro Val Glu Ala Glu Asp Ala Ala Thr Tyr Tyr Cys Gln Gln Ser Arg
                85                  90                  95

Glu Tyr Pro Tyr Thr Phe Gly Gly Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 15
<211> 218
<212> PRT
<213> Artificial Sequence

<220>
<223> huMov19 LCv1.60

<400> 15

```
Asp Ile Val Leu Thr Gln Ser Pro Leu Ser Leu Ala Val Ser Leu Gly
1               5                   10                  15

Gln Pro Ala Ile Ile Ser Cys Lys Ala Ser Gln Ser Val Ser Phe Ala
            20                  25                  30

Gly Thr Ser Leu Met His Trp Tyr His Gln Lys Pro Gly Gln Gln Pro
        35                  40                  45

Arg Leu Leu Ile Tyr Arg Ala Ser Asn Leu Glu Ala Gly Val Pro Asp
    50                  55                  60

Arg Phe Ser Gly Ser Gly Ser Lys Thr Asp Phe Thr Leu Thr Ile Ser
65                  70                  75                  80

Pro Val Glu Ala Glu Asp Ala Ala Thr Tyr Tyr Cys Gln Gln Ser Arg
                85                  90                  95

Glu Tyr Pro Tyr Thr Phe Gly Gly Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 16
<211> 17
<212> PRT
<213> Artificial Sequence

<220>
<223> muMov19 vHC CDR2 - Kabat Defined

<400> 16

```
Arg Ile His Pro Tyr Asp Gly Asp Thr Phe Tyr Asn Gln Asn Phe Lys
1               5                   10                  15

Asp
```